(19) Europäisches Patentamt
European Patent Office
Office européen des brevets

(11) EP 4 471 032 A1

(12) EUROPEAN PATENT APPLICATION
published in accordance with Art. 153(4) EPC

(43) Date of publication:
**04.12.2024 Bulletin 2024/49**

(21) Application number: **23742996.4**

(22) Date of filing: **20.01.2023**

(51) International Patent Classification (IPC):
***C07D 487/04*** *(2006.01)* ***A61K 31/495*** *(2006.01)*
***A61K 31/4985*** *(2006.01)* ***A61P 25/22*** *(2006.01)*

(52) Cooperative Patent Classification (CPC):
**A61K 31/495; A61K 31/4985; A61P 25/22; C07D 487/04**

(86) International application number:
**PCT/CN2023/073323**

(87) International publication number:
**WO 2023/138681 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.01.2022 CN 202210078957**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**
- **Changzhou Hansoh Pharmaceutical Co., Ltd.**
  **Xinbei District**
  **Changzhou**
  **Jiangsu 213001 (CN)**

(72) Inventors:
- **CHEN, Jinyao**
  **Shanghai 201203 (CN)**
- **GUO, Linsong**
  **Shanghai 201203 (CN)**

(74) Representative: **EP&C**
**P.O. Box 3241**
**2280 GE Rijswijk (NL)**

# (54) ACIDIC SALT OR CRYSTAL FORM OF NITROGEN-CONTAINING FUSED RING DERIVATIVE INHIBITOR, AND PREPARATION METHOD THEREFOR AND USE THEREOF

(57) The present invention relates to an acidic salt or a crystal form of a nitrogen-containing fused ring derivative inhibitor, and a preparation method therefor and the use thereof. The salt or crystal form of the inhibitor of the present invention can be used for treating related diseases such as depression, anxiety disorder, schizophrenia and sex hormone dependence, and has broad application prospects.

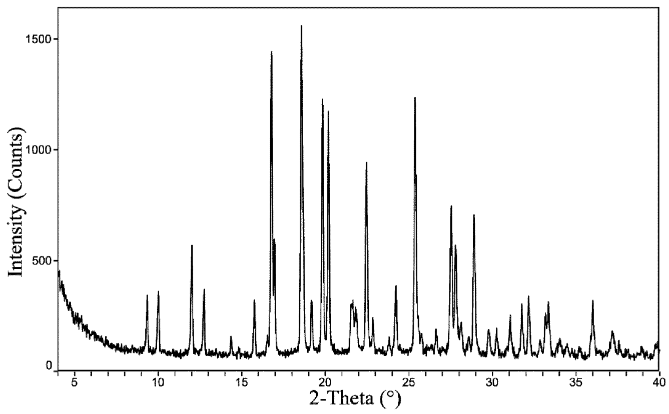


Figure 1



Processed by Luminess, 75001 PARIS (FR)

## Description

Technical Field

The present invention falls within the technical field of biomedicine, and in particular relates to a crystal form or an acidic salt of an NK3 inhibitor, and a preparation method therefor and the use thereof.

Background Art

Neurokinin (NK) includes substance P (SP), neurokinin A and neurokinin B, and the corresponding three types of receptors are neurokinin 1 receptor (NK1R), neurokinin 2 receptor (NK2R) and neurokinin 3 receptor (NK3R). These three types of receptors are all G protein-coupled receptors, of which NK1R is the most widely distributed and is distributed in both the central and peripheral nervous systems, NK2R is mainly distributed in the peripheral nervous system, and NK3R is mainly distributed in the central nervous system. At present, NK receptor inhibitors have been used to treat menopausal hot flashes, depression, schizophrenia and other diseases. In particular, NK3R is closely related to symptoms of menopausal syndrome such as hot flashes. NK3R inhibitors have been proven to have a good effect on improving menopausal hot flashes. Menopausal hot flashes refer to symptoms such as hot flashes and sweating that often occur in menopausal people, and are prominent features of menopausal syndrome. Menopausal hot flashes are caused by vasomotor dysfunction due to falling estrogen levels in the body. When estrogen in the body decreases, it causes the brain to mistakenly think that the body temperature is too high. Therefore, the brain sends a signal to the heart asking the heart to pump more blood, and the sweat glands release more sweat, accompanied by sweating, palpitations, dizziness, etc. More than three-quarters of women have hot flashes during menopause, and 80% of patients have hot flashes that can last for at least one year, and some can last until about 5 years after menopause. The current treatment for menopausal hot flashes is mainly hormone replacement therapy, but this therapy can easily cause breast cancer, stroke, coronary heart disease, dementia and other diseases, with a high risk factor. Oral drugs, such as paroxetine (an SSRI drug that treats depression), are the only approved small molecule drugs for the treatment of menopausal hot flashes. They also have side effects and are only approved in the United States. Therefore, in clinical practice, it is necessary to develop safer and more effective drugs for the treatment of menopausal syndrome.

Patent PCT/CN2021/109577 discloses a series of NK3R inhibitors, and this compound has very good effects. The present invention further studies the salt form and crystal form of the compound of formula I to meet the needs of clinical drug development. The structure of formula (I) is as follows:

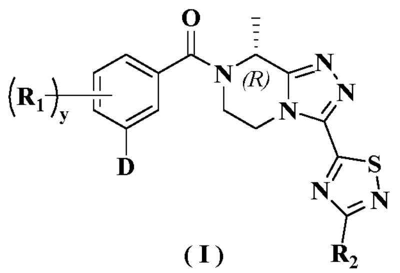

(I)

Summary of the Invention

In order to improve the solubility and solid stability of the product, reduce storage costs, extend the product cycle, and improve the bioavailability of the product, the present invention conducts a comprehensive study on the salt form and salt crystal form of the above compounds.

The present invention provides a crystal form, or an acidic salt or a crystal form of the salt of a compound as represented by general formula (I):

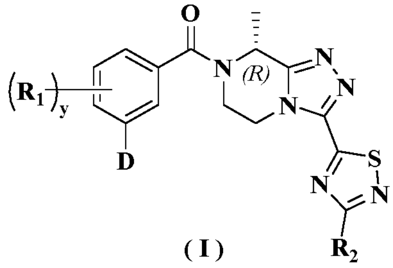

(I)

wherein:

$R_1$ is selected from hydrogen, deuterium or halogen; preferably hydrogen, deuterium, fluorine, chlorine or bromine; $R_3$ is selected from hydrogen, deuterium, halogen, $C_{1-6}$ alkyl, $C_{1-6}$ deuteroalkyl, or 3- to 6-membered heterocyclyl; the $C_{1-6}$ alkyl, $C_{1-6}$ deuteroalkyl and 3- to 6-membered heterocyclyl are optionally further substituted by one or more substituents selected from deuterium and halogen; preferably hydrogen, deuterium, fluorine, chlorine, bromine, methyl, deuterated methyl, azetidinyl, tetrahydropyrrolyl,

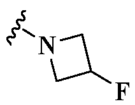 or 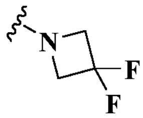 ;

y is 0, 1, 2, 3, 4 or 5.

In a preferred scheme of the present invention, the compound has the following specific structure:

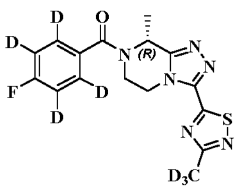 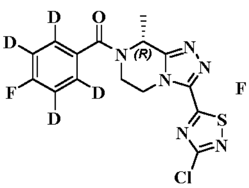 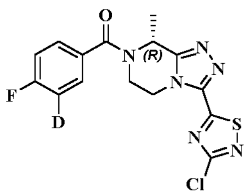 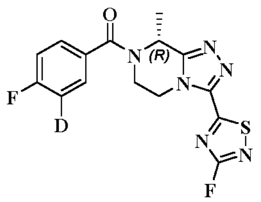

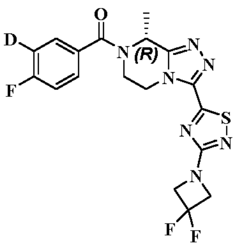 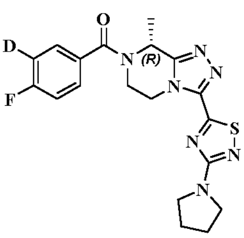 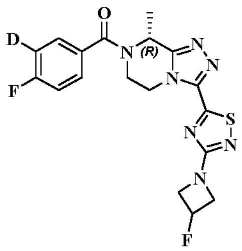 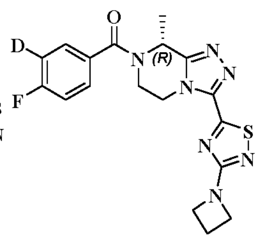

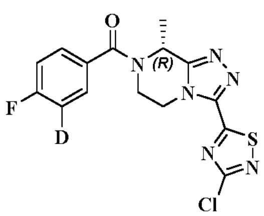

In a further preferred embodiment of the present invention, provided is a crystal form, or an acidic salt or a crystal form of the salt of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, the compound having the following structure:

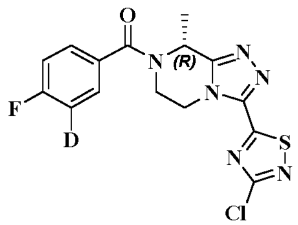

In certain embodiments of the present invention, the acidic salt of the compound of formula I refers to the salt of the compound of formula I and a pharmaceutically acceptable acid. In certain embodiments of the present invention, the salt can be an acid addition salt with the following acid, etc.: hydrochloric acid, hydrobromic acid, hydroiodic acid, sulfuric acid, nitric acid, phosphoric acid and other inorganic acids, as well as formic acid, acetic acid, propionic acid, oxalic acid, malonic acid, succinic acid, fumaric acid, maleic acid, lactic acid, malic acid, mandelic acid, tartaric acid, dibenzoyltartaric acid, dimethylbenzoyltartaric acid, citric acid, methanesulfonic acid, ethanesulfonic acid, benzenesulfonic acid, p-toluenesulfonic acid, aspartic acid, glutamic acid and other organic acids. In certain embodiments of the present invention, the salt can be an acid addition salt with the following acid, etc.: benzoic acid, 10-camphor sulfonic acid, chlorotheophylline, 1,2-ethanedisulfonic acid, glucoheptonic acid, gluconic acid, glucuronic acid, hippuric acid, hydroxyethyl sulfonic acid, lactobionic acid, lauryl sulfuric acid, methyl sulfuric acid, naphthoic acid, naphthalene sulfonic acid, stearic acid, oleic acid, pamoic acid, polygalacturonic acid, sulfosalicylic acid, trifluoroacetic acid, 2,2-dichloroacetic acid, acetylglycine, adipic acid, alginic acid, ascorbic acid, 4-acetamidobenzoic acid, 2-(4-hydroxybenzoyl)benzoic acid, butyric acid, camphor acid, decanoic acid, hexanoic acid, octanoic acid, carbonic acid, cinnamic acid, cyclohexaminosulfonic acid, di-(tert-butyl)naphthalene disulfonic acid, di-(tert-butyl)naphthalene sulfonic acid, 2-hydroxyethanesulfonic acid, galactaric acid, gentisuric acid, glucaric acid, glucoheptonic acid, glutaric acid, 2-oxoglutaric acid, glycerophosphoric acid, glycolic acid, isobutyric acid, lauric acid, naphthalene-1,5-disulfonic acid, 1-hydroxy-2-naphthoic acid, nicotinic acid, whey acid, palmitic acid, pyroglutamic acid, pyruvic acid, ortho-benzoic sulfimide, salicylic acid, 4-aminosalicylic acid, sebacic acid, thiocyanic acid, undecylenoic acid.

Further, in certain embodiments of the present invention, the acidic salt is selected from hydrochloride, methanesulfonate, nitrate, sulfate or hydrobromide.

In certain embodiments of the present invention, provided is a free base crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has a diffraction peak at 2θ of 9.3 ± 0.2°, or has a diffraction peak at 2θ of 10.0 ± 0.2°, or has a diffraction peak at 2θ of 12.0 ± 0.2°, or has a diffraction peak at 2θ of 12.8 ± 0.2°, or has a diffraction peak at 2θ of 16.8 ± 0.2°, or has a diffraction peak at 2θ of 18.6 ± 0.2°, or has a diffraction peak at 2θ of 19.2 ± 0.2°, or has a diffraction peak at 2θ of 19.8 ± 0.2°, or has a diffraction peak at 2θ of 20.2 ± 0.2°, or has a diffraction peak at 2θ of 21.8 ± 0.2°, or has a diffraction peak at 2θ of 22.5 ± 0.2°, or has a diffraction peak at 2θ of 22.8 ± 0.2°, or has a diffraction peak at 2θ of 24.2 ± 0.2°, or has a diffraction peak at 2θ of 25.4 ± 0.2°, or has a diffraction peak at 2θ of 27.5 ± 0.2°, or has a diffraction peak at 2θ of 27.8 ± 0.2°, or has a diffraction peak at 2θ of 28.9 ± 0.2°; preferably, the X-ray powder diffraction pattern thereof optionally has a diffraction peak at 2, 4, 6, 8 or 10 of the above-mentioned 2θ.

Further, in certain embodiments of the present invention, provided is a free base crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has at least a diffraction peak at one or more of 2θ of 16.8 ± 0.2°, 18.6 ± 0.2°, 19.8 ± 0.2° or 20.2 ± 0.2°; preferably, the X-ray powder diffraction pattern thereof has a diffraction peak at 2 to 4 of the above-mentioned 2θ, more preferably, at 3 or 4 of the above-mentioned 2θ, and most preferably, at 4 of the above-mentioned 2θ; optionally, the X-ray powder diffraction pattern thereof further has a diffraction peak at one or more of 2θ of 9.3 ± 0.2°, 10.0 ± 0.2°, 12.0 ± 0.2°, 12.8 ± 0.2° or 22.5 ± 0.2°, preferably at 2, 3, 4 or 5 of the above-mentioned 2θ;

furthermore, in certain embodiments of the present invention, provided is a free base crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

9.3 ± 0.2°, 10.0 ± 0.2°, 16.8 ± 0.2°, 18.6 ± 0.2°;
or 10.0 ± 0.2°, 12.0 ± 0.2°, 16.8 ± 0.2°, 18.6 ± 0.2°;
or 12.0 ± 0.2°, 12.8 ± 0.2°, 16.8 ± 0.2°, 18.6 ± 0.2°;
or 12.8 ± 0.2°, 22.5 ± 0.2°, 16.8 ± 0.2°, 18.6 ± 0.2°;
or 9.3 ± 0.2°, 10.0 ± 0.2°, 18.6 ± 0.2°, 19.8 ± 0.2°;
or 10.0 ± 0.2°, 12.0 ± 0.2°, 18.6 ± 0.2°, 19.8 ± 0.2°;
or 12.0 ± 0.2°, 12.8 ± 0.2°, 18.6 ± 0.2°, 19.8 ± 0.2°;
or 12.8 ± 0.2°, 22.5 ± 0.2°, 18.6 ± 0.2°, 19.8 ± 0.2°;

or 9.3 ± 0.2°, 10.0 ± 0.2°, 19.8 ± 0.2°, 20.2 ± 0.2°;
or 10.0 ± 0.2°, 12.0 ± 0.2°, 19.8 ± 0.2°, 20.2 ± 0.2°;
or 12.0 ± 0.2°, 12.8 ± 0.2°, 19.8 ± 0.2°, 20.2 ± 0.2°;
or 12.8 ± 0.2°, 22.5 ± 0.2°, 19.8 ± 0.2°, 20.2 ± 0.2°;
or 9.3 ± 0.2°, 10.0 ± 0.2°, 16.8 ± 0.2°, 18.6 ± 0.2°, 19.8 ± 0.2°;
or 10.0 ± 0.2°, 12.0 ± 0.2°, 16.8 ± 0.2°, 18.6 ± 0.2°, 19.8 ± 0.2°;
or 12.0 ± 0.2°, 12.8 ± 0.2°, 16.8 ± 0.2°, 18.6 ± 0.2°, 19.8 ± 0.2°;
or 12.8 ± 0.2°, 22.5 ± 0.2°, 16.8 ± 0.2°, 18.6 ± 0.2°, 19.8 ± 0.2°;
or 9.3 ± 0.2°, 10.0 ± 0.2°, 18.6 ± 0.2°, 19.8 ± 0.2°, 20.2 ± 0.2°;
or 10.0 ± 0.2°, 12.0 ± 0.2°, 18.6 ± 0.2°, 19.8 ± 0.2°, 20.2 ± 0.2°;
or 12.0 ± 0.2°, 12.8 ± 0.2°, 18.6 ± 0.2°, 19.8 ± 0.2°, 20.2 ± 0.2°;
or 12.8 ± 0.2°, 22.5 ± 0.2°, 18.6 ± 0.2°, 19.8 ± 0.2°, 20.2 ± 0.2°;
or 9.3 ± 0.2°, 10.0 ± 0.2°, 16.8 ± 0.2°, 18.6 ± 0.2°, 19.8 ± 0.2°, 20.2 ± 0.2°;
or 10.0 ± 0.2°, 12.0 ± 0.2°, 16.8 ± 0.2°, 18.6 ± 0.2°, 19.8 ± 0.2°, 20.2 ± 0.2°;
or 12.0 ± 0.2°, 12.8 ± 0.2°, 16.8 ± 0.2°, 18.6 ± 0.2°, 19.8 ± 0.2°, 20.2 ± 0.2°;
or 12.8 ± 0.2°, 22.5 ± 0.2°, 16.8 ± 0.2°, 18.6 ± 0.2°, 19.8 ± 0.2°, 20.2 ± 0.2°;
or 9.3 ± 0.2°, 10.0 ± 0.2°, 12.0 ± 0.2°, 16.8 ± 0.2°, 18.6 ± 0.2°, 19.8 ± 0.2°, 20.2 ± 0.2°;
or 10.0 ± 0.2°, 12.0 ± 0.2°, 12.8 ± 0.2°, 16.8 ± 0.2°, 18.6 ± 0.2°, 19.8 ± 0.2°, 20.2 ± 0.2°;
or 12.0 ± 0.2°, 12.8 ± 0.2°, 22.5 ± 0.2°, 16.8 ± 0.2°, 18.6 ± 0.2°, 19.8 ± 0.2°, 20.2 ± 0.2°;
or 9.3 ± 0.2°, 10.0 ± 0.2°, 12.0 ± 0.2°, 12.8 ± 0.2°, 16.8 ± 0.2°, 18.6 ± 0.2°, 19.8 ± 0.2°, 20.2 ± 0.2°;
or 10.0 ± 0.2°, 12.0 ± 0.2°, 12.8 ± 0.2°, 22.5 ± 0.2°, 16.8 ± 0.2°, 18.6 ± 0.2°, 19.8 ± 0.2°, 20.2 ± 0.2°;
or 9.3 ± 0.2°, 10.0 ± 0.2°, 12.0 ± 0.2°, 12.8 ± 0.2°, 22.5 ± 0.2°, 16.8 ± 0.2°, 18.6 ± 0.2°, 19.8 ± 0.2°, 20.2 ± 0.2°.

Further, in certain embodiments of the present invention, provided is a free base crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof optionally has a diffraction peak at one or more of 2θ of 15.88 ± 0.2°, 24.2 ± 0.2°, 25.4 ± 0.2°, 27.5 ± 0.2°, 27.8 ± 0.2° or 28.9 ± 0.2°; preferably, the X-ray powder diffraction pattern thereof has at least a diffraction peak at any 2 to 4 of the above-mentioned 2θ, or at any 5 or 6 of the above-mentioned 2θ, and further preferably, at any 4 or 6 of the above-mentioned 2θ;
furthermore, in certain embodiments of the present invention, provided is a free base crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

15.8 ± 0.2°, 24.2 ± 0.2°, 25.4 ± 0.2°, 27.5 ± 0.2°;

or 24.2 ± 0.2°, 25.4 ± 0.2°, 27.5 ± 0.2°, 27.8 ± 0.2°;

or 25.4 ± 0.2°, 27.5 ± 0.2°, 27.8 ± 0.2°, 28.9 ± 0.2°;

or 15.8 ± 0.2°, 24.2 ± 0.2°, 27.5 ± 0.2°, 27.8 ± 0.2°;

or 15.8 ± 0.2°, 24.2 ± 0.2°, 27.8 ± 0.2°, 28.9 ± 0.2°;

or 15.8 ± 0.2°, 24.2 ± 0.2°, 25.4 ± 0.2°, 28.9 ± 0.2°;

or 15.8 ± 0.2°, 24.2 ± 0.2°, 25.4 ± 0.2°, 27.5 ± 0.2°, 27.8 ± 0.2° or 28.9 ± 0.2°.

Further, in certain embodiments of the present invention, the X-ray powder diffraction pattern of the free base crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 9.3 ± 0.2°, 10.0 ± 0.2°, 12.0 ± 0.2°, 12.8 ± 0.2°, 22.5 ± 0.2°, 16.8 ± 0.2°, 18.6 ± 0.2°, 19.8 ± 0.2° and 20.2 ± 0.2°, and the crystal form is designated as crystal form A of the compound of formula I.

Further, in certain embodiments of the present invention, the X-ray powder diffraction pattern of the crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone further has a peak at diffraction angles (2θ) of 15.8 ± 0.2°, 24.2 ± 0.2°, 25.4 ± 0.2°, 27.5 ± 0.2°, 27.8 ± 0.2° and 28.9 ± 0.2°.

Further, in certain embodiments of the present invention, provided is a free base crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein by using Cu-Ka radiation, the X-ray characteristic diffraction peaks as represented by 2θ and interplanar spacing d values are shown in Table 1:

**Table 1**

| Serial No. | XRPD data of free base crystal form A | | | | | |
|---|---|---|---|---|---|---|
| | 2θ (± 0.2°) | d value | Peak height | Proportion (I%) | Area | Proportion (I%) |
| 1 | 9.3 | 9.4522 | 256 | 17.3 | 1349 | 11.1 |
| 2 | 10.0 | 8.8221 | 278 | 18.8 | 1805 | 14.9 |
| 3 | 12.0 | 7.3546 | 491 | 33.2 | 3039 | 25.1 |
| 4 | 12.8 | 6.9373 | 292 | 19.7 | 1835 | 15.2 |
| 5 | 14.4 | 6.1623 | 85 | 5.7 | 442 | 3.7 |
| 6 | 15.8 | 5.6185 | 251 | 16.9 | 1302 | 10.8 |
| 7 | 16.8 | 5.2805 | 1373 | 92.7 | 10656 | 88 |
| 8 | 17.0 | 5.2248 | 523 | 35.3 | 6179 | 51 |
| 9 | 18.6 | 4.772 | 1481 | 100 | 12108 | 100 |
| 10 | 19.2 | 4.6256 | 225 | 15.2 | 1432 | 11.8 |
| 11 | 19.8 | 4.4726 | 1139 | 76.9 | 6910 | 57.1 |
| 12 | 20.2 | 4.3932 | 1086 | 73.3 | 6847 | 56.5 |
| 13 | 21.7 | 4.1002 | 224 | 15.1 | 2805 | 23.2 |
| 14 | 21.8 | 4.0694 | 192 | 13 | 3299 | 27.2 |
| 15 | 22.5 | 3.9542 | 847 | 57.2 | 5881 | 48.6 |
| 16 | 22.8 | 3.8903 | 148 | 10 | 1187 | 9.8 |
| 17 | 24.2 | 3.6705 | 295 | 19.9 | 2104 | 17.4 |
| 18 | 25.4 | 3.5078 | 1150 | 77.7 | 9121 | 75.3 |
| 19 | 27.5 | 3.2367 | 660 | 44.6 | 6072 | 50.1 |
| 20 | 27.8 | 3.2063 | 482 | 32.5 | 5480 | 45.3 |
| 21 | 28.1 | 3.1684 | 130 | 8.8 | 1564 | 12.9 |
| 22 | 28.9 | 3.0873 | 624 | 42.1 | 4952 | 40.9 |

Further, in certain embodiments of the present invention, provided is a free base crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof is basically as shown in FIG. 1, and the DSC pattern thereof is basically as shown in FIG. 7.

In certain embodiments of the present invention, provided is a hydrochloride crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has a diffraction peak at 2θ of 8.3 ± 0.2°, or has a diffraction peak at 2θ of 10.1 ± 0.2°, or has a diffraction peak at 2θ of 15.0 ± 0.2°, or has a diffraction peak at 2θ of 16.0 ± 0.2°, or has a diffraction peak at 2θ of 16.5 ± 0.2°, or has a diffraction peak at 2θ of 17.0 ± 0.2°, or has a diffraction peak at 2θ of 17.7 ± 0.2°, or has a diffraction peak at 2θ of 21.5 ± 0.2°, or has a diffraction peak at 2θ of 22.0 ± 0.2°, or has a diffraction peak at 2θ of 24.4 ± 0.2°, or has a diffraction peak at 2θ of 24.7 ± 0.2°, or has a diffraction peak at 2θ of 27.4 ± 0.2°, or has a diffraction peak at 2θ of 30.9 ± 0.2°, or has a diffraction peak at 2θ of 32.2 ± 0.2°; preferably, the X-ray powder diffraction pattern thereof optionally has a diffraction peak at 2, 4, 6, 8 or 10 of the above-mentioned 2θ.

Furthermore, in certain embodiments of the present invention, provided is a hydrochloride crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a diffraction peak at one or more of 2θ of 8.3 ± 0.2°, 15.0 ± 0.2°, or 16.5 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 10.1 ± 0.2°, 16.0 ± 0.2°, 17.0 ± 0.2°, 17.7 ±

0.2° or 21.5 ± 0.2°, preferably at 2, 3, 4 or 5 of the above-mentioned 2θ;
furthermore, in certain embodiments of the present invention, provided is a hydrochloride crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has at least a peak at following diffraction angles (2θ):

8.3 ± 0.2°, 15.0 ± 0.2°, 10.1 ± 0.2°, 16.0 ± 0.2°;
or 8.3 ± 0.2°, 15.0 ± 0.2°, 10.1 ± 0.2°, 17.0 ± 0.2°;
or 8.3 ± 0.2°, 15.0 ± 0.2°, 10.1 ± 0.2°, 17.7 ± 0.2°;
or 8.3 ± 0.2°, 15.0 ± 0.2°, 10.1 ± 0.2°, 21.5 ± 0.2°;
or 8.3 ± 0.2°, 15.0 ± 0.2°, 16.0 ± 0.2°, 17.0 ± 0.2°;
or 8.3 ± 0.2°, 15.0 ± 0.2°, 16.0 ± 0.2°, 17.7 ± 0.2°;
or 8.3 ± 0.2°, 15.0 ± 0.2°, 16.0 ± 0.2°, 21.5 ± 0.2°;
or 8.3 ± 0.2°, 15.0 ± 0.2°, 17.0 ± 0.2°, 17.7 ± 0.2°;
or 8.3 ± 0.2°, 15.0 ± 0.2°, 17.0 ± 0.2°, 21.5 ± 0.2°;
or 8.3 ± 0.2°, 15.0 ± 0.2°, 16.5 ± 0.2°, 10.1 ± 0.2°, 16.0 ± 0.2°;
or 8.3 ± 0.2°, 15.0 ± 0.2°, 16.5 ± 0.2°, 10.1 ± 0.2°, 17.0 ± 0.2°;
or 8.3 ± 0.2°, 15.0 ± 0.2°, 16.5 ± 0.2°, 10.1 ± 0.2°, 17.7 ± 0.2°;
or 8.3 ± 0.2°, 15.0 ± 0.2°, 16.5 ± 0.2°, 10.1 ± 0.2°, 21.5 ± 0.2°;
or 8.3 ± 0.2°, 15.0 ± 0.2°, 16.5 ± 0.2°, 16.0 ± 0.2°, 17.0 ± 0.2°;
or 8.3 ± 0.2°, 15.0 ± 0.2°, 16.5 ± 0.2°, 16.0 ± 0.2°, 17.7 ± 0.2°;
or 8.3 ± 0.2°, 15.0 ± 0.2°, 16.5 ± 0.2°, 16.0 ± 0.2°, 21.5 ± 0.2°;
or 8.3 ± 0.2°, 15.0 ± 0.2°, 16.5 ± 0.2°, 17.0 ± 0.2°, 17.7 ± 0.2°;
or 8.3 ± 0.2°, 15.0 ± 0.2°, 16.5 ± 0.2°, 17.0 ± 0.2°, 21.5 ± 0.2°;
or 8.3 ± 0.2°, 16.5 ± 0.2°, 10.1 ± 0.2°, 16.0 ± 0.2°, 17.0 ± 0.2°, ;
or 8.3 ± 0.2°, 16.5 ± 0.2°, 10.1 ± 0.2°, 17.0 ± 0.2°, 17.7 ± 0.2°;
or 8.3 ± 0.2°, 16.5 ± 0.2°, 10.1 ± 0.2°, 17.7 ± 0.2°, 21.5 ± 0.2°;
or 8.3 ± 0.2°, 16.5 ± 0.2°, 10.1 ± 0.2°, 16.0 ± 0.2°, 21.5 ± 0.2°;
or 8.3 ± 0.2°, 16.5 ± 0.2°, 16.0 ± 0.2°, 17.0 ± 0.2°, 17.7 ± 0.2°;
or 8.3 ± 0.2°, 16.5 ± 0.2°, 16.0 ± 0.2°, 17.7 ± 0.2°, 21.5 ± 0.2°;
or 8.3 ± 0.2°, 16.5 ± 0.2°, 17.0 ± 0.2°, 17.7 ± 0.2°, 21.5 ± 0.2°;
or 15.0 ± 0.2°, 16.5 ± 0.2°, 10.1 ± 0.2°, 16.0 ± 0.2°, 17.0 ± 0.2°;
or 15.0 ± 0.2°, 16.5 ± 0.2°, 16.0 ± 0.2°, 17.0 ± 0.2°, 17.7 ± 0.2°;
or 15.0 ± 0.2°, 16.5 ± 0.2°, 17.0 ± 0.2°, 17.7 ± 0.2°, 21.5 ± 0.2°;
or 15.0 ± 0.2°, 16.5 ± 0.2°, 10.1 ± 0.2°, 17.0 ± 0.2°, 17.7 ± 0.2°;
or 15.0 ± 0.2°, 16.5 ± 0.2°, 10.1 ± 0.2°, 17.7 ± 0.2°, 21.5 ± 0.2°;
or 15.0 ± 0.2°, 16.5 ± 0.2°, 10.1 ± 0.2°, 16.0 ± 0.2°, 17.7 ± 0.2°;
or 15.0 ± 0.2°, 16.5 ± 0.2°, 10.1 ± 0.2°, 16.0 ± 0.2°, 21.5 ± 0.2°;
or 15.0 ± 0.2°, 16.5 ± 0.2°, 16.0 ± 0.2°, 17.7 ± 0.2°, 21.5 ± 0.2°;
or 8.3 ± 0.2°, 15.0 ± 0.2°, 16.5 ± 0.2°, 10.1 ± 0.2°, 16.0 ± 0.2°, 17.0 ± 0.2°;
or 8.3 ± 0.2°, 15.0 ± 0.2°, 16.5 ± 0.2°, 16.0 ± 0.2°, 17.0 ± 0.2°, 17.7 ± 0.2°;
or 8.3 ± 0.2°, 15.0 ± 0.2°, 16.5 ± 0.2°, 17.0 ± 0.2°, 17.7 ± 0.2°, 21.5 ± 0.2°;
or 8.3 ± 0.2°, 15.0 ± 0.2°, 16.5 ± 0.2°, 10.1 ± 0.2°, 17.0 ± 0.2°, 17.7 ± 0.2°;
or 8.3 ± 0.2°, 15.0 ± 0.2°, 16.5 ± 0.2°, 10.1 ± 0.2°, 17.7 ± 0.2°, 21.5 ± 0.2°;
or 8.3 ± 0.2°, 15.0 ± 0.2°, 16.5 ± 0.2°, 16.0 ± 0.2°, 17.7 ± 0.2°, 21.5 ± 0.2°;
or 8.3 ± 0.2°, 15.0 ± 0.2°, 16.5 ± 0.2°, 10.1 ± 0.2°, 16.0 ± 0.2°, 17.0 ± 0.2°, 17.7 ± 0.2°;
or 8.3 ± 0.2°, 15.0 ± 0.2°, 16.5 ± 0.2°, 16.0 ± 0.2°, 17.0 ± 0.2°, 17.7 ± 0.2°, 21.5 ± 0.2°;
or 8.3 ± 0.2°, 15.0 ± 0.2°, 16.5 ± 0.2°, 10.1 ± 0.2°, 17.0 ± 0.2°, 17.7 ± 0.2° or 21.5 ± 0.2°;
or 8.3 ± 0.2°, 15.0 ± 0.2°, 16.5 ± 0.2°, 10.1 ± 0.2°, 16.0 ± 0.2°, 17.7 ± 0.2°, 21.5 ± 0.2°;
or 8.3 ± 0.2°, 15.0 ± 0.2°, 16.5 ± 0.2°, 10.1 ± 0.2°, 16.0 ± 0.2°, 17.0 ± 0.2°, 21.5 ± 0.2°.

Further, in certain embodiments of the present invention, provided is a hydrochloride crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof optionally has a diffraction peak at one or more of 2θ of 24.4 ± 0.2°, 24.7 ± 0.2°, 25.2 ± 0.2°, 27.4 ± 0.2°, 28.5 ± 0.2°, 30.2 ± 0.2° or 32.2 ± 0.2°; preferably, the X-ray powder diffraction pattern thereof has at least a diffraction peak at any 2 to 4 of the above-mentioned 2θ, or at any 5 or 6 of the above-mentioned 2θ, and further preferably, at any 4 or 6 of the above-mentioned 2θ;
furthermore, in certain embodiments of the present invention, provided is a hydrochloride crystal form of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

24.4 ± 0.2°, 24.7 ± 0.2°, 25.2 ± 0.2°, 27.4 ± 0.2°;

or 24.4 ± 0.2°, 25.2 ± 0.2°, 27.4 ± 0.2°, 28.5 ± 0.2°;

or 24.4 ± 0.2°, 27.4 ± 0.2°, 28.5 ± 0.2°, 30.2 ± 0.2°;

or 24.4 ± 0.2°, 28.5 ± 0.2°, 30.2 ± 0.2°, 32.2 ± 0.2°;

or 24.7 ± 0.2°, 25.2 ± 0.2°, 27.4 ± 0.2°, 28.5 ± 0.2°;

or 24.7 ± 0.2°, 27.4 ± 0.2°, 28.5 ± 0.2°, 30.2 ± 0.2°;

or 24.7 ± 0.2°, 28.5 ± 0.2°, 30.2 ± 0.2°, 32.2 ± 0.2°;

or 25.2 ± 0.2°, 27.4 ± 0.2°, 28.5 ± 0.2°, 30.2 ± 0.2°;

or 25.2 ± 0.2°, 28.5 ± 0.2°, 30.2 ± 0.2°, 32.2 ± 0.2°;

or 27.4 ± 0.2°, 28.5 ± 0.2°, 30.2 ± 0.2°, 32.2 ± 0.2°;

or 24.4 ± 0.2°, 24.7 ± 0.2°, 27.4 ± 0.2°, 28.5 ± 0.2°;

or 24.4 ± 0.2°, 24.7 ± 0.2°, 28.5 ± 0.2°, 30.2 ± 0.2°;

or 24.4 ± 0.2°, 24.7 ± 0.2°, 30.2 ± 0.2°, 32.2 ± 0.2°;

or 24.4 ± 0.2°, 24.7 ± 0.2°, 25.2 ± 0.2°, 27.4 ± 0.2°, 28.5 ± 0.2°, 30.2 ± 0.2°;

or 24.7 ± 0.2°, 25.2 ± 0.2°, 27.4 ± 0.2°, 28.5 ± 0.2°, 30.2 ± 0.2°, 32.2 ± 0.2°;

or 24.4 ± 0.2°, 25.2 ± 0.2°, 27.4 ± 0.2°, 28.5 ± 0.2°, 30.2 ± 0.2°, 32.2 ± 0.2°;

or 24.4 ± 0.2°, 24.7 ± 0.2°, 27.4 ± 0.2°, 28.5 ± 0.2°, 30.2 ± 0.2°, 32.2 ± 0.2°;

or 24.4 ± 0.2°, 24.7 ± 0.2°, 25.2 ± 0.2°, 28.5 ± 0.2°, 30.2 ± 0.2°, 32.2 ± 0.2°;

or 24.4 ± 0.2°, 24.7 ± 0.2°, 25.2 ± 0.2°, 27.4 ± 0.2°, 30.2 ± 0.2°, 32.2 ± 0.2°;

or 24.4 ± 0.2°, 24.7 ± 0.2°, 25.2 ± 0.2°, 27.4 ± 0.2°, 28.5 ± 0.2°, 32.2 ± 0.2°.

Further, in certain embodiments of the present invention, the X-ray powder diffraction pattern of the hydrochloride crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 8.3 ± 0.2°, 10.1 ± 0.2°, 15.0 ± 0.2°, 16.5 ± 0.2°, 10.1 ± 0.2°, 16.0 ± 0.2°, 17.0 ± 0.2° and 21.5 ± 0.2°.

Further, in certain embodiments of the present invention, the X-ray powder diffraction pattern of the hydrochloride crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone further has a peak at diffraction angles (2θ) of 22.0 ± 0.2°, 24.4 ± 0.2°, 24.7 ± 0.2°, 25.2 ± 0.2°, 27.4 ± 0.2°, 28.5 ± 0.2° and 32.2 ± 0.2°.

Further, in certain embodiments of the present invention, provided is a hydrochloride crystal form of the (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein by using Cu-Ka radiation, the X-ray characteristic diffraction peaks as represented by 2θ and interplanar spacing d values are shown in Table 2:

**Table 2**

| Serial No. | XRPD data of hydrochloride | | | | | |
|---|---|---|---|---|---|---|
| | 2θ (± 0.2°) | d value | Peak height | Proportion (I%) | Area | Proportion (I%) |
| 1 | 8.3 | 10.645 | 2030 | 100 | 12346 | 100 |
| 2 | 10.1 | 8.756 | 432 | 21.3 | 2225 | 18 |
| 3 | 13.4 | 6.599 | 203 | 10 | 1469 | 11.9 |
| 4 | 15.0 | 5.906 | 1421 | 70 | 8126 | 65.8 |
| 5 | 15.2 | 5.822 | 135 | 6.7 | 1144 | 9.3 |
| 6 | 16.0 | 5.547 | 257 | 12.7 | 1327 | 10.7 |
| 7 | 16.5 | 5.359 | 1422 | 70 | 8333 | 67.5 |
| 8 | 17.0 | 5.223 | 242 | 11.9 | 1775 | 14.4 |
| 9 | 17.7 | 5.016 | 645 | 31.8 | 3455 | 28 |
| 10 | 19.1 | 4.632 | 139 | 6.8 | 787 | 6.4 |
| 11 | 20.1 | 4.406 | 198 | 9.8 | 1276 | 10.3 |
| 12 | 20.3 | 4.375 | 122 | 6 | 893 | 7.2 |
| 13 | 21.5 | 4.126 | 614 | 30.2 | 4328 | 35.1 |
| 14 | 22.0 | 4.036 | 981 | 48.3 | 6539 | 53 |
| 15 | 22.6 | 3.925 | 175 | 8.6 | 1164 | 9.4 |
| 16 | 24.4 | 3.642 | 209 | 10.3 | 1772 | 14.4 |
| 17 | 24.7 | 3.596 | 226 | 11.1 | 1660 | 13.4 |
| 18 | 25.2 | 3.536 | 474 | 23.3 | 4460 | 36.1 |
| 19 | 26.6 | 3.350 | 111 | 5.5 | 1483 | 12 |
| 20 | 27.4 | 3.251 | 330 | 16.3 | 3718 | 30.1 |
| 21 | 28.3 | 3.148 | 195 | 9.6 | 2527 | 20.5 |
| 22 | 28.5 | 3.124 | 566 | 27.9 | 7526 | 61 |
| 23 | 29.3 | 3.049 | 162 | 8 | 1184 | 9.6 |
| 24 | 30.2 | 2.959 | 326 | 16.1 | 4500 | 36.4 |
| 25 | 30.9 | 2.895 | 124 | 6.1 | 1262 | 10.2 |

Further, in certain embodiments of the present invention, provided is a hydrochloride crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof is basically as shown in FIG. 2.

In certain embodiments of the present invention, provided is a methanesulfonate crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has a diffraction peak at 2θ of 7.1 ± 0.2°, or has a diffraction peak at 2θ of 10.1 ± 0.2°, or has a diffraction peak at 2θ of 10.5 ± 0.2°, or has a diffraction peak at 2θ of 12.0 ± 0.2°, or has a diffraction peak at 2θ of 13.2 ± 0.2°, or has a diffraction peak at 2θ of 16.9 ± 0.2°, or has a diffraction peak at 2θ of 18.7 ± 0.2°, or has a diffraction peak at 2θ of 21.1 ± 0.2°, or has a diffraction peak at 2θ of 16.8 ± 0.2°, or has a diffraction peak at 2θ of 18.6 ± 0.2°, or has a diffraction peak at 2θ of 19.7 ± 0.2°, or has a diffraction peak at 2θ of 21.5 ± 0.2°, or has a diffraction peak at 2θ of 21.7 ± 0.2°, or has a diffraction peak at 2θ of 22.5 ± 0.2°, or has a diffraction peak at 2θ of 23.3 ± 0.2°, or has a diffraction peak at 2θ of 25.5 ± 0.2°, or has a diffraction peak at 2θ of 28.0 ± 0.2°; preferably, the X-ray powder diffraction pattern thereof optionally has a diffraction peak at 2, 4, 6, 8 or 10 of the above-mentioned 2θ.

Further, in certain embodiments of the present invention, provided is a methanesulfonate crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a diffraction peak at one or more of 2θ of 7.1 ± 0.2°, 10.1 ± 0.2°, or 10.5 ± 0.2°; preferably, the X-ray powder diffraction pattern thereof has a diffraction

peak at 2 of the above-mentioned 2θ, more preferably, at 3 of the above-mentioned 2θ; optionally, the X-ray powder diffraction pattern thereof further has a diffraction peak at one or more of 2θ of 12.0 ± 0.2°, 13.2 ± 0.2°, 16.9 ± 0.2°, 18.7 ± 0.2° or 21.1 ± 0.2°, preferably at 2, 3, 4 or 5 of the above-mentioned 2θ;

furthermore, in certain embodiments of the present invention, provided is a methanesulfonate crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has at least a peak at following diffraction angles (2θ):

7.1 ± 0.2°, 10.1 ± 0.2°, 12.0 ± 0.2°, 13.2 ± 0.2°;

or 7.1 ± 0.2°, 10.1 ± 0.2°, 13.2 ± 0.2°, 16.9 ± 0.2°;

or 7.1 ± 0.2°, 10.1 ± 0.2°, 16.9 ± 0.2°, 18.7 ± 0.2°;

or 7.1 ± 0.2°, 10.1 ± 0.2°, 18.7 ± 0.2°, 21.1 ± 0.2°;

or 7.1 ± 0.2°, 10.1 ± 0.2°, 12.0 ± 0.2°, 16.9 ± 0.2°;

or 7.1 ± 0.2°, 10.1 ± 0.2°, 12.0 ± 0.2°, 18.7 ± 0.2°;

or 7.1 ± 0.2°, 10.1 ± 0.2°, 12.0 ± 0.2°, 21.1 ± 0.2°;

or 7.1 ± 0.2°, 10.1 ± 0.2°, 13.2 ± 0.2°, 18.7 ± 0.2°;

or 7.1 ± 0.2°, 10.1 ± 0.2°, 13.2 ± 0.2°, 21.1 ± 0.2°;

or 7.1 ± 0.2°, 10.1 ± 0.2°, 16.9 ± 0.2°, 21.1 ± 0.2°;

or 7.1 ± 0.2°, 10.1 ± 0.2°, 10.5 ± 0.2°, 12.0 ± 0.2°, 13.2 ± 0.2°;

or 7.1 ± 0.2°, 10.1 ± 0.2°, 10.5 ± 0.2°, 13.2 ± 0.2°, 16.9 ± 0.2°;

or 7.1 ± 0.2°, 10.1 ± 0.2°, 10.5 ± 0.2°, 16.9 ± 0.2°, 18.7 ± 0.2°;

or 7.1 ± 0.2°, 10.1 ± 0.2°, 10.5 ± 0.2°, 18.7 ± 0.2°, 21.1 ± 0.2°;

or 7.1 ± 0.2°, 10.1 ± 0.2°, 10.5 ± 0.2°, 12.0 ± 0.2°, 16.9 ± 0.2°;

or 7.1 ± 0.2°, 10.1 ± 0.2°, 10.5 ± 0.2°, 12.0 ± 0.2°, 18.7 ± 0.2°;

or 7.1 ± 0.2°, 10.1 ± 0.2°, 10.5 ± 0.2°, 12.0 ± 0.2°, 21.1 ± 0.2°;

or 7.1 ± 0.2°, 10.1 ± 0.2°, 10.5 ± 0.2°, 13.2 ± 0.2°, 18.7 ± 0.2°;

or 7.1 ± 0.2°, 10.1 ± 0.2°, 10.5 ± 0.2°, 13.2 ± 0.2°, 21.1 ± 0.2°;

or 7.1 ± 0.2°, 10.1 ± 0.2°, 10.5 ± 0.2°, 16.9 ± 0.2°, 21.1 ± 0.2°;

or 7.1 ± 0.2°, 10.1 ± 0.2°, 10.5 ± 0.2°, 12.0 ± 0.2°, 13.2 ± 0.2°, 16.9 ± 0.2°;

or 7.1 ± 0.2°, 10.1 ± 0.2°, 10.5 ± 0.2°, 13.2 ± 0.2°, 16.9 ± 0.2°, 18.7 ± 0.2°;

or 7.1 ± 0.2°, 10.1 ± 0.2°, 10.5 ± 0.2°, 16.9 ± 0.2°, 18.7 ± 0.2°, 21.1 ± 0.2°;

or 7.1 ± 0.2°, 10.1 ± 0.2°, 10.5 ± 0.2°, 12.0 ± 0.2°, 16.9 ± 0.2°, 18.7 ± 0.2°;

or 7.1 ± 0.2°, 10.1 ± 0.2°, 10.5 ± 0.2°, 12.0 ± 0.2°, 18.7 ± 0.2°, 21.1 ± 0.2°;

or 7.1 ± 0.2°, 10.1 ± 0.2°, 10.5 ± 0.2°, 13.2 ± 0.2°, 18.7 ± 0.2°, 21.1 ± 0.2°;

or 7.1 ± 0.2°, 10.1 ± 0.2°, 10.5 ± 0.2°, 12.0 ± 0.2°, 13.2 ± 0.2°, 16.9 ± 0.2°, 18.7 ± 0.2°;

or 7.1 ± 0.2°, 10.1 ± 0.2°, 10.5 ± 0.2°, 13.2 ± 0.2°, 16.9 ± 0.2°, 18.7 ± 0.2°, 21.1 ± 0.2°;

or 7.1 ± 0.2°, 10.1 ± 0.2°, 10.5 ± 0.2°, 12.0 ± 0.2°, 16.9 ± 0.2°, 18.7 ± 0.2°, 21.1 ± 0.2°;

or 7.1 ± 0.2°, 10.1 ± 0.2°, 10.5 ± 0.2°, 12.0 ± 0.2°, 13.2 ± 0.2°, 18.7 ± 0.2°, 21.1 ± 0.2°;

or 7.1 ± 0.2°, 10.1 ± 0.2°, 10.5 ± 0.2°, 12.0 ± 0.2°, 13.2 ± 0.2°, 16.9 ± 0.2°, 18.7 ± 0.2°, 21.1 ± 0.2°.

Further, in certain embodiments of the present invention, provided is a methanesulfonate crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof optionally has a diffraction peak at one or more of 2θ of 16.8 ± 0.2°, 18.6 ± 0.2°, 19.7 ± 0.2°, 21.5 ± 0.2°, 21.7 ± 0.2°, 22.5 ± 0.2°, 23.3 ± 0.2°, 25.5 ± 0.2° or 28.0 ± 0.2°; preferably, the X-ray powder diffraction pattern thereof has at least a diffraction peak at any 2 to 4 of the above-mentioned 2θ, or at any 5 or 6 of the above-mentioned 2θ, or at any 7 to 9 of the above-mentioned 2θ, and further preferably, at any 4, 6 or 8 of the above-mentioned 2θ;
furthermore, in certain embodiments of the present invention, provided is a methanesulfonate crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has at least a peak at following diffraction angles (2θ):

16.8 ± 0.2°, 18.6 ± 0.2°, 19.7 ± 0.2°, 21.5 ± 0.2°;

or 18.6 ± 0.2°, 19.7 ± 0.2°, 21.5 ± 0.2°, 21.7 ± 0.2°;

or 19.7 ± 0.2°, 21.5 ± 0.2°, 21.7 ± 0.2°, 22.5 ± 0.2°;

or 21.5 ± 0.2°, 21.7 ± 0.2°, 22.5 ± 0.2°, 23.3 ± 0.2°;

or 21.7 ± 0.2°, 22.5 ± 0.2°, 23.3 ± 0.2°, 25.5 ± 0.2°;

or 22.5 ± 0.2°, 23.3 ± 0.2°, 25.5 ± 0.2°, 28.0 ± 0.2°;

or 16.8 ± 0.2°, 19.7 ± 0.2°, 21.5 ± 0.2°, 21.7 ± 0.2°;

or 16.8 ± 0.2°, 21.5 ± 0.2°, 21.7 ± 0.2°, 22.5 ± 0.2°;

or 16.8 ± 0.2°, 21.7 ± 0.2°, 22.5 ± 0.2°, 23.3 ± 0.2°;

or 16.8 ± 0.2°, 22.5 ± 0.2°, 23.3 ± 0.2°, 25.5 ± 0.2°;

or 16.8 ± 0.2°, 23.3 ± 0.2°, 25.5 ± 0.2°, 28.0 ± 0.2°;

or 16.8 ± 0.2°, 18.6 ± 0.2°, 21.5 ± 0.2°, 21.7 ± 0.2°;

or 16.8 ± 0.2°, 18.6 ± 0.2°, 21.7 ± 0.2°, 22.5 ± 0.2°;

or 16.8 ± 0.2°, 18.6 ± 0.2°, 22.5 ± 0.2°, 23.3 ± 0.2°;

or 16.8 ± 0.2°, 18.6 ± 0.2°, 23.3 ± 0.2°, 25.5 ± 0.2°;

or 16.8 ± 0.2°, 18.6 ± 0.2°, 25.5 ± 0.2°, 28.0 ± 0.2°;

or 16.8 ± 0.2°, 18.6 ± 0.2°, 19.7 ± 0.2°, 21.7 ± 0.2°;

or 16.8 ± 0.2°, 18.6 ± 0.2°, 19.7 ± 0.2°, 22.5 ± 0.2°;

or 16.8 ± 0.2°, 18.6 ± 0.2°, 19.7 ± 0.2°, 23.3 ± 0.2°;

or 16.8 ± 0.2°, 18.6 ± 0.2°, 19.7 ± 0.2°, 25.5 ± 0.2°;

or 16.8 ± 0.2°, 18.6 ± 0.2°, 19.7 ± 0.2°, 28.0 ± 0.2°;

or 18.6 ± 0.2°, 19.7 ± 0.2°, 21.7 ± 0.2°, 22.5 ± 0.2°;

or 18.6 ± 0.2°, 19.7 ± 0.2°, 22.5 ± 0.2°, 23.3 ± 0.2°;

or 18.6 ± 0.2°, 19.7 ± 0.2°, 23.3 ± 0.2°, 25.5 ± 0.2°;

or 18.6 ± 0.2°, 19.7 ± 0.2°, 25.5 ± 0.2°, 28.0 ± 0.2°;

or 19.7 ± 0.2°, 21.5 ± 0.2°, 22.5 ± 0.2°, 23.3 ± 0.2°;

or 19.7 ± 0.2°, 21.5 ± 0.2°, 23.3 ± 0.2°, 25.5 ± 0.2°;

or 19.7 ± 0.2°, 21.5 ± 0.2°, 25.5 ± 0.2°, 28.0 ± 0.2°;

or 21.5 ± 0.2°, 21.7 ± 0.2°, 23.3 ± 0.2°, 25.5 ± 0.2°;

or 21.5 ± 0.2°, 21.7 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 21.7 ± 0.2°, 22.5 ± 0.2°;

or 18.6 ± 0.2°, 19.7 ± 0.2°, 21.5 ± 0.2°, 21.7 ± 0.2°, 22.5 ± 0.2°, 23.3 ± 0.2°;

or 19.7 ± 0.2°, 21.5 ± 0.2°, 21.7 ± 0.2°, 22.5 ± 0.2°, 23.3 ± 0.2°, 25.5 ± 0.2°;

or 21.5 ± 0.2°, 21.7 ± 0.2°, 22.5 ± 0.2°, 23.3 ± 0.2°, 25.5 ± 0.2°, 28.0 ± 0.2°;

or 16.8 ± 0.2°, 19.7 ± 0.2°, 21.5 ± 0.2°, 21.7 ± 0.2°, 22.5 ± 0.2°, 23.3 ± 0.2°;

or 16.8 ± 0.2°, 21.5 ± 0.2°, 21.7 ± 0.2°, 22.5 ± 0.2°, 23.3 ± 0.2°, 25.5 ± 0.2°;

or 16.8 ± 0.2°, 21.7 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 21.5 ± 0.2°, 21.7 ± 0.2°, 22.5 ± 0.2°, 23.3 ± 0.2°;

or 16.8 ± 0.2°, 18.6 ± 0.2°, 21.7 ± 0.2°, 22.5 ± 0.2°, 23.3 ± 0.2°, 25.5 ± 0.2°;

or 16.8 ± 0.2°, 18.6 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.7 ± 0.2°, 22.5 ± 0.2°, 23.3 ± 0.2°;

or 16.8 ± 0.2°, 18.6 ± 0.2°, 19.7 ± 0.2°, 22.5 ± 0.2°, 23.3 ± 0.2°, 25.5 ± 0.2°;

or 16.8 ± 0.2°, 18.6 ± 0.2°, 19.7 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 22.5 ± 0.2°, 23.3 ± 0.2°;

or 16.8 ± 0.2°, 18.6 ± 0.2°, 19.7 ± 0.2°, 21.5 ± 0.2°, 23.3 ± 0.2°, 25.5 ± 0.2°;

or 16.8 ± 0.2°, 18.6 ± 0.2°, 19.7 ± 0.2°, 21.5 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 21.7 ± 0.2°, 23.3 ± 0.2°;

or 16.8 ± 0.2°, 18.6 ± 0.2°, 19.7 ± 0.2°, 21.5 ± 0.2°, 21.7 ± 0.2°, 25.5 ± 0.2°;

or 16.8 ± 0.2°, 18.6 ± 0.2°, 19.7 ± 0.2°, 21.5 ± 0.2°, 21.7 ± 0.2°, 28.0 ± 0.2°;

or 18.6 ± 0.2°, 19.7 ± 0.2°, 21.7 ± 0.2°, 22.5 ± 0.2°, 23.3 ± 0.2°, 25.5 ± 0.2°;

or 18.6 ± 0.2°, 19.7 ± 0.2°, 22.5 ± 0.2°, 23.3 ± 0.2°, 25.5 ± 0.2°, 28.0 ± 0.2°;

or 19.7 ± 0.2°, 21.5 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 21.7 ± 0.2°, 22.5 ± 0.2°, 23.3 ± 0.2°, 25.5 ± 0.2°;

or 18.6 ± 0.2°, 19.7 ± 0.2°, 21.5 ± 0.2°, 21.7 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 21.5 ± 0.2°, 21.7 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.7 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 22.5 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 21.7 ± 0.2°, 23.3 ± 0.2°, 25.5 ± 0.2°, 28.0 ± 0.2°;

or 16.8 ± 0.2°, 18.6 ± 0.2°, 19.7 ± 0.2°, 21.5 ± 0.2°, 21.7 ± 0.2°, 22.5 ± 0.2°, 25.5 ± 0.2°, 28.0 ± 0.2°.

Further, in certain embodiments of the present invention, the X-ray powder diffraction pattern of the methanesulfonate crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 7.1 ± 0.2°, 10.1 ± 0.2°, 10.5 ± 0.2°, 12.0 ± 0.2°, 13.2 ± 0.2°, 16.9 ± 0.2°, 18.7 ± 0.2°, 21.1 ± 0.2°, 16.8 ± 0.2° and 18.6 ± 0.2°.

Further, in certain embodiments of the present invention, the X-ray powder diffraction pattern of the methanesulfonate crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone further has a peak at diffraction angles (2θ) of 19.7 ± 0.2°, 21.5 ± 0.2°, 21.7 ± 0.2°, 22.5 ± 0.2°, 25.5 ± 0.2° and 28.0 ± 0.2°.

Further, in certain embodiments of the present invention, provided is a methanesulfonate crystal form of the (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein by using Cu-Ka radiation, the X-ray characteristic diffraction peaks as represented by 2θ and interplanar spacing d values are shown in Table 3:

**Table 3**

| Serial No. | XRPD data of methanesulfonate crystal form A | | | | | |
|---|---|---|---|---|---|---|
| | 2θ (± 0.2°) | d value | Peak height | Proportion (I%) | Area | Proportion (I%) |
| 1 | 7.1 | 12.3691 | 335 | 22.3 | 1447 | 13.3 |
| 2 | 10.1 | 8.7122 | 1503 | 100 | 7661 | 70.6 |
| 3 | 10.5 | 8.4458 | 695 | 46.2 | 3751 | 34.6 |
| 4 | 12.0 | 7.3657 | 224 | 14.9 | 1372 | 12.7 |
| 5 | 13.2 | 6.7084 | 304 | 20.2 | 1291 | 11.9 |
| 6 | 16.8 | 5.2852 | 381 | 25.3 | 3634 | 33.5 |
| 7 | 16.9 | 5.236 | 664 | 44.2 | 5777 | 53.3 |
| 8 | 18.4 | 4.8145 | 160 | 10.6 | 690 | 6.4 |
| 9 | 18.6 | 4.7763 | 394 | 26.2 | 3534 | 32.6 |
| 10 | 18.7 | 4.7323 | 304 | 20.2 | 3479 | 32.1 |
| 11 | 19.7 | 4.5136 | 744 | 49.5 | 4465 | 41.2 |
| 12 | 19.8 | 4.4773 | 213 | 14.2 | 1777 | 16.4 |
| 13 | 20.2 | 4.3972 | 246 | 16.4 | 1531 | 14.1 |

(continued)

| Serial No. | XRPD data of methanesulfonate crystal form A | | | | | |
|---|---|---|---|---|---|---|
| | 2θ (± 0.2°) | d value | Peak height | Proportion (I%) | Area | Proportion (I%) |
| 14 | 20.7 | 4.2854 | 331 | 22 | 1294 | 11.9 |
| 15 | 21.1 | 4.2041 | 1045 | 69.5 | 8913 | 82.2 |
| 16 | 21.5 | 4.1301 | 656 | 43.6 | 5261 | 48.5 |
| 17 | 21.7 | 4.0841 | 1030 | 68.5 | 5779 | 53.3 |
| 18 | 22.5 | 3.9504 | 551 | 36.7 | 3130 | 28.9 |
| 19 | 23.3 | 3.8136 | 501 | 33.3 | 2671 | 24.6 |
| 20 | 25.5 | 3.4853 | 1454 | 96.7 | 10845 | 100 |
| 21 | 28.0 | 3.1836 | 672 | 44.7 | 4900 | 45.2 |

Further, in certain embodiments of the present invention, provided is a methanesulfonate crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof is basically as shown in FIG. 3.

In certain embodiments of the present invention, provided is a nitrate crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has a diffraction peak at 2θ of 9.3 ± 0.2°, or has a diffraction peak at 2θ of 11.9 ± 0.2°, or has a diffraction peak at 2θ of 12.5 ± 0.2°, or has a diffraction peak at 2θ of 14.3 ± 0.2°, or has a diffraction peak at 2θ of 14.6 ± 0.2°, or has a diffraction peak at 2θ of 16.2 ± 0.2°, or has a diffraction peak at 2θ of 16.7 ± 0.2°, or has a diffraction peak at 2θ of 17.9 ± 0.2°, or has a diffraction peak at 2θ of 18.7 ± 0.2°, or has a diffraction peak at 2θ of 22.9 ± 0.2°, or has a diffraction peak at 2θ of 25.1 ± 0.2°, or has a diffraction peak at 2θ of 25.9 ± 0.2°, or has a diffraction peak at 2θ of 26.6 ± 0.2°, or has a diffraction peak at 2θ of 28.2 ± 0.2°, or has a diffraction peak at 2θ of 28.6 ± 0.2°; preferably, the X-ray powder diffraction pattern thereof optionally has a diffraction peak at 2, 4, 6, 8 or 10 of the above-mentioned 2θ.

Further, in certain embodiments of the present invention, provided is a nitrate crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, wherein the X-ray powder diffraction pattern thereof has at least a diffraction peak at one or more of 2θ of 9.3 ± 0.2°, 11.9 ± 0.2°, or 12.5 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 14.3 ± 0.2°, 14.6 ± 0.2°, 16.2 ± 0.2°, 16.7 ± 0.2° or 17.9 ± 0.2°, preferably at 2, 3, 4 or 5 of the above-mentioned 2θ;

furthermore, in certain embodiments of the present invention, provided is a nitrate crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

9.3 ± 0.2°, 11.9 ± 0.2°, 14.3 ± 0.2°, 14.6 ± 0.2°;
or 9.3 ± 0.2°, 11.9 ± 0.2°, 14.6 ± 0.2°, 16.2 ± 0.2°;
or 9.3 ± 0.2°, 11.9 ± 0.2°, 16.2 ± 0.2°, 16.7 ± 0.2°;
or 9.3 ± 0.2°, 11.9 ± 0.2°, 16.7 ± 0.2°, 17.9 ± 0.2°;
or 9.3 ± 0.2°, 11.9 ± 0.2°, 14.3 ± 0.2°, 16.2 ± 0.2°;
or 9.3 ± 0.2°, 11.9 ± 0.2°, 14.3 ± 0.2°, 16.7 ± 0.2°;
or 9.3 ± 0.2°, 11.9 ± 0.2°, 14.3 ± 0.2°, 17.9 ± 0.2°;
or 9.3 ± 0.2°, 11.9 ± 0.2°, 14.6 ± 0.2°, 16.7 ± 0.2°;
or 9.3 ± 0.2°, 11.9 ± 0.2°, 14.6 ± 0.2°, 17.9 ± 0.2°;
or 9.3 ± 0.2°, 11.9 ± 0.2°, 16.2 ± 0.2°, 17.9 ± 0.2°;
or 9.3 ± 0.2°, 11.9 ± 0.2°, 12.5 ± 0.2°, 14.3 ± 0.2°, 14.6 ± 0.2°;
or 9.3 ± 0.2°, 11.9 ± 0.2°, 12.5 ± 0.2°, 14.6 ± 0.2°, 16.2 ± 0.2°;
or 9.3 ± 0.2°, 11.9 ± 0.2°, 12.5 ± 0.2°, 16.2 ± 0.2°, 16.7 ± 0.2°;
or 9.3 ± 0.2°, 11.9 ± 0.2°, 12.5 ± 0.2°, 16.7 ± 0.2°, 17.9 ± 0.2°;
or 9.3 ± 0.2°, 11.9 ± 0.2°, 12.5 ± 0.2°, 14.3 ± 0.2°, 16.2 ± 0.2°;
or 9.3 ± 0.2°, 11.9 ± 0.2°, 12.5 ± 0.2°, 14.3 ± 0.2°, 16.7 ± 0.2°;
or 9.3 ± 0.2°, 11.9 ± 0.2°, 12.5 ± 0.2°, 14.3 ± 0.2°, 17.9 ± 0.2°;

or 9.3 ± 0.2°, 11.9 ± 0.2°, 12.5 ± 0.2°, 14.6 ± 0.2°, 16.7 ± 0.2°;
or 9.3 ± 0.2°, 11.9 ± 0.2°, 12.5 ± 0.2°, 14.6 ± 0.2°, 17.9 ± 0.2°;
or 9.3 ± 0.2°, 11.9 ± 0.2°, 12.5 ± 0.2°, 16.2 ± 0.2°, 17.9 ± 0.2°;
or 9.3 ± 0.2°, 11.9 ± 0.2°, 12.5 ± 0.2°, 14.3 ± 0.2°, 14.6 ± 0.2°, 16.2 ± 0.2°;
or 9.3 ± 0.2°, 11.9 ± 0.2°, 12.5 ± 0.2°, 14.6 ± 0.2°, 16.2 ± 0.2°, 16.7 ± 0.2°;
or 9.3 ± 0.2°, 11.9 ± 0.2°, 12.5 ± 0.2°, 16.2 ± 0.2°, 16.7 ± 0.2°, 17.9 ± 0.2°;
or 9.3 ± 0.2°, 11.9 ± 0.2°, 12.5 ± 0.2°, 14.3 ± 0.2°, 16.2 ± 0.2°, 16.7 ± 0.2°;
or 9.3 ± 0.2°, 11.9 ± 0.2°, 12.5 ± 0.2°, 14.3 ± 0.2°, 16.7 ± 0.2°, 17.9 ± 0.2°;
or 9.3 ± 0.2°, 11.9 ± 0.2°, 12.5 ± 0.2°, 14.6 ± 0.2°, 16.7 ± 0.2°, 17.9 ± 0.2°;
or 9.3 ± 0.2°, 11.9 ± 0.2°, 12.5 ± 0.2°, 14.3 ± 0.2°, 14.6 ± 0.2°, 16.7 ± 0.2°;
or 9.3 ± 0.2°, 11.9 ± 0.2°, 12.5 ± 0.2°, 14.3 ± 0.2°, 14.6 ± 0.2°, 17.9 ± 0.2°;
or 9.3 ± 0.2°, 11.9 ± 0.2°, 12.5 ± 0.2°, 14.3 ± 0.2°, 14.6 ± 0.2°, 16.2 ± 0.2°, 16.7 ± 0.2°;
or 9.3 ± 0.2°, 11.9 ± 0.2°, 12.5 ± 0.2°, 14.6 ± 0.2°, 16.2 ± 0.2°, 16.7 ± 0.2°, 17.9 ± 0.2°;
or 9.3 ± 0.2°, 11.9 ± 0.2°, 12.5 ± 0.2°, 14.3 ± 0.2°, 16.2 ± 0.2°, 16.7 ± 0.2°, 17.9 ± 0.2°;
or 9.3 ± 0.2°, 11.9 ± 0.2°, 12.5 ± 0.2°, 14.3 ± 0.2°, 14.6 ± 0.2°, 16.7 ± 0.2° or 17.9 ± 0.2°;
or 9.3 ± 0.2°, 11.9 ± 0.2°, 12.5 ± 0.2°, 14.3 ± 0.2°, 14.6 ± 0.2°, 16.2 ± 0.2°, 17.9 ± 0.2°;
or 9.3 ± 0.2°, 11.9 ± 0.2°, 12.5 ± 0.2°, 14.3 ± 0.2°, 14.6 ± 0.2°, 16.2 ± 0.2°, 16.7 ± 0.2°, 17.9 ± 0.2°.

Further, in certain embodiments of the present invention, provided is a nitrate crystal form of the compound of formula I, (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof optionally has a diffraction peak at one or more of 2θ of 18.7 ± 0.2°, 22.9 ± 0.2°, 25.1 ± 0.2°, 25.9 ± 0.2°, 26.6 ± 0.2°, 28.2 ± 0.2° or 28.6 ± 0.2°; preferably, the X-ray powder diffraction pattern thereof has at least a diffraction peak at any 2 to 4 of the above-mentioned 2θ, or at any 5 to 7 of the above-mentioned 2θ, and further preferably, at any 4 or 6 of the above-mentioned 2θ;
furthermore, in certain embodiments of the present invention, provided is a nitrate crystal form of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

18.7 ± 0.2°, 22.9 ± 0.2°, 25.1 ± 0.2°, 25.9 ± 0.2°;

or 18.7 ± 0.2°, 25.1 ± 0.2°, 25.9 ± 0.2°, 26.6 ± 0.2°;

or 18.7 ± 0.2°, 25.9 ± 0.2°, 26.6 ± 0.2°, 28.2 ± 0.2°;

or 18.7 ± 0.2°, 26.6 ± 0.2°, 28.2 ± 0.2°, 28.6 ± 0.2°;

or 18.7 ± 0.2°, 22.9 ± 0.2°, 25.9 ± 0.2°, 26.6 ± 0.2°;

or 18.7 ± 0.2°, 22.9 ± 0.2°, 26.6 ± 0.2°, 28.2 ± 0.2°;

or 18.7 ± 0.2°, 22.9 ± 0.2°, 28.2 ± 0.2°, 28.6 ± 0.2°;

or 18.7 ± 0.2°, 22.9 ± 0.2°, 25.1 ± 0.2°, 26.6 ± 0.2°;

or 18.7 ± 0.2°, 22.9 ± 0.2°, 25.1 ± 0.2°, 28.2 ± 0.2°;

or 18.7 ± 0.2°, 22.9 ± 0.2°, 25.1 ± 0.2°, 28.6 ± 0.2°;

or 22.9 ± 0.2°, 25.1 ± 0.2°, 25.9 ± 0.2°, 26.6 ± 0.2°;

or 22.9 ± 0.2°, 25.9 ± 0.2°, 26.6 ± 0.2°, 28.2 ± 0.2°;

or 22.9 ± 0.2°, 26.6 ± 0.2°, 28.2 ± 0.2°, 28.6 ± 0.2°;

or 25.1 ± 0.2°, 25.9 ± 0.2°, 26.6 ± 0.2°, 28.2 ± 0.2°;

or 25.1 ± 0.2°, 26.6 ± 0.2°, 28.2 ± 0.2°, 28.6 ± 0.2°;

or 25.9 ± 0.2°, 26.6 ± 0.2°, 28.2 ± 0.2°, 28.6 ± 0.2°;

or 18.7 ± 0.2°, 22.9 ± 0.2°, 25.1 ± 0.2°, 25.9 ± 0.2°, 26.6 ± 0.2°, 28.2 ± 0.2°;

or 22.9 ± 0.2°, 25.1 ± 0.2°, 25.9 ± 0.2°, 26.6 ± 0.2°, 28.2 ± 0.2°, 28.6 ± 0.2°;

or 18.7 ± 0.2°, 25.1 ± 0.2°, 25.9 ± 0.2°, 26.6 ± 0.2°, 28.2 ± 0.2°, 28.6 ± 0.2°;

or 18.7 ± 0.2°, 22.9 ± 0.2°, 25.9 ± 0.2°, 26.6 ± 0.2°, 28.2 ± 0.2°, 28.6 ± 0.2°;

or 18.7 ± 0.2°, 22.9 ± 0.2°, 25.1 ± 0.2°, 26.6 ± 0.2°, 28.2 ± 0.2°, 28.6 ± 0.2°;

or 18.7 ± 0.2°, 22.9 ± 0.2°, 25.1 ± 0.2°, 25.9 ± 0.2°, 28.2 ± 0.2°, 28.6 ± 0.2°;

or 18.7 ± 0.2°, 22.9 ± 0.2°, 25.1 ± 0.2°, 25.9 ± 0.2°, 26.6 ± 0.2°, 28.6 ± 0.2°.

Further, in certain embodiments of the present invention, the X-ray powder diffraction pattern of the free base crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 9.3 ± 0.2°, 11.9 ± 0.2°, 12.5 ± 0.2°, 14.3 ± 0.2°, 14.6 ± 0.2°, 16.2 ± 0.2°, 16.7 ± 0.2°, 17.9 ± 0.2° and 18.7 ± 0.2°.

Further, in certain embodiments of the present invention, the X-ray powder diffraction pattern of the crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone further has a peak at diffraction angles (2θ) of 22.9 ± 0.2°, 25.1 ± 0.2°, 25.9 ± 0.2°, 26.6 ± 0.2°, 28.2 ± 0.2° and 28.6 ± 0.2°.

Further, in certain embodiments of the present invention, provided is a free base crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein by using Cu-Ka radiation, the X-ray characteristic diffraction peaks as represented by 2θ and interplanar spacing d values are shown in Table 4:

**Table 4**

| Serial | XRPD data of nitrate crystal form A | | | | | |
|---|---|---|---|---|---|---|
| No. | 2θ (± 0.2°) | d value | Peak height | Proportion (I%) | Area | Proportion (I%) |
| 1 | 9.3 | 9.5368 | 624 | 20.8 | 3376 | 20.3 |
| 2 | 10.3 | 8.5677 | 148.0 | 4.9 | 829 | 5 |
| 3 | 11.9 | 7.401 | 457 | 15.2 | 3036 | 18.3 |
| 4 | 12.5 | 7.0899 | 3000 | 100 | 16600 | 100 |
| 5 | 14.3 | 6.1826 | 1180.0 | 39.3 | 6293 | 37.9 |
| 6 | 14.6 | 6.0 | 553 | 18.4 | 3276 | 19.7 |
| 7 | 16.2 | 5.4733 | 459 | 15.3 | 2864 | 17.3 |
| 8 | 16.7 | 5.3074 | 765 | 25.5 | 6418 | 38.7 |
| 9 | 17.9 | 4.9606 | 635 | 21.2 | 3340 | 20.1 |
| 10 | 18.7 | 4.7321 | 1420 | 47.3 | 9975 | 60.1 |
| 11 | 19.5 | 4.5419 | 224 | 7.5 | 1377 | 8.3 |
| 12 | 22.9 | 3.8848 | 1772 | 59.1 | 14031 | 84.5 |
| 13 | 23.7 | 3.7588 | 170 | 5.7 | 1088 | 6.6 |
| 14 | 24.2 | 3.6696 | 112 | 3.7 | 948 | 5.7 |
| 15 | 24.5 | 3.6361 | 108 | 3.6 | 1136 | 6.8 |
| 16 | 24.6 | 3.6162 | 149 | 5 | 1484 | 8.9 |
| 17 | 25.1 | 3.6 | 261 | 8.7 | 1653 | 10 |
| 18 | 25.9 | 3.4328 | 368 | 12.3 | 2335 | 14.1 |

(continued)

| Serial | XRPD data of nitrate crystal form A | | | | | |
|---|---|---|---|---|---|---|
| No. | 2θ (± 0.2°) | d value | Peak height | Proportion (I%) | Area | Proportion (I%) |
| 19 | 26.6 | 3.3449 | 1907 | 63.6 | 13947 | 84 |
| 20 | 27.6 | 3.2266 | 347 | 11.6 | 3185 | 19.2 |
| 21 | 28.2 | 3.1586 | 485 | 16.2 | 5069 | 30.5 |
| 22 | 28.6 | 3.1173 | 425 | 14.2 | 4821 | 29 |

Further, in certain embodiments of the present invention, provided is a nitrate crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof is basically as shown in FIG. 4.

In certain embodiments of the present invention, provided is a sulfate crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has a diffraction peak at 2θ of 7.1 ± 0.2°, or has a diffraction peak at 2θ of 13.4 ± 0.2°, or has a diffraction peak at 2θ of 14.2 ± 0.2°, or has a diffraction peak at 2θ of 15.5 ± 0.2°, or has a diffraction peak at 2θ of 16.3 ± 0.2°, or has a diffraction peak at 2θ of 17.4 ± 0.2°, or has a diffraction peak at 2θ of 17.7°, or has a diffraction peak at 2θ of 18.0 ± 0.2°, or has a diffraction peak at 2θ of 19.8 ± 0.2°, or has a diffraction peak at 2θ of 20.1 ± 0.2°; preferably, the X-ray powder diffraction pattern thereof optionally has a diffraction peak at 2, 4, 6, 8 or 10 of the above-mentioned 2θ.

Further, in certain embodiments of the present invention, provided is a sulfate crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a diffraction peak at one or more of 2θ of 7.1 ± 0.2°, 16.3 ± 0.2°, or 18.0 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 13.4 ± 0.2°, 14.2 ± 0.2°, 15.5 ± 0.2°, 17.4 ± 0.2° or 17.7 ± 0.2°, preferably at 2, 3, 4 or 5 of the above-mentioned 2θ;

furthermore, in certain embodiments of the present invention, provided is a sulfate crystal form of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

7.1 ± 0.2°, 16.3 ± 0.2°, 13.4 ± 0.2°, 14.2 ± 0.2°;
or 7.1 ± 0.2°, 16.3 ± 0.2°, 14.2 ± 0.2°, 15.5 ± 0.2°;
or 7.1 ± 0.2°, 16.3 ± 0.2°, 15.5 ± 0.2°, 17.4 ± 0.2°;
or 7.1 ± 0.2°, 16.3 ± 0.2°, 17.4 ± 0.2°, 17.7 ± 0.2°;
or 7.1 ± 0.2°, 16.3 ± 0.2°, 13.4 ± 0.2°, 15.5 ± 0.2°;
or 7.1 ± 0.2°, 16.3 ± 0.2°, 13.4 ± 0.2°, 17.4 ± 0.2°;
or 7.1 ± 0.2°, 16.3 ± 0.2°, 13.4 ± 0.2°, 17.7 ± 0.2°;
or 7.1 ± 0.2°, 16.3 ± 0.2°, 14.2 ± 0.2°, 17.4 ± 0.2°;
or 7.1 ± 0.2°, 16.3 ± 0.2°, 14.2 ± 0.2°, 17.7 ± 0.2°;
or 7.1 ± 0.2°, 16.3 ± 0.2°, 15.5 ± 0.2°, 17.7 ± 0.2°;
or 7.1 ± 0.2°, 13.4 ± 0.2°, 14.2 ± 0.2°, 18.0 ± 0.2°;
or 7.1 ± 0.2°, 14.2 ± 0.2°, 15.5 ± 0.2°, 18.0 ± 0.2°;
or 7.1 ± 0.2°, 15.5 ± 0.2°, 17.4 ± 0.2°, 18.0 ± 0.2°;
or 7.1 ± 0.2°, 17.4 ± 0.2°, 17.7 ± 0.2°, 18.0 ± 0.2°;
or 7.1 ± 0.2°, 13.4 ± 0.2°, 15.5 ± 0.2°, 18.0 ± 0.2°;
or 7.1 ± 0.2°, 13.4 ± 0.2°, 17.4 ± 0.2°, 18.0 ± 0.2°;
or 7.1 ± 0.2°, 13.4 ± 0.2°, 17.7 ± 0.2°, 18.0 ± 0.2°;
or 7.1 ± 0.2°, 14.2 ± 0.2°, 17.4 ± 0.2°, 18.0 ± 0.2°;
or 7.1 ± 0.2°, 14.2 ± 0.2°, 17.7 ± 0.2°, 18.0 ± 0.2°;
or 7.1 ± 0.2°, 15.5 ± 0.2°, 17.7 ± 0.2°, 18.0 ± 0.2°;
or 16.3 ± 0.2°, 13.4 ± 0.2°, 14.2 ± 0.2°, 18.0 ± 0.2°;
or 16.3 ± 0.2°, 14.2 ± 0.2°, 15.5 ± 0.2°, 18.0 ± 0.2°;
or 16.3 ± 0.2°, 15.5 ± 0.2°, 17.4 ± 0.2°, 18.0 ± 0.2°;
or 16.3 ± 0.2°, 17.4 ± 0.2°, 17.7 ± 0.2°, 18.0 ± 0.2°;
or 16.3 ± 0.2°, 13.4 ± 0.2°, 15.5 ± 0.2°, 18.0 ± 0.2°;

or 16.3 ± 0.2°, 13.4 ± 0.2°, 17.4 ± 0.2°, 18.0 ± 0.2°;
or 16.3 ± 0.2°, 13.4 ± 0.2°, 17.7 ± 0.2°, 18.0 ± 0.2°;
or 16.3 ± 0.2°, 14.2 ± 0.2°, 17.4 ± 0.2°, 18.0 ± 0.2°;
or 16.3 ± 0.2°, 14.2 ± 0.2°, 17.7 ± 0.2°, 18.0 ± 0.2°;
or 16.3 ± 0.2°, 15.5 ± 0.2°, 17.7 ± 0.2°, 18.0 ± 0.2°;
or 7.1 ± 0.2°, 16.3 ± 0.2°, 13.4 ± 0.2°, 14.2 ± 0.2°, 18.0 ± 0.2°;
or 7.1 ± 0.2°, 16.3 ± 0.2°, 14.2 ± 0.2°, 15.5 ± 0.2°, 18.0 ± 0.2°;
or 7.1 ± 0.2°, 16.3 ± 0.2°, 15.5 ± 0.2°, 17.4 ± 0.2°, 18.0 ± 0.2°;
or 7.1 ± 0.2°, 16.3 ± 0.2°, 17.4 ± 0.2°, 17.7 ± 0.2°, 18.0 ± 0.2°;
or 7.1 ± 0.2°, 16.3 ± 0.2°, 13.4 ± 0.2°, 15.5 ± 0.2°, 18.0 ± 0.2°;
or 7.1 ± 0.2°, 16.3 ± 0.2°, 13.4 ± 0.2°, 17.4 ± 0.2°, 18.0 ± 0.2°;
or 7.1 ± 0.2°, 16.3 ± 0.2°, 13.4 ± 0.2°, 17.7 ± 0.2°, 18.0 ± 0.2°;
or 7.1 ± 0.2°, 16.3 ± 0.2°, 14.2 ± 0.2°, 17.4 ± 0.2°, 18.0 ± 0.2°;
or 7.1 ± 0.2°, 16.3 ± 0.2°, 14.2 ± 0.2°, 17.7 ± 0.2°, 18.0 ± 0.2°;
or 7.1 ± 0.2°, 16.3 ± 0.2°, 15.5 ± 0.2°, 17.7 ± 0.2°, 18.0 ± 0.2°;
or 7.1 ± 0.2°, 16.3 ± 0.2°, 13.4 ± 0.2°, 14.2 ± 0.2°, 15.5 ± 0.2°, 18.0 ± 0.2°;
or 7.1 ± 0.2°, 16.3 ± 0.2°, 14.2 ± 0.2°, 15.5 ± 0.2°, 17.4 ± 0.2°, 18.0 ± 0.2°;
or 7.1 ± 0.2°, 16.3 ± 0.2°, 15.5 ± 0.2°, 17.4 ± 0.2°, 17.7 ± 0.2°, 18.0 ± 0.2°;
or 7.1 ± 0.2°, 16.3 ± 0.2°, 13.4 ± 0.2°, 15.5 ± 0.2°, 17.4 ± 0.2°, 18.0 ± 0.2°;
or 7.1 ± 0.2°, 16.3 ± 0.2°, 13.4 ± 0.2°, 15.5 ± 0.2°, 17.7 ± 0.2°, 18.0 ± 0.2°;
or 7.1 ± 0.2°, 16.3 ± 0.2°, 13.4 ± 0.2°, 14.2 ± 0.2°, 17.4 ± 0.2°, 18.0 ± 0.2°;
or 7.1 ± 0.2°, 16.3 ± 0.2°, 13.4 ± 0.2°, 14.2 ± 0.2°, 17.7 ± 0.2°, 18.0 ± 0.2°;
or 7.1 ± 0.2°, 16.3 ± 0.2°, 14.2 ± 0.2°, 15.5 ± 0.2°, 17.7 ± 0.2°, 18.0 ± 0.2°;
or 7.1 ± 0.2°, 16.3 ± 0.2°, 13.4 ± 0.2°, 14.2 ± 0.2°, 15.5 ± 0.2°, 17.4 ± 0.2°, 18.0 ± 0.2°;
or 7.1 ± 0.2°, 16.3 ± 0.2°, 14.2 ± 0.2°, 15.5 ± 0.2°, 17.4 ± 0.2°, 17.7 ± 0.2°, 18.0 ± 0.2°;
or 7.1 ± 0.2°, 16.3 ± 0.2°, 13.4 ± 0.2°, 15.5 ± 0.2°, 17.4 ± 0.2°, 17.7 ± 0.2°, 18.0 ± 0.2°;
or 7.1 ± 0.2°, 16.3 ± 0.2°, 13.4 ± 0.2°, 14.2 ± 0.2°, 17.4 ± 0.2°, 17.7 ± 0.2°, 18.0 ± 0.2°;
or 7.1 ± 0.2°, 16.3 ± 0.2°, 13.4 ± 0.2°, 14.2 ± 0.2°, 15.5 ± 0.2°, 17.7 ± 0.2°, 18.0 ± 0.2°;
or 7.1 ± 0.2°, 13.4 ± 0.2°, 14.2 ± 0.2°, 15.5 ± 0.2°, 16.3 ± 0.2°, 17.4 ± 0.2°, 17.7 ± 0.2°, 18.0 ± 0.2°.

Further, in certain embodiments of the present invention, provided is a sulfate crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof optionally has a diffraction peak at one or more of 2θ of 19.8 ± 0.2°, 20.1 ± 0.2°, 21.0 ± 0.2°, 21.2 ± 0.2°, 22.6 ± 0.2°, 24.0 ± 0.2°, 24.8 ± 0.2°, 25.4 ± 0.2°, 26.1 ± 0.2°, 27.9 ± 0.2° or 28.6 ± 0.2°; preferably, the X-ray powder diffraction pattern thereof has at least a diffraction peak at any 2 to 4 of the above-mentioned 2θ, or at any 5 to 7 of the above-mentioned 2θ, and further preferably, at any 4 or 6 of the above-mentioned 2θ; furthermore, in certain embodiments of the present invention, provided is a sulfate crystal form of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

19.8 ± 0.2°, 20.1 ± 0.2°, 21.0 ± 0.2°, 21.2 ± 0.2°;

or 20.1 ± 0.2°, 21.0 ± 0.2°, 21.2 ± 0.2°, 22.6 ± 0.2°;

or 21.0 ± 0.2°, 21.2 ± 0.2°, 22.6 ± 0.2°, 24.0 ± 0.2°;

or 21.2 ± 0.2°, 22.6 ± 0.2°, 24.0 ± 0.2°, 24.8 ± 0.2°;

or 22.6 ± 0.2°, 24.0 ± 0.2°, 24.8 ± 0.2°, 25.4 ± 0.2°;

or 19.8 ± 0.2°, 20.1 ± 0.2°, 21.0 ± 0.2°, 21.2 ± 0.2°, 22.6 ± 0.2°, 24.0 ± 0.2°;

or 20.1 ± 0.2°, 21.0 ± 0.2°, 21.2 ± 0.2°, 22.6 ± 0.2°, 24.0 ± 0.2°, 24.8 ± 0.2°;

or 21.0 ± 0.2°, 21.2 ± 0.2°, 22.6 ± 0.2°, 24.0 ± 0.2°, 24.8 ± 0.2°, 25.4 ± 0.2°;

or 21.2 ± 0.2°, 22.6 ± 0.2°, 24.0 ± 0.2°, 24.8 ± 0.2°, 25.4 ± 0.2°, 26.1 ± 0.2°;

or 22.6 ± 0.2°, 24.0 ± 0.2°, 24.8 ± 0.2°, 25.4 ± 0.2°, 26.1 ± 0.2°, 27.9 ± 0.2°;

or 24.0 ± 0.2°, 24.8 ± 0.2°, 25.4 ± 0.2°, 26.1 ± 0.2°, 27.9 ± 0.2°, 28.6 ± 0.2°.

Further, in certain embodiments of the present invention, the X-ray powder diffraction pattern of the sulfate crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 7.1 ± 0.2°, 13.4 ± 0.2°, 14.2 ± 0.2°, 15.5 ± 0.2°, 16.3 ± 0.2°, 17.4 ± 0.2°, 17.7 ± 0.2°, 18.0 ± 0.2° and 19.8 ± 0.2°.

Further, in certain embodiments of the present invention, the X-ray powder diffraction pattern of the sulfate crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone further has a peak at diffraction angles (2θ) of 20.1 ± 0.2°, 21.0 ± 0.2°, 21.2 ± 0.2°, 22.6 ± 0.2°, 24.0 ± 0.2°, 24.8 ± 0.2°, 25.4 ± 0.2°, 26.1 ± 0.2°, 27.9 ± 0.2°, 28.6 ± 0.2° and 33.013 ± 0.2°.

Further, in certain embodiments of the present invention, provided is a sulfate crystal form of the (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein by using Cu-Ka radiation, the X-ray characteristic diffraction peaks as represented by 2θ and interplanar spacing d values are shown in Table 5:

**Table 5**

| Serial No. | XRPD data of sulfate | | | | | |
|---|---|---|---|---|---|---|
| | 2θ (± 0.2°) | d value | Peak height | Proportion (I%) | Area | Proportion (I%) |
| 1 | 7.1 | 12.4777 | 957 | 43.8 | 6134 | 31.3 |
| 2 | 8.9 | 9.9668 | 69 | 3.2 | 512 | 2.6 |
| 3 | 10.8 | 8.2 | 70 | 3.2 | 538 | 2.7 |
| 4 | 13.4 | 6.6 | 449 | 20.5 | 2645 | 13.5 |
| 5 | 13.8 | 6.4 | 117 | 5.4 | 950 | 4.9 |
| 6 | 14.2 | 6.2 | 375 | 17.2 | 2976 | 15.2 |
| 7 | 15.5 | 5.7155 | 286.0 | 13.1 | 2441 | 12.5 |
| 8 | 16.3 | 5.4383 | 1374.0 | 62.9 | 8084 | 41.3 |
| 9 | 17.4 | 5.0972 | 461 | 21.1 | 3308 | 16.9 |
| 10 | 17.7 | 4.9936 | 790 | 36.2 | 5883 | 30 |
| 11 | 18.0 | 4.9268 | 1893 | 86.6 | 12396 | 63.3 |
| 12 | 19.8 | 4.4903 | 668 | 30.6 | 4777 | 24.4 |
| 13 | 20.1 | 4.4238 | 513 | 23.5 | 3855 | 19.7 |
| 14 | 21.0 | 4.2328 | 346 | 15.8 | 2817 | 14.4 |
| 15 | 21.2 | 4.184 | 341 | 15.6 | 2507 | 12.8 |
| 16 | 22.6 | 3.9358 | 2185 | 100 | 19581 | 100 |
| 17 | 24.0 | 3.7006 | 841 | 38.5 | 6035 | 30.8 |
| 18 | 24.8 | 3.587 | 654 | 29.9 | 4823 | 24.6 |
| 19 | 25.4 | 3.5075 | 398 | 18.2 | 3203 | 16.4 |
| 20 | 26.1 | 3.4146 | 406 | 18.6 | 2815 | 14.4 |
| 21 | 27.9 | 3.1929 | 287 | 13.1 | 3483 | 17.8 |
| 22 | 28.6 | 3.1175 | 282 | 12.9 | 3019 | 15.4 |

In certain embodiments of the present invention, provided is a sulfate crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof is basically as shown in FIG. 5.

In certain embodiments of the present invention, provided is a hydrobromide crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has a diffraction peak at 2θ of 9.2 ± 0.2°, or has a diffraction peak at 2θ of 12.0 ± 0.2°, or has a diffraction peak at 2θ of 12.9 ± 0.2°, or has a diffraction peak at 2θ of 14.6 ± 0.2°, or has a diffraction peak at 2θ of 16.8 ± 0.2°, or has a diffraction peak at 2θ of 17.1 ± 0.2°, or has a diffraction peak at 2θ of 18.6 ± 0.2°, or has a diffraction peak at 2θ of 22.7 ± 0.2°, or has a diffraction peak at 2θ of 19.5 ± 0.2°, or has a diffraction peak at 2θ of 19.8 ± 0.2°, or has a diffraction peak at 2θ of 20.2 ± 0.2°, or has a diffraction peak at 2θ of 22.5 ± 0.2°, or has a diffraction peak at 2θ of 25.4 ± 0.2°, or has a diffraction peak at 2θ of 26.4 ± 0.2°, or has a diffraction peak at 2θ of 27.5 ± 0.2°, or has a diffraction peak at 2θ of 27.8 ± 0.2°, or has a diffraction peak at 2θ of 28.9 ± 0.2°, or has a diffraction peak at 2θ of 29.3 ± 0.2°, or has a diffraction peak at 2θ of 32.3 ± 0.2°; preferably, the X-ray powder diffraction pattern thereof optionally has a diffraction peak at 2, 4, 6, 8 or 10 of the above-mentioned 2θ.

In certain embodiments of the present invention, provided is a hydrobromide crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a diffraction peak at one or more of 2θ of 9.2 ± 0.2°, 12.0 ± 0.2°, or 12.9 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 14.6 ± 0.2°, 16.8 ± 0.2°, 17.1 ± 0.2°, 18.6 ± 0.2° or 22.7 ± 0.2°, preferably at 2, 3, 4 or 5 of the above-mentioned 2θ;

furthermore, in certain embodiments of the present invention, provided is a hydrobromide crystal form of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

9.2 ± 0.2°, 12.0 ± 0.2°, 14.6 ± 0.2°, 16.8 ± 0.2°;
or 9.2 ± 0.2°, 12.0 ± 0.2°, 16.8 ± 0.2°, 17.1 ± 0.2°;
or 9.2 ± 0.2°, 12.0 ± 0.2°, 17.1 ± 0.2°, 18.6 ± 0.2°;
or 9.2 ± 0.2°, 12.0 ± 0.2°, 18.6 ± 0.2°, 22.7 ± 0.2°;
or 9.2 ± 0.2°, 12.0 ± 0.2°, 14.6 ± 0.2°, 17.1 ± 0.2°;
or 9.2 ± 0.2°, 12.0 ± 0.2°, 14.6 ± 0.2°, 18.6 ± 0.2°;
or 9.2 ± 0.2°, 12.0 ± 0.2°, 14.6 ± 0.2°, 22.7 ± 0.2°;
or 9.2 ± 0.2°, 12.0 ± 0.2°, 16.8 ± 0.2°, 18.6 ± 0.2°;
or 9.2 ± 0.2°, 12.0 ± 0.2°, 16.8 ± 0.2°, 22.7 ± 0.2°;
or 9.2 ± 0.2°, 12.0 ± 0.2°, 17.1 ± 0.2°, 22.7 ± 0.2°;
or 12.0 ± 0.2°, 12.9 ± 0.2°, 14.6 ± 0.2°, 16.8 ± 0.2°;
or 12.0 ± 0.2°, 12.9 ± 0.2°, 16.8 ± 0.2°, 17.1 ± 0.2°;
or 12.0 ± 0.2°, 12.9 ± 0.2°, 17.1 ± 0.2°, 18.6 ± 0.2°;
or 12.0 ± 0.2°, 12.9 ± 0.2°, 18.6 ± 0.2°, 22.7 ± 0.2°;
or 12.0 ± 0.2°, 12.9 ± 0.2°, 14.6 ± 0.2°, 17.1 ± 0.2°;
or 12.0 ± 0.2°, 12.9 ± 0.2°, 14.6 ± 0.2°, 18.6 ± 0.2°;
or 12.0 ± 0.2°, 12.9 ± 0.2°, 14.6 ± 0.2°, 22.7 ± 0.2°;
or 12.0 ± 0.2°, 12.9 ± 0.2°, 16.8 ± 0.2°, 18.6 ± 0.2°;
or 12.0 ± 0.2°, 12.9 ± 0.2°, 16.8 ± 0.2°, 22.7 ± 0.2°;
or 12.0 ± 0.2°, 12.9 ± 0.2°, 17.1 ± 0.2°, 22.7 ± 0.2°;
or 9.2 ± 0.2°, 12.9 ± 0.2°, 14.6 ± 0.2°, 16.8 ± 0.2°;
or 9.2 ± 0.2°, 12.9 ± 0.2°, 16.8 ± 0.2°, 17.1 ± 0.2°;
or 9.2 ± 0.2°, 12.9 ± 0.2°, 17.1 ± 0.2°, 18.6 ± 0.2°;
or 9.2 ± 0.2°, 12.9 ± 0.2°, 18.6 ± 0.2°, 22.7 ± 0.2°;
or 9.2 ± 0.2°, 12.9 ± 0.2°, 14.6 ± 0.2°, 17.1 ± 0.2°;
or 9.2 ± 0.2°, 12.9 ± 0.2°, 14.6 ± 0.2°, 18.6 ± 0.2°;
or 9.2 ± 0.2°, 12.9 ± 0.2°, 14.6 ± 0.2°, 22.7 ± 0.2°;
or 9.2 ± 0.2°, 12.9 ± 0.2°, 16.8 ± 0.2°, 18.6 ± 0.2°;
or 9.2 ± 0.2°, 12.9 ± 0.2°, 16.8 ± 0.2°, 22.7 ± 0.2°;
or 9.2 ± 0.2°, 12.9 ± 0.2°, 17.1 ± 0.2°, 22.7 ± 0.2°;
or 9.2 ± 0.2°, 12.0 ± 0.2°, 14.6 ± 0.2°, 16.8 ± 0.2°, 17.1 ± 0.2°;
or 9.2 ± 0.2°, 12.0 ± 0.2°, 16.8 ± 0.2°, 17.1 ± 0.2°, 18.6 ± 0.2°;
or 9.2 ± 0.2°, 12.0 ± 0.2°, 17.1 ± 0.2°, 18.6 ± 0.2°, 22.7 ± 0.2°;
or 9.2 ± 0.2°, 12.0 ± 0.2°, 14.6 ± 0.2°, 17.1 ± 0.2°, 18.6 ± 0.2°;
or 9.2 ± 0.2°, 12.9 ± 0.2°, 14.6 ± 0.2°, 16.8 ± 0.2°, 17.1 ± 0.2°;
or 9.2 ± 0.2°, 12.9 ± 0.2°, 16.8 ± 0.2°, 17.1 ± 0.2°, 18.6 ± 0.2°;
or 9.2 ± 0.2°, 12.9 ± 0.2°, 17.1 ± 0.2°, 18.6 ± 0.2°, 22.7 ± 0.2°;
or 9.2 ± 0.2°, 12.9 ± 0.2°, 14.6 ± 0.2°, 17.1 ± 0.2°, 18.6 ± 0.2°;

or 12.0 ± 0.2°, 12.9 ± 0.2°, 14.6 ± 0.2°, 16.8 ± 0.2°, 17.1 ± 0.2°;
or 12.0 ± 0.2°, 12.9 ± 0.2°, 16.8 ± 0.2°, 17.1 ± 0.2°, 18.6 ± 0.2°;
or 12.0 ± 0.2°, 12.9 ± 0.2°, 17.1 ± 0.2°, 18.6 ± 0.2°, 22.7 ± 0.2°;
or 12.0 ± 0.2°, 12.9 ± 0.2°, 14.6 ± 0.2°, 17.1 ± 0.2°, 18.6 ± 0.2°;
or 9.2 ± 0.2°, 12.0 ± 0.2°, 12.9 ± 0.2°, 14.6 ± 0.2°, 16.8 ± 0.2°, 17.1 ± 0.2°;
or 9.2 ± 0.2°, 12.0 ± 0.2°, 12.9 ± 0.2°, 16.8 ± 0.2°, 17.1 ± 0.2°, 18.6 ± 0.2°;
or 9.2 ± 0.2°, 12.0 ± 0.2°, 12.9 ± 0.2°, 17.1 ± 0.2°, 18.6 ± 0.2°, 22.7 ± 0.2°;
or 9.2 ± 0.2°, 12.0 ± 0.2°, 12.9 ± 0.2°, 14.6 ± 0.2°, 17.1 ± 0.2°, 18.6 ± 0.2°;
or 9.2 ± 0.2°, 12.0 ± 0.2°, 12.9 ± 0.2°, 14.6 ± 0.2°, 16.8 ± 0.2°, 17.1 ± 0.2°, 18.6 ± 0.2°;
or 9.2 ± 0.2°, 12.0 ± 0.2°, 12.9 ± 0.2°, 16.8 ± 0.2°, 17.1 ± 0.2°, 18.6 ± 0.2°, 22.7 ± 0.2°;
or 9.2 ± 0.2°, 12.0 ± 0.2°, 12.9 ± 0.2°, 14.6 ± 0.2°, 17.1 ± 0.2°, 18.6 ± 0.2°, 22.7 ± 0.2°;
or 9.2 ± 0.2°, 12.0 ± 0.2°, 12.9 ± 0.2°, 14.6 ± 0.2°, 16.8 ± 0.2°, 18.6 ± 0.2°, 22.7 ± 0.2°;
or 9.2 ± 0.2°, 12.0 ± 0.2°, 12.9 ± 0.2°, 14.6 ± 0.2°, 16.8 ± 0.2°, 17.1 ± 0.2°, 22.7 ± 0.2°;
or 9.2 ± 0.2°, 12.0 ± 0.2°, 12.9 ± 0.2°, 14.6 ± 0.2°, 16.8 ± 0.2°, 17.1 ± 0.2°, 18.6 ± 0.2°, 22.7 ± 0.2°.

Further, in certain embodiments of the present invention, provided is a hydrobromide crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof optionally has a diffraction peak at one or more of 2θ of 19.5 ± 0.2°, 19.8 ± 0.2°, 20.2 ± 0.2°, 22.5 ± 0.2°, 25.4 ± 0.2°, 26.4 ± 0.2°, 27.5 ± 0.2°, 27.8 ± 0.2°, 28.9 ± 0.2°, 29.3 ± 0.2° or 32.3 ± 0.2°; preferably, the X-ray powder diffraction pattern thereof has at least a diffraction peak at any 2 to 4 of the above-mentioned 2θ, or at any 5 to 7 of the above-mentioned 2θ, and further preferably, at any 4 or 6 of the above-mentioned 2θ;
furthermore, in certain embodiments of the present invention, provided is a hydrobromide crystal form of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof has at least a peak at following diffraction angles (2θ):

19.5 ± 0.2°, 19.8 ± 0.2°, 20.2 ± 0.2°, 22.5 ± 0.2°;

or 19.8 ± 0.2°, 20.2 ± 0.2°, 22.5 ± 0.2°, 25.4 ± 0.2°;

or 20.2 ± 0.2°, 22.5 ± 0.2°, 25.4 ± 0.2°, 26.4 ± 0.2°;

or 22.5 ± 0.2°, 25.4 ± 0.2°, 26.4 ± 0.2°, 27.5 ± 0.2°;

or 25.4 ± 0.2°, 26.4 ± 0.2°, 27.5 ± 0.2°, 27.8 ± 0.2°;

or 26.4 ± 0.2°, 27.5 ± 0.2°, 27.8 ± 0.2°, 28.9 ± 0.2°;

or 27.5 ± 0.2°, 27.8 ± 0.2°, 28.9 ± 0.2°, 29.3 ± 0.2°;

or 27.8 ± 0.2°, 28.9 ± 0.2°, 29.3 ± 0.2°, 32.3 ± 0.2°;

or 19.5 ± 0.2°, 19.8 ± 0.2°, 20.2 ± 0.2°, 22.5 ± 0.2°, 25.4 ± 0.2°, 26.4 ± 0.2°;

or 19.8 ± 0.2°, 20.2 ± 0.2°, 22.5 ± 0.2°, 25.4 ± 0.2°, 26.4 ± 0.2°, 27.5 ± 0.2°;

or 20.2 ± 0.2°, 22.5 ± 0.2°, 25.4 ± 0.2°, 26.4 ± 0.2°, 27.5 ± 0.2°, 27.8 ± 0.2°;

or 22.5 ± 0.2°, 25.4 ± 0.2°, 26.4 ± 0.2°, 27.5 ± 0.2°, 27.8 ± 0.2°, 28.9 ± 0.2°;

or 25.4 ± 0.2°, 26.4 ± 0.2°, 27.5 ± 0.2°, 27.8 ± 0.2°, 28.9 ± 0.2°, 29.3 ± 0.2°;

or 26.4 ± 0.2°, 27.5 ± 0.2°, 27.8 ± 0.2°, 28.9 ± 0.2°, 29.3 ± 0.2°, 32.3 ± 0.2°.

Further, in certain embodiments of the present invention, the X-ray powder diffraction pattern of the hydrobromide crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 9.2 ± 0.2°, 12.0 ± 0.2°, 12.9 ± 0.2°, 14.6 ± 0.2°, 16.8 ± 0.2°, 17.1 ± 0.2°, 18.6 ± 0.2°, 22.7 ± 0.2° and 26.4 ± 0.2°.

Further, in certain embodiments of the present invention, the X-ray powder diffraction pattern of the hydrobromide crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone has a peak at diffraction angles (2θ) of 26.4 ± 0.2°, 27.5 ± 0.2°, 27.8 ± 0.2°, 28.9 ± 0.2°, 29.3 ± 0.2°, 32.3 ± 0.2° and 32.589 ± 0.2°.

Further, in certain embodiments of the present invention, provided is a hydrobromide crystal form of the (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein by using Cu-Ka radiation, the X-ray characteristic diffraction peaks as represented by 2θ and interplanar spacing d values are shown in Table 6:

**Table 6**

| Serial No. | XRPD data of hydrobromide | | | | | |
|---|---|---|---|---|---|---|
| | 2θ (± 0.2°) | d value | Peak height | Proportion (I%) | Area | Proportion (I%) |
| 1 | 9.2 | 9.6114 | 930 | 85.9 | 5901 | 48.1 |
| 2 | 10.4 | 8.5180 | 144 | 13.3 | 707 | 5.8 |
| 3 | 12.0 | 7.3506 | 477 | 44 | 3647 | 29.7 |
| 4 | 12.9 | 6.8352 | 509 | 47 | 2999 | 24.4 |
| 5 | 14.6 | 6.0520 | 354 | 32.7 | 2065 | 16.8 |
| 6 | 16.8 | 5.2822 | 804 | 74.2 | 4893 | 39.9 |
| 7 | 17.1 | 5.1949 | 777 | 71.7 | 4948 | 40.3 |
| 8 | 18.6 | 4.7778 | 1083 | 100 | 6649 | 54.2 |
| 9 | 19.5 | 4.5609 | 285 | 26.3 | 1584 | 12.9 |
| 10 | 19.8 | 4.4763 | 532 | 49.1 | 3445 | 28.1 |
| 11 | 20.2 | 4.4001 | 577 | 53.3 | 2440 | 19.9 |
| 12 | 22.5 | 3.9506 | 573 | 52.9 | 6497 | 52.9 |
| 13 | 22.7 | 3.9120 | 923 | 85.2 | 12274 | 100 |
| 14 | 24.2 | 3.6757 | 195 | 18 | 1518 | 12.4 |
| 15 | 25.4 | 3.5051 | 614 | 56.7 | 3446 | 28.1 |
| 16 | 26.4 | 3.3761 | 543 | 50.1 | 2999 | 24.4 |
| 17 | 27.5 | 3.2388 | 588 | 54.3 | 3902 | 31.8 |
| 18 | 27.8 | 3.2064 | 366 | 33.8 | 5464 | 44.5 |
| 19 | 28.9 | 3.0854 | 311 | 28.7 | 3420 | 27.9 |
| 20 | 29.3 | 3.0433 | 147 | 13.6 | 3154 | 25.7 |
| 21 | 30.8 | 2.9038 | 256 | 23.6 | 1898 | 15.5 |
| 22 | 31.9 | 2.8001 | 930 | 85.9 | 5901 | 48.1 |
| 23 | 32.3 | 2.7658 | 117 | 10.8 | 570 | 4.6 |

Further, in certain embodiments of the present invention, provided is a hydrobromide crystal form of the compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, wherein the X-ray powder diffraction pattern thereof is basically as shown in FIG. 6.

As those skilled in the art will understand, measurements of the peak position (2θ) will vary due to differences in XRPD instruments, sometimes by as much as 0.2°. Therefore, for such variation or shifts, the term "basically the same" can also be used to express the variability in the position and intensity of X-ray diffraction peaks. In addition, those skilled in the art can also understand that factors such as the XRPD sample preparation method, XRPD instrument, sample crystallinity, sample dosage, and preferred crystal orientation will also lead to changes in the relative peak intensity in the XRPD diffraction pattern of the sample.

Compared with amorphous substances, the crystalline form obtained by the present invention is more stable both chemically and physically, which is beneficial to drug production, transportation and storage; and also more suitable for

drug development needs. It is also more advantageous for drug purification, decolorization, filtration and other process operations. Therefore, the crystal form of the compound of the present invention has significant improvement significance and meets the requirements of clinical development.

In another aspect of the present invention, provided is a method for preparing the crystal form as described above, comprising the steps of:

a) weighing an appropriate amount of the compound, dissolving same with an organic solvent to clarification,

b) stirring, filtering and drying same to obtain the target product;

wherein the organic solvent is selected from methanol, ethanol, n-propanol, isopropanol, n-butanol, acetonitrile, acetone, methyl ethyl ketone, tetrahydrofuran, dioxane, N,N-dimethylformamide, dimethyl sulfoxide, ethyl acetate, isopropyl acetate, dichloromethane, trichloroethane, carbon tetrachloride, methyl tert-butyl ether, isopropyl ether, benzene, toluene, xylene or a combination thereof.

In another aspect of the present invention, provided is a method for preparing the acidic salt or crystal form thereof as described above, comprising the steps of:

a) weighing an appropriate amount of the free base crystal form of the compound and adding same to an organic solvent;

b) weighing an appropriate amount of the acid, dissolving same with the organic solvent, stirring and then filtering same or dissolving and evaporating same to obtain a gel, to which an antisolvent is added, stirring and filtering same, and drying same in vacuum and separating same to obtain the target product;

wherein:
the organic solvent is selected from methanol, ethanol, n-propanol, isopropanol, n-butanol, acetonitrile, acetone, methyl ethyl ketone, tetrahydrofuran, dioxane, N,N-dimethylformamide, dimethyl sulfoxide, ethyl acetate, isopropyl acetate, dichloromethane, trichloroethane, carbon tetrachloride, methyl tert-butyl ether, isopropyl ether, benzene, toluene, xylene or a combination thereof; the antisolvent is selected from ethyl acetate, n-heptane, n-hexane, isooctane, pentane, cyclohexane, cyclopentane, diethyl ether or a combination thereof; the acid in the acid solution is selected from an inorganic acid or an organic acid, wherein the inorganic acid is selected from hydrochloric acid, sulfuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexane aminosulfonic acid, camphor sulfonic acid, aspartic acid, camphor acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, ethane-1,2-disulfonic acid, ethanesulfonic acid, formic acid, fumaric acid, galactonic acid, gentisuric acid, glutaric acid, 2-ketoglutaric acid, glycolic acid, benzoylglycine, hydroxyethyl sulfonic acid, lactobionic acid, ascorbic acid, aspartic acid, lauric acid, camphor acid, maleic acid, malonic acid, methanesulfonic acid, 1,5-naphthalene disulfonic acid, naphthalene-2-sulfonic acid, nicotinic acid, oleic acid, whey acid, oxalic acid, palmitic acid, pamoic acid, propanoic acid, salicylic acid, 4-aminosalicylic acid, sebacic acid, stearic acid, succinic acid, thiocyanic acid, pamoic acid, formic acid, undecylenoic acid, trifluoroacetic acid, benzenesulfonic acid, p-toluenesulfonic acid or L-malic acid.

In another aspect of the present invention, provided is a method for preparing the acidic salt or crystal form thereof of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone as described above, comprising the following steps:
adding the free base crystal form A of compound-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone into an organic solvent, into which an acid solution is added, stirring and then filtering same or dissolving and evaporating same to obtain a gel, to which an antisolvent is added, stirring and filtering same, and drying same in vacuum and separating same to obtain the crystal form of the salt.

The dissolution refers to the general operation by those of ordinary skill in the art. Usually, the raw materials can be dissolved or dissolved to clarification by heating appropriately, or the amount of solvents can be increased to dissolve or dissolve the raw materials to clarification, or the technical solutions can be modified or equivalently replaced, all of which should be included within the scope of the present invention.

Further, in certain embodiments of the present invention, in the preparation method, the organic solvent is selected from methanol, ethanol, n-propanol, isopropanol, n-butanol, acetonitrile, acetone, methyl ethyl ketone, tetrahydrofuran, dioxane, N,N-dimethylformamide, dimethyl sulfoxide, ethyl acetate, isopropyl acetate, dichloromethane, trichloroethane, carbon tetrachloride, methyl tert-butyl ether, isopropyl ether, benzene, toluene, xylene or a combination

thereof; the antisolvent is selected from ethyl acetate, n-heptane, n-hexane, isooctane, pentane, cyclohexane, cyclopentane, diethyl ether or a combination thereof; the acid solution is selected from hydrogen chloride in methanol, methanesulfonic acid in methanol, nitric acid in methanol, sulfuric acid in methanol or hydrobromic acid in methanol.

Further, in certain embodiments of the present invention, in the preparation method, the organic solvent is selected from tetrahydrofuran or ethyl acetate; the antisolvent is selected from ethyl acetate; the acid solution is selected from hydrogen chloride in methanol, methanesulfonic acid in methanol, nitric acid in methanol, sulfuric acid in methanol or hydrobromic acid in methanol.

Further, in certain embodiments of the present invention, provided is an acidic salt crystal form of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone obtained by separation, wherein the X-ray powder diffraction peak or pattern thereof is as described in any one of the previous embodiments.

Further, in certain embodiments of the present invention, provided is a crystal form of the substantially pure (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone obtained by separation in step 3) of the preparation method, wherein the substantially pure crystal form refers to a crystal form with a crystal purity greater than 90%.

Preferably, the crystal purity of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone obtained by separation in step 3) of the preparation method is greater than 95%.

Most preferably, the crystal purity of (R)-(3-(3-chloro-1,2,4-thiadiazol-5-yl)-8-methyl-5,6-dihydro-[1,2,4]triazolo [4,3-a]pyrazin-7(8H)-yl)(4-fluorophenyl-3-d)methanone obtained by separation in step 3) of the preparation method is greater than 98%.

In yet another aspect of the present invention, provided is a pharmaceutical composition comprising a therapeutically effective amount of the foregoing crystal form or acidic salt of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, as well as a pharmaceutically acceptable carrier.

In the embodiments provided by the present invention, the pharmaceutical composition comprises a therapeutically effective amount of the crystal form, or the acidic salt or crystal form thereof, wherein the therapeutically effective amount is selected from 0.0001-99%, 0.0001-95%, 0.0001-90%, 0.0001-85%, 0.0001-80%, 0.0001-75%, 0.0001-70%, 0.001-60%, 0.001-55%, 0.01-50%, 0.01-40%, 0.01-30%, 0.01-20%, 0.01-10% or 0.01-5%.

In yet another aspect of the present invention, provided is use of the foregoing crystal form or acidic salt of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone, or the foregoing pharmaceutical composition in the preparation of NK3 inhibitor-related drugs, preferably in the preparation of drugs for the treatment and/or prevention of related diseases such as psychiatric disorders, cognitive impairment, Parkinson's disease, Alzheimer's disease, attention deficit hyperactivity disorder, pain, convulsions, obesity, inflammatory diseases, vomiting, preeclampsia, airway related diseases, reproductive disorders, hormone dependent diseases, or gynecological disease; further preferably, in the preparation of drugs for the treatment and/or prevention of diseases related to menopausal syndrome, which includes hot flashes, sweating, palpitations, dizziness and obesity.

Brief Description of the Drawings

FIG. 1 is the X-ray powder diffraction pattern of (R)-(3-(3-chloro-1,2,4-thiadiazol-5-yl)-8-methyl-5,6-dihydro-[1,2,4] triazolo[4,3-a]pyrazin-7(8H)-yl)(4-fluorophenyl-3-d) free base crystal form; the X-axis is the diffraction peak angle 2θ (º), and the Y-axis is the intensity of the peak.

FIG. 2 is the X-ray powder diffraction pattern of (R)-(3-(3-chloro-1,2,4-thiadiazol-5-yl)-8-methyl-5,6-dihydro-[1,2,4] triazolo[4,3-a]pyrazin-7(8H)-yl)(4-fluorophenyl-3-d)hydrochloride; the X-axis is the diffraction peak angle 2θ (º), and the Y-axis is the intensity of the peak.

FIG. 3 is the X-ray powder diffraction pattern of (R)-(3-(3-chloro-1,2,4-thiadiazol-5-yl)-8-methyl-5,6-dihydro-[1,2,4] triazolo[4,3-a]pyrazin-7(8H)-yl)(4-fluorophenyl-3-d)methanesulfonate; the X-axis is the diffraction peak angle 2θ (º), and the Y-axis is the intensity of the peak.

FIG. 4 is the X-ray powder diffraction pattern of (R)-(3-(3-chloro-1,2,4-thiadiazol-5-yl)-8-methyl-5,6-dihydro-[1,2,4] triazolo[4,3-a]pyrazin-7(8H)-yl)(4-fluorophenyl-3-d)nitrate; the X-axis is the diffraction peak angle 2θ (º), and the Y-axis is the intensity of the peak.

FIG. 5 is the X-ray powder diffraction pattern of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)sulfate; the X-axis is the diffraction peak angle 2θ (º), and the Y-axis is the intensity of the peak.

FIG. 6 is the X-ray powder diffraction pattern of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)hydrobromide; the X-axis is the diffraction peak angle 2θ (º), and the Y-axis is the intensity of the peak.

FIG. 7 is the DSC pattern of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) free base; the X-axis is temperature (°C), and the Y-axis is heat flow (W/G).

Detailed Description of Embodiments

The present invention will be further described below with reference to examples, but these examples do not limit the scope of the present invention.

I. Preparation of compounds

Compound A

(R)-(3-(benzo[d]thiazol-2-yl)-8-methyl-5,6-dihydro-[1,2,4]triazolo[4,3-a]pyrazin-7(8H)-yl)(4-fluorophenyl)methanone

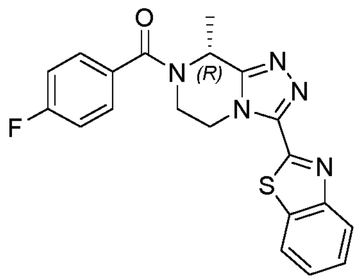

Step 1: Preparation of (R)-1-(2,4-dimethoxybenzyl)-5-ethoxy-6-methyl-1,2,3,6-tetrahydropyrazine

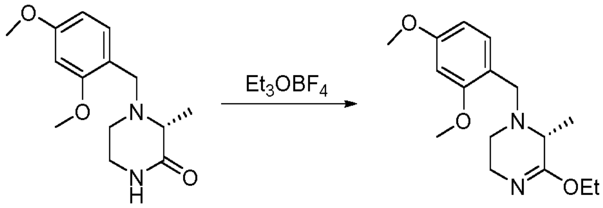


(R)-4-(2,4-dimethoxybenzyl)-3-methylpiperazin-2-one (0.20 g, 0.38 mmol) was dissolved in dichloroethane (1 mL) and cooled to 0°C. Triethyloxonium tetrafluoroborate (4.5 mL, 4.5 mmol) was added, and the reaction was stirred at room temperature for 3 hours. Water (10 mL) was added, and same was stirred for half an hour. A solid was precipitated, and same was filtered. The aqueous phase was extracted with dichloromethane (20 mL × 2), and the organic phases were combined, dried over anhydrous sodium sulfate, filtered, and concentrated to obtain (R)-1-(2,4-dimethoxybenzyl)-5-ethoxy-6-methyl-1,2,3,6-tetrahydropyrazine (0.2 g), which was used directly in the next step.
MS m/z (ESI): 293.2 $[M+H]^+$.

**Step 2: Preparation of benzo[d]thiazole-2-formylhydrazine**

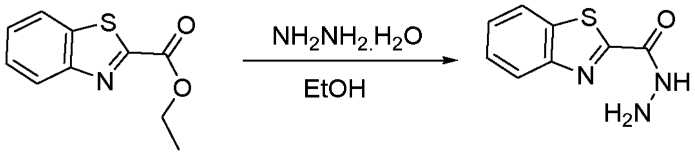


Ethyl benzo[d]thiazole-2-carboxylate (300 mg, 1.45 mmol) was dissolved in absolute ethanol (10 mL), 85% hydrazine hydrate (102 mg, 1.74 mmol) was added, and the mixture was stirred at room temperature for 4 hours. The mixture was filtered and dried to obtain benzo[d]thiazole-2-formylhydrazine (270 mg, yield: 96%).
MS m/z (ESI): 194.2 [M+H]+.

**Step 3: Preparation of (R)-2-(7-(2,4-dimethoxybenzyl)-8-methyl-5,6,7,8-tetrahydro-[1,2,4]triazolo[4,3-a]pyrazin-3-yl)benzo[d]thiazole**

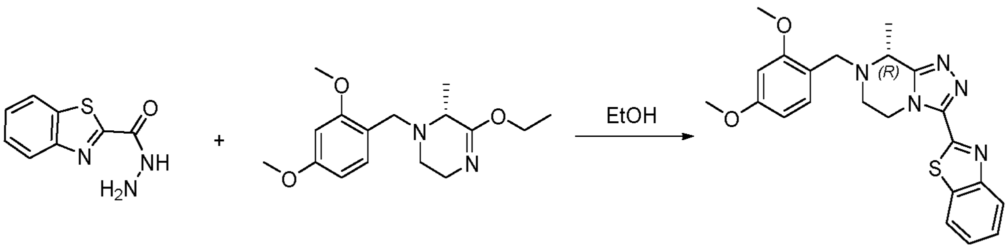


(R)-1-(2,4-dimethoxybenzyl)-5-ethoxy-6-methyl-1,2,3,6-tetrahydropyrazine (200 mg, 0.68 mmol) was dissolved in ethanol (10 mL), benzo[d]thiazole-2-formylhydrazine (120 mg, 0.62 mmol) was added, and the reaction was stirred at 80°C overnight. The reaction solution was spun to dryness, and the crude product was separated by column chromatography (petroleum ether/ethyl acetate: 5/1 - 1/2) to obtain (R)-2-(7-(2,4-dimethoxybenzyl)-8-methyl-5,6,7,8-tetrahydro-[1,2,4]triazolo[4,3-a]pyrazin-3-yl)benzo[d]thiazole (100 mg, yield: 38%).
MS m/z (ESI): 422.2 [M+H]+.

**Step 4: Preparation of (R)-2-(8-methyl-5,6,7,8-tetrahydro-[1,2,4]triazolo[4,3-a]pyrazin-3-yl)benzo[d]thiazole**

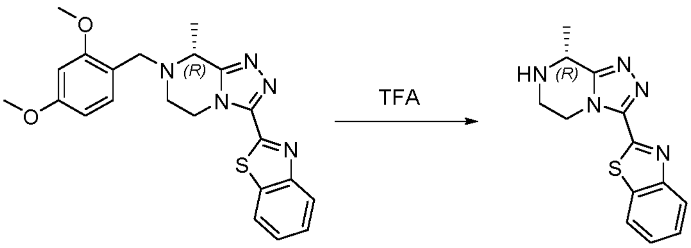


(R)-2-(7-(2,4-dimethoxybenzyl)-8-methyl-5,6,7,8-tetrahydro-[1,2,4]triazolo[4,3-a]pyrazin-3-yl)benzo[d]thiazole (100 mg, 0.24 mmol) was dissolved in dichloromethane (2 mL), trifluoroacetic acid (1 mL) was added, and the reaction was stirred at room temperature for 1 hour. TLC showed that the reaction was complete. Water (10 mL) was added and same was stirred for 5 minutes. After filtering, 3M sodium hydroxide solution was added to the filtrate until the pH of the aqueous phase was > 14. The mixture was extracted with dichloromethane (10 mL × 3), and the organic phases were combined, washed with saturated brine (10 mL), dried over anhydrous sodium sulfate, and concentrated under reduced pressure to obtain (R)-2-(8-methyl-5,6,7,8-tetrahydro-[1,2,4]triazolo[4,3-a]pyrazin-3-yl)benzo[d]thiazole (40 mg, yield: 62%).
MS m/z (ESI): 272.2 [M+H]+.

**Step 5: Preparation of (R)-(3-(benzo[d]thiazol-2-yl)-8-methyl-5,6-dihydro-[1,2,4]triazolo[4,3-a]pyrazin-7(8H)-yl)(4-fluorophenyl)methanone**

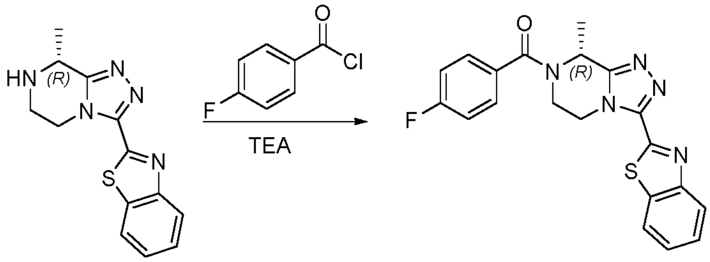


(R)-2-(8-methyl-5,6,7,8-tetrahydro-[1,2,4]triazolo[4,3-a]pyrazin-3-yl)benzo[d]thiazole (40 mg, 0.15 mmol) was dissolved in dichloromethane (2 mL), triethylamine (23 mg, 0.23 mmol) was added, and then 4-fluorobenzoyl chloride (28 mg, 0.18 mmol) was added, and the reaction was stirred at room temperature for 1 hour. Water (10 mL) was added for washing, and the mixture was extracted with dichloromethane (10 mL × 2). The organic phases were combined, washed with saturated brine (10 mL), dried over anhydrous sodium sulfate, and concentrated under reduced pressure to obtain a crude product, which was purified by preparative high-performance liquid chromatography to obtain (R)-(3-(benzo[d]thiazol-2-yl)-8-methyl-5,6-dihydro-[1,2,4]triazolo[4,3-a]pyrazin-7(8H)-yl)(4-fluorophenyl)methanone (25 mg, yield: 42%).

MS m/z (ESI): 394.2 $[M+H]^+$.

$^1H$ NMR (400 MHz, Chloroform-d) δ 8.06 (d, J = 8.1 Hz, 1H), 7.98 (d, J = 7.9 Hz, 1H), 7.57 - 7.45 (m, 4H), 7.27 - 7.16 (m, 2H), 5.78 (br s, 1H), 5.12 (d, J = 14.6 Hz, 1H), 4.84 - 4.55 (m, 1H), 4.42 - 4.35 (m, 1H), 3.60 - 3.54 (m, 1H), 1.77 (d, J = 6.9 Hz, 3H).

**Compound 1**

**(R)-(4-fluorophenyl-2,3,5,6-d4)(8-methyl-3-(3-(methyl-d3)-1,2,4-thiadiazol-5-yl)-5,6-dihydro-[1,2,4]triazolo[4,3-a]pyrazin-7(8H)-yl)methanone**

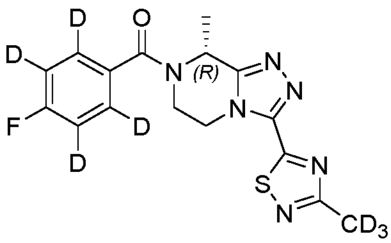

Referring to compound A step 5 for the preparation of compound 1.

MS m/z (ESI): 366.1$[M+H]^+$.

$^1H$ NMR (400 MHz, Chloroform-d) δ 5.97 - 5.56 (m, 1H), 5.11-4.79 (m, 1H), 4.73-4.147(m, 1H), 4.41-4.12 (m, 1H), 3.73 - 3.37 (m, 1H), 1.90 - 1.69 (m, 3H).

**Compound 2**

**(R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-2,3,5,6-d4)methanone**

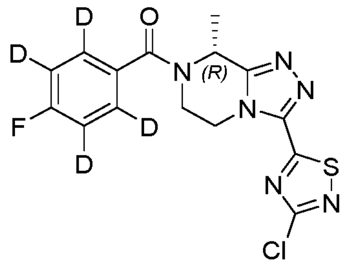

**Step 1: 3-chloro-5-(1-ethoxyvinyl)-1,2,4-thiadiazole**

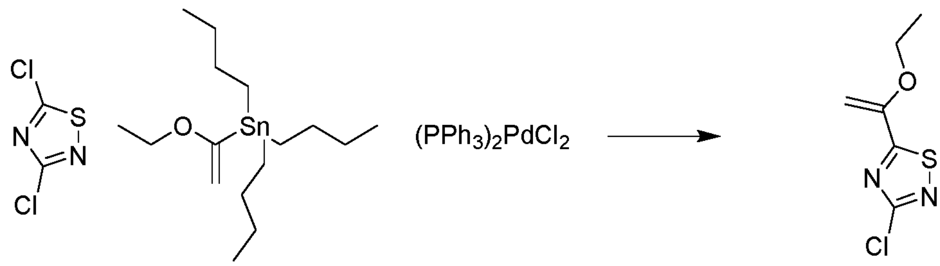


Bistriphenylphosphine palladium dichloride (1.13 g, 1.61 mmol) was added to 3,5-dichloro-1,2,4-thiadiazole (5 g, 32.26 mmol) and tributyl(1-ethoxyethylene)tin (10.48 g, 29.03 mmol) in anhydrous DMF (20 ml), and the reaction solution was heated to 75°C under nitrogen protection for 14 hours. The reaction solution was cooled, quenched with aqueous potassium fluoride solution (50 ml), and filtered to remove the solid, the liquid phase was extracted with ethyl acetate (75ml*2), and the organic phase was washed with saturated brine (20ml*6), dried and evaporated to obtain the crude product. The crude product was subjected to column separation (petroleum ether: ethyl acetate = 99: 1) to obtain the product 3-chloro-5-(1-ethoxyvinyl)-1,2,4-thiadiazole (4.3 g, yield: 70%).

$^1$H NMR (400 MHz, Chloroform-d) δ 5.52 (d, J = 3.1 Hz, 1H), 4.58 (d, J = 3.1 Hz, 1H), 4.02 (q, J = 7.0 Hz, 2H), 1.43 (t, J = 7.0 Hz, 3H).

**Step 2: Ethyl 3-chloro-1,2,4-thiadiazole-5-carboxylate**

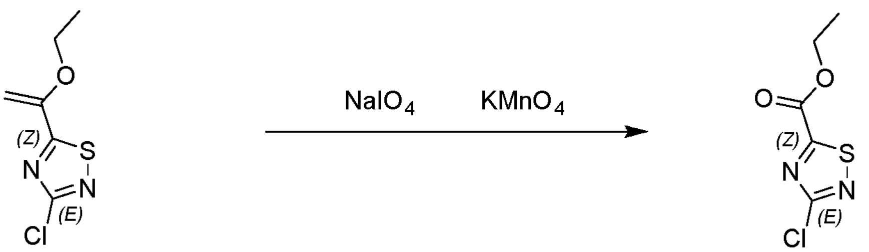


A solution of sodium periodate (8.30 g, 38.81 mmol) in water (35 ml) was added to a solution of 3-chloro-5-(1-ethoxyvinyl)-1,2,4-thiadiazole (3.7 g, 19.41 mmol) in dioxane (70 ml), the reaction solution was stirred for 2 minutes, then potassium permanganate (460.05 mg, 2.91 mmol) was added to the reaction solution, and the reaction solution was stirred at room temperature overnight. The reaction solution was extracted with ethyl acetate (100 mL × 3), and the organic phases were combined, washed with saturated brine (50 mL × 2), dried over anhydrous sodium sulfate, and concentrated under reduced pressure. The residue was purified by flash silica gel chromatography (petroleum ether: ethyl acetate = 100 : 0 to 95 : 5 elution) to obtain the target product ethyl 3-chloro-1,2,4-thiadiazole-5-carboxylate (1 g, yield: 27.03%).

$^1$H NMR (400 MHz, Chloroform-d) δ 4.54 (q, J = 7.1 Hz, 2H), 1.47 (t, J = 7.2 Hz, 3H).

**Step 3: 3-chloro-1,2,4-thiadiazole-5-formylhydrazine**

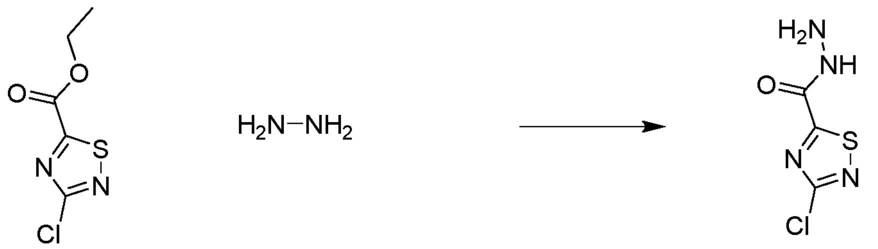

Hydrazine hydrate (229 mg, 3.89 mmol, 85% aqueous) was added to a solution of ethyl 3-chloro-1,2,4-thiadiazole-5-carboxylate (0.5 g, 2.6 mmol) in ethanol (10 ml). The reaction solution was stirred at room temperature for 1 hour. The solid product 3-chloro-1,2,4-thiadiazole-5-formylhydrazine (440 mg, yield: 95%) was then obtained by filtration.

MS m/z (ESI):179.0 $[M+H]^+$.

**Step 4: (5R)-4-[(2,4-dimethoxyphenyl)methyl]-6-ethoxy-5-methyl-3,5-dihydro-2H-pyrazine**

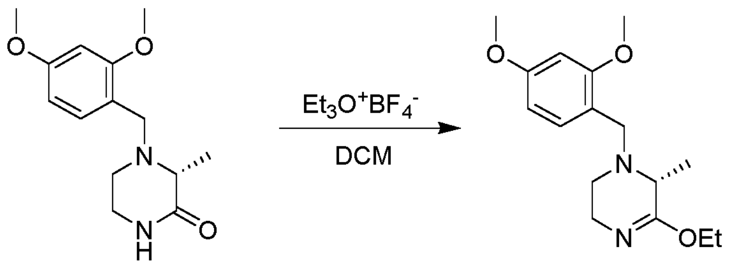


(3R)-4-[(2,4-dimethoxyphenyl)methyl]-3-methyl-piperazin-2-one (0.6 g, 2.27 mmol) was dissolved in dichloromethane (7 mL), and at 0°C, triethyloxonium tetrafluoroborate (1.08 g, 5.67 mmol) was added in three batches, with an interval of 10 minutes between each batch. The mixture was reacted with stirring for 3 hours at 25°C. The reaction solution was added to cold aqueous sodium hydroxide solution (2 M, 10 ml), and the mixture was subjected to liquid separation. The aqueous phase was extracted with dichloromethane (10mL × 2), and the organic phases were combined, dried over anhydrous sodium sulfate, and concentrated under reduced pressure to obtain the target product (5R)-4-[(2,4-dimethoxyphenyl)methyl]-6-ethoxy-5-methyl-3,5-dihydro-2H-pyrazine (0.65g) as a colorless oil. The crude product was directly used for the next step.

MS m/z (ESI): 293.2$[M+H]^+$.

**Step 5: 3-chloro-5-[(8R)-7-[(2,4-dimethoxyphenyl)methyl]-8-methyl-6,8-dihydro-5H-[1,2,4]triazolo[4,3-a]pyrazin-3-yl]-1,2,4-thiadiazole**

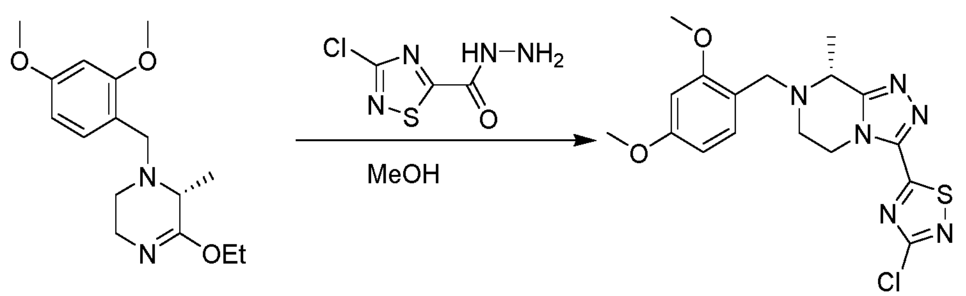


(5R)-4-[(2,4-dimethoxyphenyl)methyl]-6-ethoxy-5-methyl-3,5-dihydro-2H-pyrazine (0.66 g, 2.26 mmol) and 3-chloro-1,2,4-thiadiazole-5-formylhydrazine (322.54 mg, 1.81 mmol) were dissolved in MeOH (10 mL), and the mixture was reacted with stirring at 80°C under nitrogen protection for 14 hours. The reaction solution was concentrated under reduced pressure, and the residue was purified by flash silica gel chromatography (dichloromethane : methanol = 100 : 0 to

95 : 5 elution) to obtain the target product 3-chloro-5-[(8R)-7-[(2,4-dimethoxyphenyl)methyl]-8-methyl-6,8-dihydro-5H-[1,2,4]triazolo[4,3-a]pyrazin-3-yl]-1,2,4-thiadiazole (0.22 g, yield: 23.95%) as a light yellow solid.

MS m/z (ESI): 406.8[M+H]+.

**Step 6: 3-chloro-5-[(8R)-8-methyl -5,6,7,8-tetrahydro-[1,2,4]triazolo[4,3-a]pyrazin-3-yl]-1,2,4-thiadiazole**

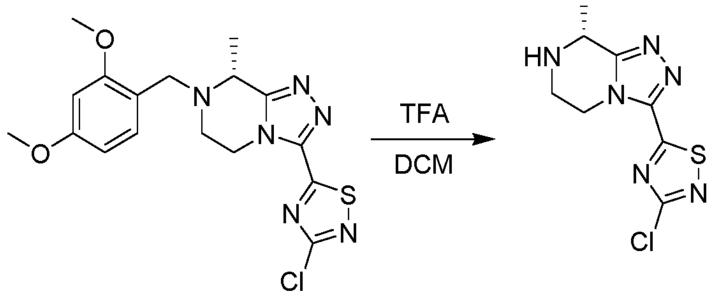


3-Chloro-5-[(8R)-7-[(2,4-dimethoxyphenyl)methyl]-8-methyl-6,8-dihydro-5H-[1,2,4]triazolo[4,3-a]pyrazin-3-yl]-1,2,4-thiadiazole (0.22 g, 540.69 umol) was dissolved in dichloromethane (1.5 mL), and trifluoroacetic acid (3 mL) was added. The mixture was reacted with stirring for 1 hour at 25°C. The reaction solution was evaporated to dryness under reduced pressure, then water (7 ml) was added, and the mixture was stirred at room temperature for 10 minutes, and then filtered to remove the solid. The liquid phase was lyophilized to obtain the product, a trifluoroacetate salt of 3-chloro-5-[(8R)-8-methyl-5,6,7,8-tetrahydro-[1,2,4]triazolo[4,3-a]pyrazin-3-yl]-1,2,4-thiadiazol (0.2 g, yield: 99.77%).

MS m/z (ESI): 257.0[M+H]+.

**Step 7: 4-fluoro-2,3,5,6- d4 benzoic acid**

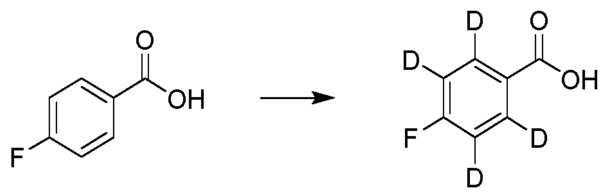

Under nitrogen protection, 10% Pd/C (0.73 g, containing 50% w/w water) was added to a solution of p-fluorobenzoic acid (1.8 g, 12.9 mmol) in isopropanol (50 mL) and deuterated water (100 mL), and the reaction solution was reacted with stirring at 100°C for 3 days. The reaction solution was cooled, and extracted with ethyl acetate (30mL*3), and the organic phases were dried over anhydrous sodium sulfate, and concentrated under reduced pressure to obtain the target product 4-fluoro-2,3,5,6- d4 benzoic acid (1.5g, yield: 81%).

MS m/z (ESI): 143.0[M-H]-

**Step 8: (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-2,3,5,6-d4)methanone**

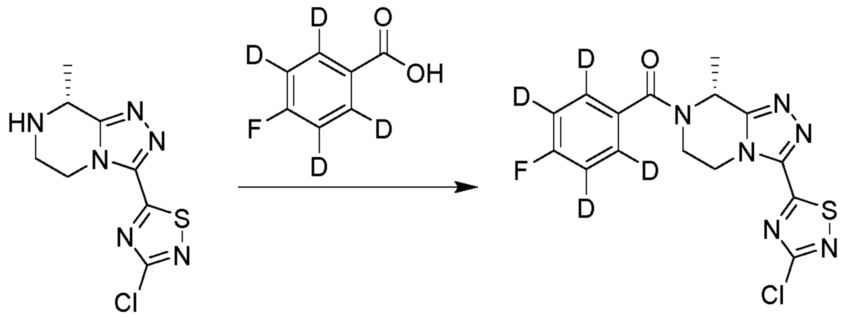

2-(7-Oxybenzotriazole)-N,N,N',N'-tetramethylurea hexafluorophosphate (32 mg, 84 umol) was added to a solution of a trifluoroacetate salt of 3-chloro-5-[(8R)-8-methyl-5,6,7,8-tetrahydro-[1,2,4]triazolo[4,3-a]pyrazin-3-yl]-1,2,4-thiadiazole of trifluoroacetate (20 mg, 56 umol), 4-fluoro-2,3,5,6-d4 benzoic acid (10 mg, 68 umol) and N,N-diisopropylethylamine (22 mg, 169 umol) in N,N-dimethylformamide (1.5 ml), and the reaction solution was stirred at room temperature for 16 hours. The crude product was directly separated by Prep-HPLC to obtain the product (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-2,3,5,6-d4) methanone (10 mg, yield: 46%).

MS m/z (ESI): 383.1[M+H]$^+$.

$^1$H NMR (400 MHz, Chloroform-d) δ 5.97 - 5.68 (m, 1H), 4.94-4.81 (m, 1H), 4.74-4.39 (m, 1H), 4.37-4.20 (m, 1H), 3.64 - 3.46 (m, 1H), 1.76 (d, J = 6.8 Hz, 3H).

**Compound 3**

**(R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone**

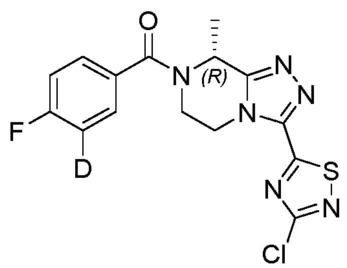

**Synthesis of intermediate 3-chloro-5-[(8R)-8-methyl-5,6,7,8-tetrahydro-[1,2,4]triazolo[4,3-a]pyrazin-3-yl]-1,2,4-thiadiazole:**

**Step 1: 3-chloro-5-(1-ethoxyvinyl)-1,2,4-thiadiazole**

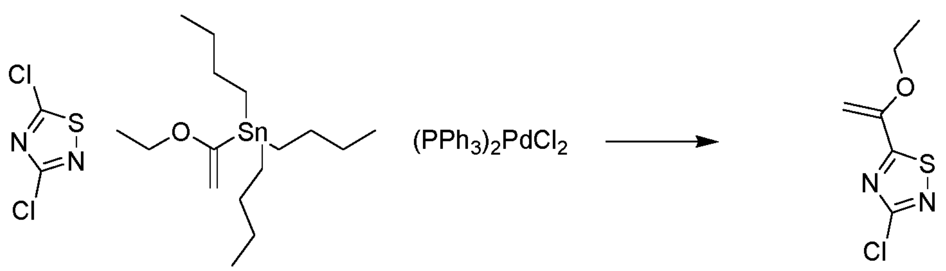


Bistriphenylphosphine palladium dichloride (1.13 g, 1.61 mmol) was added to 3,5-dichloro-1,2,4-thiadiazole (5 g, 32.26 mmol) and tributyl(1-ethoxyethylene)tin (10.48 g, 29.03 mmol) in anhydrous DMF (20 ml), and the reaction solution was heated to 75°C under nitrogen protection for 14 hours. The reaction solution was cooled, quenched with aqueous

potassium fluoride solution (50 ml), and filtered to remove the solid, the liquid phase was extracted with ethyl acetate (75ml*2), and the organic phase was washed with saturated brine (20ml*6), dried and evaporated to obtain the crude product. The crude product was subjected to column separation (petroleum ether: ethyl acetate = 99 : 1) to obtain the product 3-chloro-5-(1-ethoxyvinyl)-1,2,4-thiadiazole (4.3 g, yield: 70%).

$^1$H NMR (400 MHz, Chloroform-d) δ 5.52 (d, J = 3.1 Hz, 1H), 4.58 (d, J = 3.1 Hz, 1H), 4.02 (q, J = 7.0 Hz, 2H), 1.43 (t, J = 7.0 Hz, 3H).

**Step 2: Ethyl 3-chloro-1,2,4-thiadiazole-5-carboxylate**

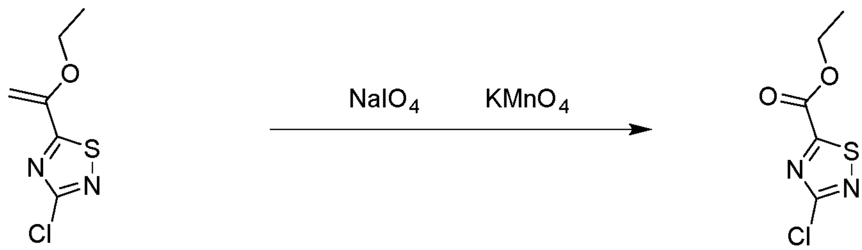


A solution of sodium periodate (8.30 g, 38.81 mmol) in water (35 ml) was added to a solution of 3-chloro-5-(1-ethoxyvinyl)-1,2,4-thiadiazole (3.7 g, 19.41 mmol) in dioxane (70 ml), the reaction solution was stirred for 2 minutes, then potassium permanganate (460.05 mg, 2.91 mmol) was added to the reaction solution, and the reaction solution was stirred at room temperature overnight. The reaction solution was extracted with ethyl acetate (100 mL × 3), and the organic phases were combined, washed with saturated brine (50 mL × 2), dried over anhydrous sodium sulfate, and concentrated under reduced pressure. The residue was purified by flash silica gel chromatography (petroleum ether: ethyl acetate=100 : 0 to 95 : 5 elution) to obtain the target product ethyl 3-chloro-1,2,4-thiadiazole-5-carboxylate (1 g, yield: 27.03%).

$^1$H NMR (400 MHz, Chloroform-d) δ 4.54 (q, J = 7.1 Hz, 2H), 1.47 (t, J = 7.2 Hz, 3H).

**Step 3: 3-chloro-1,2,4-thiadiazole-5-formylhydrazine**

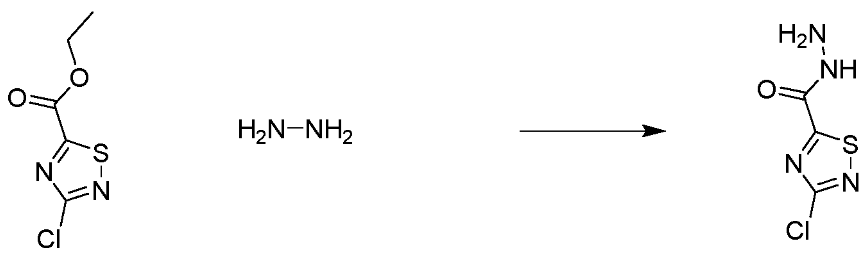

Hydrazine hydrate (229 mg, 3.89 mmol, 85% aqueous) was added to a solution of ethyl 3-chloro-1,2,4-thiadiazole-5-carboxylate (0.5 g, 2.6 mmol) in ethanol (10 ml). The reaction solution was stirred at room temperature for 1 hour. The solid product 3-chloro-1,2,4-thiadiazole-5-formylhydrazine (440 mg, yield: 95%) was then obtained by filtration.

MS m/z (ESI):179.0 [M+H]$^+$.

**Step 4: (5R)-4-[(2,4-dimethoxyphenyl)methyl]-6-ethoxy-5-methyl-3,5-dihydro-2H-pyrazine**

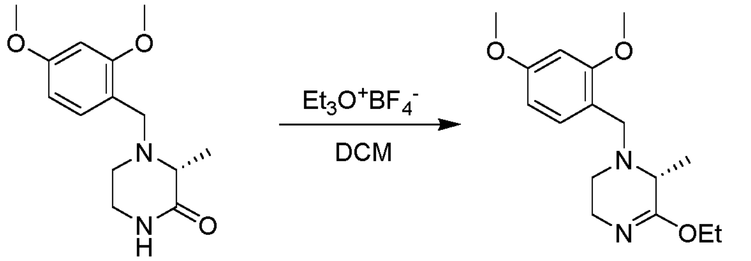


(3R)-4-[(2,4-dimethoxyphenyl)methyl]-3-methyl-piperazin-2-one (0.6 g, 2.27 mmol) was dissolved in dichloromethane (7 mL), and at 0°C, triethyloxonium tetrafluoroborate (1.08 g, 5.67 mmol) was added in three batches, with an interval of 10 minutes between each batch. The mixture was reacted with stirring for 3 hours at 25°C. The reaction solution was added to cold aqueous sodium hydroxide solution (2 M, 10 ml), and the mixture was subjected to liquid separation. The aqueous phase was extracted with dichloromethane (10mL × 2), and the organic phases were combined, dried over anhydrous sodium sulfate, and concentrated under reduced pressure to obtain the target product (5R)-4-[(2,4-dimethoxyphenyl)methyl]-6-ethoxy-5-methyl-3,5-dihydro-2H-pyrazine (0.65g) as a colorless oil. The crude product was directly used for the next step.

MS m/z (ESI): 293.2[M+H]$^+$.

**Step 5: 3-chloro-5-[(8R)-7-[(2,4-dimethoxyphenyl)methyl]-8-methyl-6,8-dihydro-5H-[1,2,4]triazolo[4,3-a]pyrazin-3-yl]-1,2,4-thiadiazole**

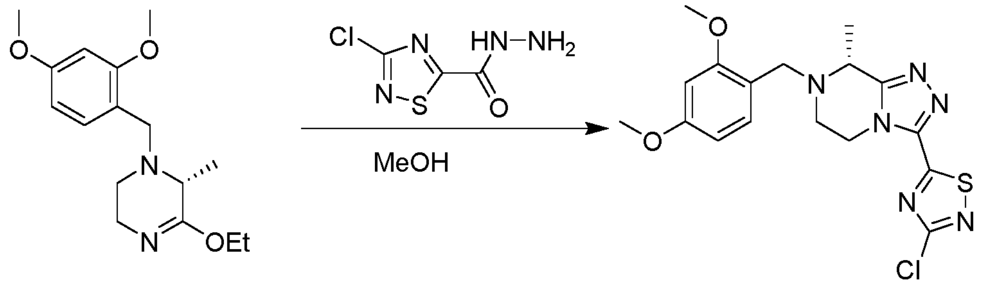


(5R)-4-[(2,4-dimethoxyphenyl)methyl]-6-ethoxy-5-methyl-3,5-dihydro-2H-pyrazine (0.66 g, 2.26 mmol) and 3-chloro-1,2,4-thiadiazole-5-formylhydrazine (322.54 mg, 1.81 mmol) were dissolved in MeOH (10 mL), and the mixture was reacted with stirring at 80°C under nitrogen protection for 14 hours. The reaction solution was concentrated under reduced pressure, and the residue was purified by flash silica gel chromatography (dichloromethane : methanol=100 : 0 to 95 : 5 elution) to obtain the target product 3-chloro-5-[(8R)-7-[(2,4-dimethoxyphenyl)methyl]-8-methyl-6,8-dihydro-5H-[1,2,4]triazolo[4,3-a]pyrazin-3-yl]-1,2,4-thiadiazole (0.22 g, yield: 23.95%) as a light yellow solid.

MS m/z (ESI): 406.8[M+H]$^+$.

**Step 6: 3-chloro-5-[(8R)-8-methyl -5,6,7,8-tetrahydro-[1,2,4]triazolo[4,3-a]pyrazin-3-yl]-1,2,4-thiadiazole**

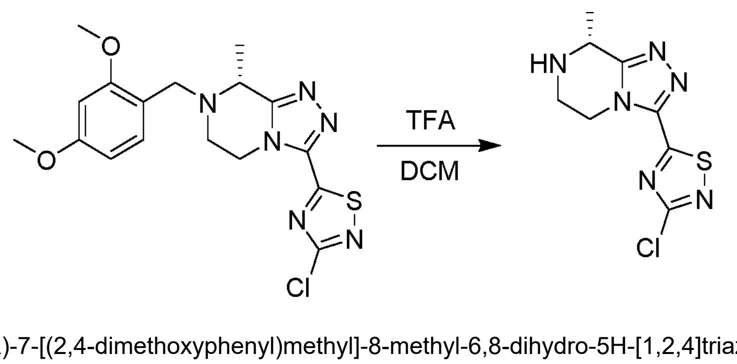


3-Chloro-5-[(8R)-7-[(2,4-dimethoxyphenyl)methyl]-8-methyl-6,8-dihydro-5H-[1,2,4]triazolo[4,3-a]pyrazin-3-yl]-1,2,4-thiadiazole (0.22 g, 540.69 umol) was dissolved in dichloromethane (1.5 mL), and trifluoroacetic acid (3 mL) was added. The mixture was reacted with stirring for 1 hour at 25°C. The reaction solution was evaporated to dryness under reduced pressure, then water (7 ml) was added, and the mixture was stirred at room temperature for 10 minutes, and then filtered to remove the solid. The liquid phase was lyophilized to obtain the product, a trifluoroacetate salt of 3-chloro-5-[(8R)-8-methyl-5,6,7,8-tetrahydro-[1,2,4]triazolo[4,3-a]pyrazin-3-yl]-1,2,4-thiadiazol (0.2 g, yield: 99.77%).

MS m/z (ESI): 257.0[M+H]+.

**Synthesis of intermediate 3-deutero-4-fluoro-benzoyl chloride:**

**Step 1: Preparation of 3-deutero-4-fluoro-benzoic acid**

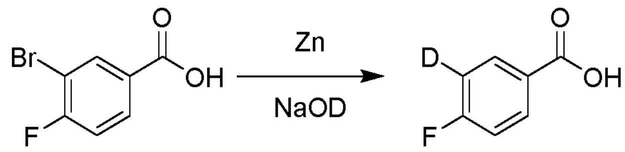


3-Bromo-4-fluorobenzoic acid (5 g, 22.83 mmol) was dissolved in deuterated sodium hydroxide (30 mL, 2 M in $D_2O$), and zinc powder (5.97 g, 91.32 mmol) was added. The mixture was reacted with stirring for 24 hours at 25°C. The reaction solution was filtered to remove the solid. The aqueous phase was adjusted to pH = 1 with 1N HCl. The aqueous phase was extracted with ethyl acetate (50 mL×3). The organic phases were combined, dried over anhydrous sodium sulfate, and concentrated under reduced pressure to obtain the product 3-deutero-4-fluoro-benzoic acid (3 g, yield: 93.12%).
MS m/z (ESI): 139.8[M-H]-

**Step 2: Preparation of 3-deutero-4-fluoro-benzoyl chloride**

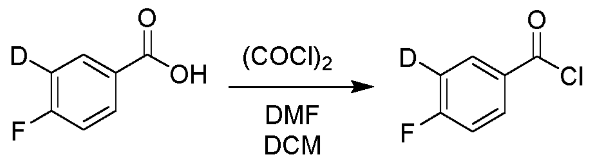


3-Deutero-4-fluoro-benzoic acid (200 mg, 1.42 mmol) was dissolved in dichloromethane (5 mL), oxalyl chloride (359.77 mg, 2.83 mmol) was added, followed by N,N-dimethylformamide (0.05 mL), and the mixture was reacted with stirring at 25 °C for 2 hours. The reaction solution was concentrated under reduced pressure to obtain the residue, 3-deutero-4-fluoro-benzoyl chloride (0.22 g), which was used directly in the next step.

**Synthesis of the target product (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone:**

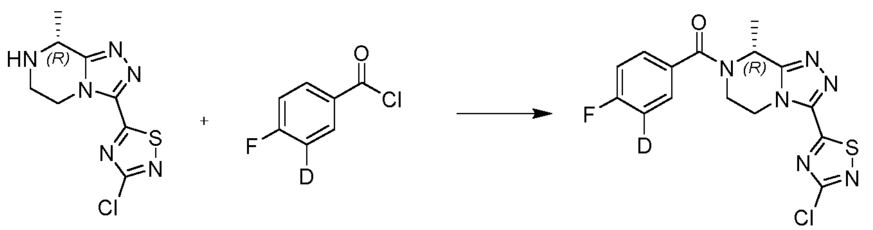

The trifluoroacetate salt of 3-chloro-5-[(8R)-8-methyl-5,6,7,8-tetrahydro-[1,2,4]triazolo[4,3-a]pyrazin-3-yl]-1,2,4-thiadiazole (0.35 g, 0.94 mmol) and triethylamine (286.59 mg, 2.83 mmol) were dissolved in dichloromethane (5 mL), and 3-deutero-4-fluoro-benzoyl chloride (210.89 mg, 1.32 mmol) was added. The mixture was reacted with stirring

for 20 minutes at 25°C. Saturated brine (5 mL) was added to the reaction solution to quench the reaction. The mixture was subjected to liquid separation. The organic phase was dried over anhydrous sodium sulfate and concentrated under reduced pressure to obtain a crude product. The crude product was separated by preparative high performance liquid chromatography to obtain the product (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone (66 mg, 172.03 umol, yield: 18.22%) as a white solid.

MS m/z (ESI): 379.8 $[M+H]^+$.

$^1H$ NMR (400 MHz, Chloroform-d) δ 7.58 - 7.40 (m, 2H), 7.22 - 7.10 (m, 1H), 5.99 - 5.61 (m, 1H), 5.01 - 4.80 (m, 1H), 4.80 - 4.43 (m, 1H), 4.38 - 4.16 (m, 1H), 3.68 - 3.45 (m, 1H), 1.88 - 1.68 (m, 3H).

**Compound 4**

**(R)-(3-(3-chloro-1,2,4-thiadiazol-5-yl)-8-methyl-5,6-dihydro-[1,2,4]triazolo[4,3-a]pyrazin-7(8H)-yl)(4-chlorophenyl-3-d)methanone**

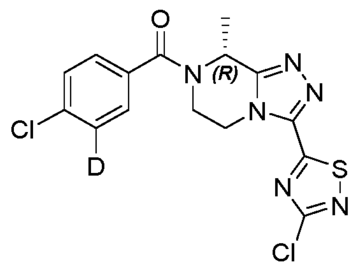

**Step 1: Preparation of 3-deutero-4-chloro-benzoic acid**

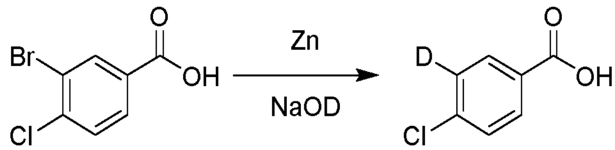


3-Bromo-4-chlorobenzoic acid (5 g, 21.23 mmol) was dissolved in 2 M $NaOD/D_2O$ (15 mL), and zinc powder (5.55 g, 84.94 mmol) was added. The mixture was reacted with stirring for 96 hours at 25°C. The reaction solution was filtered to remove the solid. The aqueous phase was adjusted to pH = 1 with 1N HCl. The aqueous phase was extracted with ethyl acetate (50 mL × 3). The organic phases were combined, dried over anhydrous sodium sulfate, and concentrated under reduced pressure to obtain the crude product. The crude product was separated by preparative high performance liquid chromatography to obtain the product 3-deutero-4-chloro-benzoic acid (1.3 g, yield: 38.85%). MS m/z (ESI): 156.0$[M-H]^-$

**Step 2: Preparation of 3-deutero-4-chloro-benzoyl chloride**

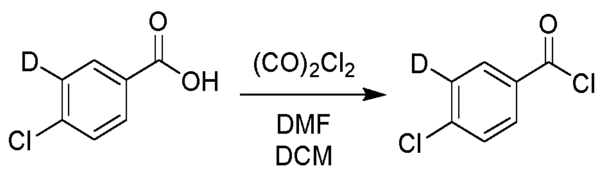


3-Deutero-4-chloro-benzoic acid (0.1 g, 634.63 umol) was dissolved in DCM (5 mL), oxalyl chloride (161.10 mg, 1.27 mmol) was added, followed by DMF (0.05 mL), and the mixture was reacted with stirring at 25°C for 2 hours. The reaction solution was concentrated under reduced pressure, to obtain the residue 3-deutero-4-chloro-benzoyl chloride (110 mg), which was used directly in the next step.

**Step 3: Referring to compound 3 for the preparation of compound 4.**

MS m/z (ESI): 395.9 [M+H]+.

$^1$H NMR (400 MHz, Chloroform-d) δ 7.51 - 7.44 (m, 1H), 7.44 - 7.36 (m, 2H), 6.12 - 5.57 (m, 1H), 4.99 - 4.79 (m, 1H), 4.78 - 4.35 (m, 1H), 4.35 - 4.15 (m, 1H), 3.67 - 3.42 (m, 1H), 1.76 (d, J = 6.9 Hz, 3H).

**Example 5**

**(R)-(3-(3-fluoro-1,2,4-thiadiazol-5-yl)-8-methyl-5,6-dihydro-[1,2,4]triazolo[4,3-a]pyrazin-7(8H)-yl)(4-fluorophenyl-3-d)methanone**

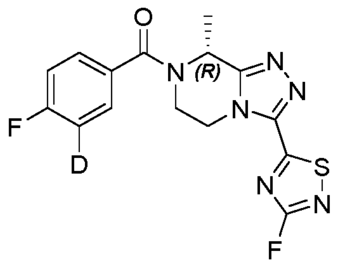

Referring to compound 3 for the preparation of compound 5.

MS m/z (ESI): 364.1 [M+H]+.

**Compound 6**

**(R)-(3-(3-(3,3-difluoroazetidin-1-yl)-1,2,4-thiadiazol-5-yl)-8-methyl-5,6-dihydro-[1,2,4]triazolo[4,3-a]pyrazin-7(8H)-yl)(4-fluorophenyl-3-d)methanone**

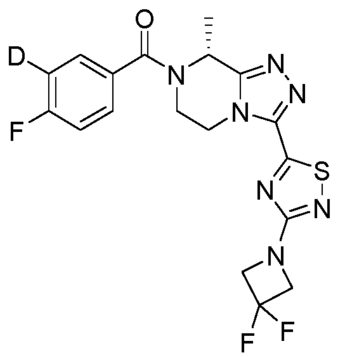

Step 1: (R)-3-(3,3-difluoroazetidin-1-yl)-5-(7-(2,4-dimethoxybenzyl)-8-methyl-5,6,7,8-tetrahydro-[1,2,4]triazolo[4,3-a]pyrazin-3-yl)-1,2,4-thiadiazole

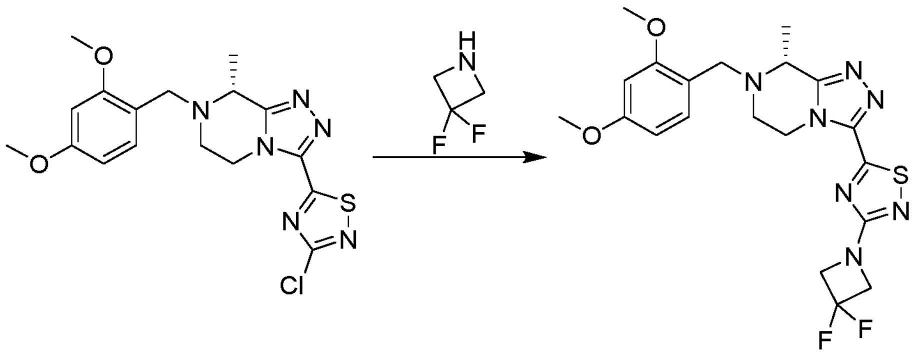

(R)-3-Chloro-5-(7-(2,4-dimethoxybenzyl)-8-methyl-5,6,7,8-tetrahydro-[1,2,4]triazolo[4,3-a]pyrazin-3-yl)-1,2,4-thiadiazole (75 mg, 184.33 μmol), 3,3-difluoroazetidine hydrochloride (238.77 mg, 1.84 mmol), and N,N-diisopropylethylamine (476.46 mg, 3.69 mmol, 642.12 μL) were dissolved in N,N-dimethylformamide (5 mL), and cesium carbonate (180.17 mg, 552.98 μmol) was added. The mixture was reacted under microwave conditions for 1 hour at 110°C. The reaction solution was concentrated under reduced pressure, and the residue was purified by flash silica gel chromatography (dichloromethane : methanol=20 : 1) to obtain the target product (R)-3-(3,3-difluoroazetidin-1-yl)-5-(7-(2,4-dimethoxybenzyl)-8-methyl-5,6,7,8-tetrahydro-[1,2,4]triazolo[4,3-a]pyrazin-3-yl)-1,2,4-thiadiazole (60 mg, 129.45 μmol, yield: 70.23%).

MS m/z (ESI): 464.2[M+H]$^+$.

Referring to reference compound A for subsequent steps to obtain (R)-(3-(3-(3,3-difluoroazetidin-1-yl)-1,2,4-thiadiazol-5-yl)-8-methyl-5,6-dihydro-[1,2,4]triazolo[4,3-a]pyrazin-7(8H)-yl)(4-fluorophenyl-3-d)methanone

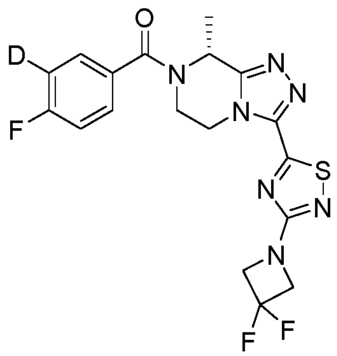

MS m/z (ESI): 437.1[M+H]$^+$.

$^1$H NMR (400 MHz, Chloroform-d) δ 7.55 - 7.41 (m, 2H), 7.23 - 7.11 (m, 1H), 5.97 - 5.51 (m, 1H), 4.90 - 4.76 (m, 1H), 4.71 - 4.44 (m, 5H), 4.35 - 4.12 (m, 1H), 3.62 - 3.38 (m, 1H), 1.75 (d, J = 6.9 Hz, 3H).

**Compound 7**

**(R)-(3-(3-(3-fluoroazetidin-1-yl)-1,2,4-thiadiazol-5-yl)-8-methyl-5,6-dihydro-[1,2,4]triazolo[4,3-a]pyrazin-7(8H)-yl)(4-fluorophenyl-3-d)methanone**

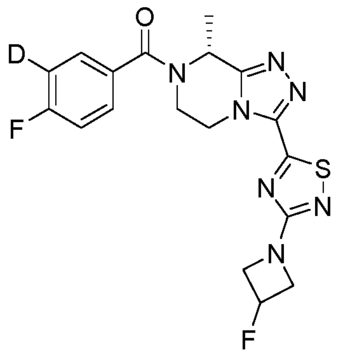

Referring to compound 6 for the preparation of compound 7.

MS m/z (ESI): 419.1[M+H]$^+$.

$^1$H NMR (400 MHz, Chloroform-d) δ 7.58 - 7.39 (m, 2H), 7.23 - 7.12 (m, 1H), 5.92 - 5.62 (m, 1H), 5.58 -5.33 (m, 1H), 4.90 - 4.76 (m, 1H), 4.72 - 4.03 (m, 6H), 3.63 - 3.40 (m, 1H), 1.75 (d, J = 6.9 Hz, 3H).

**Compound 9**

**(R)-(3-(3-(azetidin-1-yl)-1,2,4-thiadiazol-5-yl)-8-methyl-5,6-dihydro-[1,2,4]triazolo[4,3-a]pyrazin-7(8H)-yl)(4-fluorophenyl-3-d)methanone**

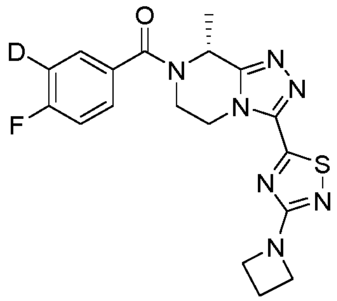

Referring to compound 6 for the preparation of compound 8.

MS m/z (ESI):401.1 [M+H]$^+$.

$^1$H NMR (400 MHz, Chloroform-d) δ 7.55 - 7.40 (m, 2H), 7.23 - 7.12 (m, 1H), 5.85 - 5.62 (m, 1H), 4.91-4.78 (m, 1H), 4.75 - 4.52 (m, 1H), 4.35 - 4.12 (m, 4H), 3.74 - 3.59 (m, 1H), 3.59 - 3.43 (m, 1H), 2.54 - 2.38 (m, 2H), 1.75 (d, J = 6.9 Hz, 3H).

<u>**Biological test and evaluation**</u>

The present invention will be further described and explained below in conjunction with test examples, but these examples are not meant to limit the scope of the present invention.

**Test example 1. Determination of the effect of compounds of the present invention on calcium ion mobilization capacity in cells stably expressing NK3 receptor**

Experimental purpose: The purpose of this test example is to measure the inhibitory effect of compounds on NK3 receptor.

Experimental instruments:

384-well assay plate (Corning; 3712);

Pipette (Axygen);

FLIPR (Molecular Devices).

Experimental reagents:

DMEM (Invitrogen; 11965);

Fetal bovine serum (Biowest; S1810-500);

Dialyzed serum (S-FBS-AU-065; Serana);

Penicillin and streptomycin (Biowest; L0022-100);

Hygromycin B (CABIOCHEM, 400052);

Matrigel (BD; 354230);

DMSO (Sigma; D2650);

HBSS (Invitrogen; 14065);

HEPES (Invitrogen; 15630080);

Probenecid (Sigma; P8761);

BSA (renview; FA016);

Trypsin (HDB; 0458).

Experimental method:

1. Buffer preparation: 1 × HBSS, 20 mM HEPES, 2.5 mM probenecid (probenecid is 400 mM stock in 1 M NaOH), 0.1% BSA; freshly prepared probenecid and BSA were added on the day of the experiment; the experimental buffers include a dye buffer, a compound dilution buffer, etc.

2. The cells were digested with trypsin, then seeded into a 384-well assay plate at a density of $1 \times 10^4$ cells/well, and incubated for 16-24 hours (at least overnight).

3. The culture medium was discarded, and 20 μL of dye was added; incubation was performed at 37°C for 60 min in the dark, and the calcium signals were read.

4. An antagonist was prepared before experiment; 5 × concentration of the antagonist compound was added into a 384-well assay plate at 5 uL/well, and incubated at room temperature for 15 min in the dark; the assay plate was transferred to FLIPR, and 6 × concentration of agonist compound was added at 5 uL/well; the values were read using FLIPR, and the data was saved; and the total assay volume in the well was 30 uL, including 20 uL of dye buffer, 5 uL of 5 × concentration of experimental compound and 5 uL of 6 × concentration of agonist compound.

Experimental data processing method:
The calcium signal values were read using FLIPR, the calculated output result at each sampling time point in the experiment was the ratio of signal at wavelength of 340/510 nm to signal at wavelength of 380/510 nm, and the maximum value minus the minimum value was calculated according to the signal ratio curve; GraphPad prism was used to fit percent inhibition rate and ten-point concentration data to a parametric nonlinear logistic formula to calculate the $IC_{50}$ value of the compound.

Experimental results:

Table 9: $IC_{50}$ value of compounds for calcium ion mobilization capacity in cells stably expressing NK3 receptor

| Example number | NK3R $IC_{50}$ (nM) FLIPR |
|---|---|
| Fezolinetant | 904.32 |
| Compound 1 | 146.99 |
| Compound 2 | 219.07 |
| Compound 3 | 154.85 |
| Compound 4 | 266.65 |

Experimental conclusion:

It can be seen from the data in the table that the compounds of formula I of the present invention show good inhibitory activity in the calcium flux assay in cells stably expressing NK3.

**Test example 2. Determination of the effect of compounds of the present invention on calcium ion mobilization capacity in cells stably expressing NK1/NK2 receptor**

Experimental purpose: The purpose of this test example is to measure the inhibitory effect of compounds on NK1/NK2 receptor.

Experimental instruments:

384-well assay plate (Corning; 3712);

Pipette (Axygen);

FLIPR (Molecular Devices).

Experimental reagents:

DMEM (Invitrogen; 11965);

Fetal bovine serum (Biowest; S1810-500);

Dialyzed serum (S-FBS-AU-065; Serana);

Penicillin and streptomycin (Biowest; L0022-100);

Hygromycin B (CABIOCHEM, 400052);

Matrigel (BD; 354230);

DMSO (Sigma; D2650);

HBSS (Invitrogen; 14065);

HEPES (Invitrogen; 15630080);

Probenecid (Sigma; P8761);

BSA (renview; FA016);

Trypsin (HDB; 0458).

Experimental method:

1. Buffer preparation: 1 × HBSS, 20 mM HEPES, 2.5 mM probenecid (probenecid is 400 mM stock in 1 M NaOH), 0.1% BSA. Freshly prepared probenecid and BSA were added on the day of the experiment. The experimental buffers include a dye buffer, a compound dilution buffer, etc.

2. The cells were digested with trypsin, then seeded into a 384-well assay plate at a density of 1 × $10^4$ cells/well, and incubated for 16-24 hours (at least overnight).

3. The culture medium was discarded, and 20 μL of dye was added. Incubation was performed at 37°C for 60 min in the dark, and the calcium signals were read.

4. An antagonist was prepared before experiment. Antagonist mode: 5 × concentration of the antagonist compound was added into a 384-well assay plate at 5 uL/well, and incubated at room temperature for 15 min in the dark. The assay plate was transferred to FLIPR, and 6 × concentration of agonist compound was added at 5 uL/well. The values were read using FLIPR, and the data was saved. The total assay volume in the well was 30 uL, including 20 uL of dye buffer, 5 uL of 5 × concentration of experimental compound and 5 uL of 6 × concentration of agonist compound.

Experimental data processing method:
The calcium signal values were read using FLIPR. The calculated output result at each sampling time point in the experiment was the ratio of signal at wavelength of 340/510 nm to signal at wavelength of 380/510 nm. The maximum value minus the minimum value was calculated according to the signal ratio curve. GraphPad prism was used to fit percent inhibition rate and ten-point concentration data to a parametric nonlinear logistic formula to calculate the $IC_{50}$ value of the compound.

Experimental results:

The compounds of the present invention exhibit both NK1R $IC_{50}$ (nM) value and NK2R $IC_{50}$ (nM) value > 10,000 for the calcium ion mobilization capacity in cells stably expressing NK1/NK2 receptor.

Experimental conclusion:

The compounds of the present invention show good selectivity in the calcium flux assay in cells stably expressing NK3 and NK1/NK2 receptors.

**Test example 3. Determination of the effect of compounds of the present invention on IP1 in cells stably expressing HEK293-NK3**

1. Experimental purpose:

The purpose of this test example is to measure the antagonistic effect of compounds on HEK293-NK3 cells.

2. Experimental instruments and reagents:

2.1 Experimental instruments and consumables:

384-well cell plate (Corning; 3824);

384-well Echo compound plate (Labcyte: LP-0200);

Bravo Tip (Agilent: 10734-202);

Plate reader EnVision Multilabel Reader (PE: 2104-0010);

Pipetting workstations Bravo (Agilent) and ECHO 550 (LABCYTE);

Liquid injector (Multidrop Combi).

2.2 Experimental reagents:

DMEM, high glucose (Gibco: 12100);

Fetal bovine serum (Biosera: FB-1058/500);

P/S (Biosera: XC-A4122);

5× Matrigel (Corning: 354230);

IP-ONE - Gq Kit (Cisbio: 62IPAPEJ);

Agonist Senktide (MCE: HY-P0187);

Positive control compound Talnetant (MCE: HY-14552);

Complete culture medium: DMEM + 10% FBS + 1× P/S; 1× Matrigel: 5× Matrigel was diluted with DMEM;

Cell line: HDB HEK293-NK3, HD Biosciences Co., Ltd.

3. Experimental method:

1. A 150 mm culture dish was coated with 1× Matrigel at room temperature for 10 minutes.

2. HEK293-NK3 cell line was seeded in the coated culture dish, and cultured with a complete medium at 37°C, 5% $CO_2$ to 70% to 90% confluence.

3. A positive control compound and test compounds were prepared:

1) instrument Bravo was used to dilute the compound to 11 concentration points on the 384-well Echo compound plate (LABCYTE: LP-0200);

2) then the instrument ECHO was used to transfer 10 nL of compound per well (compound storage concentration, for example, as the highest concentration point 10 mM) to the 384-well compound plate (Corning: 3824); and the compound was placed at room temperature for later use.

4. The cells were digested and resuspended in 1× Stimulation Buffer (IP-ONE-Gq Kit), seeded into a 384-well cell culture plate (contained the test compound) at 5000 cells/well/5 μL using Multidrop Combi, centrifuged at 300 rpm for 60 seconds, and then incubated at 37°C, 5% $CO_2$ for 15 minutes.

5. The cell culture plate was taken out from the $CO_2$ incubator, 5 μL of 2× $EC_{80}$ (final concentration 3 nM) was added using Multidrop Combi, centrifugation was performed at 300 rpm for 60 seconds at room temperature, and incubation was then performed at 37°C, 5% $CO_2$ for 2 hours.

6. The cell culture plate was taken out from the $CO_2$ incubator, 5 μL of IP1 d2 reagent was added using Multidrop Combi, 5 μL of IP1 Tb Cryptate Antibody reagent was added, centrifugation was performed at 300 rpm for 60 seconds at room temperature, and same was then placed at room temperature for 1 hour.

7. The plate was read using EnVision Multilabel Reader while the data was read and collected.

Experimental data processing method:
EnVision Multilabel Reader was used to read and collect the ratio (Ratio) of fluorescence signal values. The percent

antagonism data based on the readings of the Low control (DMSO control) and High control (positive compound) experimental groups was calculated: {% antagonism rate = (Ratio sample-Ratio low control)/(Ratio high control-Ratio low control)× 100}. The concentrations of the test compound were 11 concentrations after diluting the reaction system 3 times, ranging from 10 uM to 0.17 nM. XLFit was used to fit the percentage antagonism rate and 11-point concentration data to a parametric nonlinear logic formula to calculate the $IC_{50}$ value of the compound.

4. Experimental results:

Table 10 ICso values of compounds for antagonism on IP1 in cells stably expressing NK3 receptor

| Example number | NK3R $IC_{50}$ (nM) IP1 |
|---|---|
| Compound 2 | 88.32 |
| Compound 3 | 75.99 |

5. Experimental conclusion:

It can be seen from the data in the table that the compounds of the present invention show good antagonistic activity in the IP1 function assay in cells stably expressing NK3.

**Test example 4. Pharmacokinetic assay in SD rats**

1. Study purpose:

SD rats were used as test animals to study the pharmacokinetic behavior of the compound of the present invention in the rat body (plasma and brain tissue) after oral administration at a dose of 5 mg/kg.

2. Experimental scheme:

2.1 Experimental drugs:

the example compound of the present invention, made in house.

2.2 Experimental animals:

21 SD rats/group, male, from Shanghai Jiesijie Experimental Animal Co., Ltd., animal production license number (SCXK (Shanghai) 2013-0006 N0.311620400001794).

2.3 Formulation prescription:

0.5% CMC-Na (1% Tween80) was dissolved with ultrasound, and formulated into a clear solution or homogeneous suspension.

2.4 Administration:

21 SD rats/group, male; p.o. respectively at a dose of 5 mg/kg and an administration volume of 10 mL/kg after the rats were fasted overnight.

2.5 Sample collection:

Rats were sacrificed with $CO_2$ before administration and at 0, 0.5, 1, 2, 4, 8 and 24 hours after administration, 8 mL of blood was taken from the heart, and placed in an EDTA-$K_2$ test tube, and the plasma was separated by centrifugation at 6000 rpm for 6 minutes at 4°C, and stored at - 80°C; the whole brain tissue was taken out and weighed, and then placed in a 2 mL centrifugal tube and stored at -80°C.

2.6 Sample processing:

1) 160 uL of acetonitrile was added to 40 uL of plasma sample for precipitation, and same was mixed and centrifuged at 3500 × g for 5 to 20 min.

2) 90 μL of acetonitrile containing internal standard (100 ng/mL) was added to 30 μL of plasma and brain homogenate samples for precipitation, and same was mixed and centrifuged at 13000 rpm for 8 min.

3) 70 uL of the treated supernatant solution was taken, 70 μL of water was added, vortex mixing was performed for 10 minutes, and then 20 μL of the resulting mixture was taken and subjected to LC/MS/MS to analyze the concentration of the test compound. LC/MS/MS analysis instrument: AB Sciex API 4000 Qtrap.

2.7 Liquid phase analysis:

• Liquid phase conditions: Shimadzu LC-20AD pump
• Chromatographic column: Agilent ZORBAX XDB-C18 (50 × 2.1 mm, 3.5 μm) mobile phase: liquid A is 0.1% aqueous formic acid solution, liquid B is acetonitrile
• flow rate: 0.4 mL/min
• Elution time: 0-4.0 min, the eluent is as follows:

| time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

The main pharmacokinetic parameters were calculated using WinNonlin 6.1. The results of pharmacokinetic experiments in rats are as shown in Table 3 below:

Table 11: Results of pharmacokinetic experiments in rats

| Example | Pharmacokinetic experiments (5 mg/kg) | | | | |
|---|---|---|---|---|---|
| | Peak time | Blood drug concentration | Curve area | Half-time | Average retention time |
| | $t_{max}$ (h) | $C_{max}$ (ng/mL) | $AUC_{0-t}$ (ng/mL× h) | $t_{1/2}$ (h) | MRT (h) |
| Compound 3 Plasma | 2.0 | 1170 | 6147 | 3.1 | 5.1 |
| Compound 3 Brain | 1.0 | 798 | 2710 | NA | NA |
| NA: not detected. | | | | | |

4. Experimental conclusion:

It can be seen from the results of the pharmacokinetic experiments in rats in the table: the example compounds of the present invention exhibit good metabolic properties, and perform well in both exposure (AUC) and maximum blood drug concentration $C_{max}$.

**Test example 5. Effect of compounds of the present invention on the content of luteinizing hormone (LH) in plasma of bilaterally ovariectomized (OVX) rats**

1. Experimental purpose:

The purpose of this test example is to measure the effect of compounds on luteinizing hormone (LH) in plasma of bilaterally ovariectomized (OVX) rats.

2. Experimental instruments and reagents:

2.1 Experimental instruments:

Balance (PR2202ZH/E, OHAUS);

Centrifuge (5424R, Eppendorf);

Microplate reader (BioTek Synergy H1);

Plate washer (Thermo Scientific);

Pipette (Eppendorf & Rainin);

Water purifier (Thermo Scientific);

2.2 Experimental reagents:

CMCNa (30036365, Sinopharm Chemical Reagent Co., Ltd.);

Tween 80 (30189828, Sinopharm Chemical Reagent Co., Ltd.);

Rat LH ELISA Kit (S-type) purchased from Shibayagi Japan, with catalog number of AKRLH-010S, comprising Assay buffer C, Sample dilution buffer G, Washing buffer, biotinylated anti-LH$\alpha$ antibody, HRP-conjugated streptavidin and Chromogen (TMB).

2.3 Experimental animals:

Bilaterally ovariectomized SD rats, aged 10 weeks, female, purchased from Beijing Vital River Laboratory Animal Technology Co., Ltd. The animals were kept in SPF-grade animal room, with 5 SD rats/cage. The cages, bedding, feed and water were subjected to high-temperature disinfection before use, and all animals had free access to food and water.

3. Experimental method:

Grouping and administration: Female SD rats aged 10 weeks were used for experiments at 3 weeks after bilateral ovariectomy. Before the experiment, the SD rats were fasted overnight, and then randomly grouped before administration, with 3 rats/group. Each administration group was orally administered a different test compound at a dose of 30 mg/kg and an administration volume of 10 mL/kg.

Sample collection: Before administration and at 0, 0.5, 1, 2, 4, 8 hours after administration, 0.2-0.3 mL of whole blood was collected from the SD rats in each group, respectively, placed in an EDTA-K2 test tube, shaken uniformly by inversion, and centrifuged at 5000 rpm for 5 min at 4°C, and then the plasma was separated, and stored in an ultra-low temperature refrigerator (-80°C $\pm$ 10°C).

Sample detection: All reagents, samples and ELISA plates were transferred to room temperature 20°C-25°C and equilibrated for at least 30 min, and dissolution, vortex and centrifugation were performed for later use. Standard curve regents were prepared using Assay buffer C in the concentration order of concentrations 10, 5, 2.5, 1.25, 0.625, 0.313, and 0. The sample was diluted twice using Sample dilution buffer G, and left to stand at room temperature for 10 min. The

sample from the previous step was diluted 2.5 times using Assay buffer C, and placed at room temperature for later use. 1× Washing buffer was added to the 96-well plate at 300 μL/well to wash the plate, with the operation repeating 4 times. Following the set-up of standard wells and sample wells, 50 μL of the corresponding standard and 50 μL of sample were added to each well, and mixed uniformly, the plate was sealed, and incubation was performed at 20°C-25°C for 2 h. 50 μL of 1× biotinylated anti-LHα antibody was added to each well, uniform mixing was performed, the plate was sealed, and incubation was performed at 20°C-25°C for 1 h. 1× Washing buffer was added at 300 μL/well to wash the plate, with the operation repeating 4 times. 50 μL of prepared 1× HRP-conjugated streptavidin was added to each well, uniform mixing was performed, the plate was sealed, and incubation was performed at 20°C-25°C for 0.5 h. 1× Washing buffer was added at 300 μL/well to wash the plate, with the operation repeating 4 times. 50 μL of Chromogen (TMB) was added to each well, uniform mixing was performed, and incubation was performed at 20°C-25°C for 20 min. 50 μL of stop solution (1 M $H_2SO_4$) was added to each well, and uniform mixing was performed. The OD value of each well was read using the microplate reader at a wavelength of 450 nm.

4. Experimental data processing method:

Graphpad was used to draw a standard curve, and sample concentrations were calculated. If the dilution was performed during the sample detection, the calculated sample concentration needed to be multiplied by the corresponding dilution ratio in the final calculation, which was the actual concentration of the sample.

5. Experimental results:

Table 12: Effect of compounds on the content of luteinizing hormone (LH) in plasma of bilaterally ovariectomized (OVX) rats

| **Example number** | **ΣAUC (ΔLH/LH•h, Mean ± SD)** |
|---|---|
| Compound 3 | -5.44 ± 3.02* (1.37 ± 1.98) |
| Compound 4 | -2.04 ± 1.36 (-1.84 ± 0.24) |

Remarks: data in parentheses indicate the corresponding Vehicle group (i.e., control group) in this example

6. Experimental conclusion:

It can be seen from the data in the table that the example compounds of the present invention can significantly reduce the content of LH in the plasma of ovariectomized (OVX) rats.

**Test example 6. Pharmacodynamic study of test compound in Senktide-induced bilaterally ovariectomized rat tail temperature model**

1. Experimental purpose:

The purpose of this test example is to evaluate the effect of test compound on Senktide-induced tail temperature in bilaterally ovariectomized rats.

2. Experimental instruments and reagents:

2.1 Instruments:

Balance (BSA2202s-CW, Sartorius);

Thermometer (BAT-10, Physitemp);

Probe (SST-1, Physitemp);

2.2 Reagents

Senktide (106128-89-6, MCE);

NaCl (10019318, Sinopharm Chemical Reagent Co., Ltd.);

DMSO (D2650-100ML, Sigma);

CMCNa (30036365, Sinopharm Chemical Reagent Co., Ltd.);

Tween 80 (30189828, Sinopharm Chemical Reagent Co., Ltd.).

2.3 Experimental animals

Bilaterally ovariectomized SD rats, aged 10 weeks, female, purchased from Beijing Vital River Laboratory Animal Technology Co., Ltd. The animals were kept in SPF-grade animal room, with 5 SD rats/cage. The cages, bedding, feed and water were subjected to high-temperature disinfection before use, and all animals had free access to food and water.

2.4 Test compound:

the example compound of the present invention, made in house.

3 Experimental operation:

Female SD rats aged 10 weeks, two weeks after recovery from bilateral ovariectomy, were randomly divided into a negative control group, a model group and an administration group according to body weight, with 8 rats/group. A medical tape was used to fix the probe on the dorsal side of the rat tail at a position 1-2 cm away from the root of the rat tail.

The negative control group and the model group were orally administered vehicle, and each administration group was orally administered a different test compound at a dose of 30 mg/kg and an administration volume of 10 mL/kg.

30 minutes after oral administration of vehicle or test compound, the negative control group was subcutaneously injected with physiological saline (injection volume of 5 mL/kg); the model group and each administration group were subcutaneously injected with Senktide at a concentration of 0.2 mg/mL (injection volume of 5 mL/kg) to induce hot flash-like symptoms. The tail skin temperature (TST) at 0 minutes before injection was measured and recorded; measurement was performed every 5 minutes after injection, with a cumulative measurement for 75 min.

4. Data processing:

The tail skin temperature change values (ΔTST) were calculated for each time point relative to point 0 and the ΔTST (tail skin temperature change value)-time curve was drawn, and the area under curve ($AUC_{\Delta TST}$) was calculated. $\Delta TST = TST_n - TST_0$, $TST_0$ is the tail skin temperature at 0 minutes before subcutaneous injection of physiological saline or Senktide; $TST_n$ is the tail skin temperature at the n minutes after subcutaneous injection.

$$AUC_{\Delta TST} = \sum(\Delta TST_n + \Delta TST_{n+5})*5/2$$

5. Experimental results:

Table 13: Pharmacodynamic data in OVX rat tail temperature models

| Example number | $AUC_{\Delta TST}$, °C•min |
|---|---|
| Fezolinetant | 116.7 ± 50.6 (150.2 ± 40.4) |

(continued)

| Example number | $AUC_{\Delta TST}$, °C•min |
|---|---|
| Compound 3 | 53.8 ± 19.8* (150.2 ± 40.4) |
| Remarks: data in parentheses indicate the corresponding Vehicle/Senktide group (i.e., control group) in this example | |

6. Experimental conclusion:

It can be seen from the data in the table that the example compounds of the present invention can effectively inhibit Senktide-induced hot flash symptom in ovariectomized rats.

**II. Study on compound and crystal form thereof**

**1. Terminology**

The term "pharmaceutically acceptable" as used herein refers to those compounds, materials, compositions and/or dosage forms that are, within the scope of sound medical judgment, suitable for contact with the tissues of human beings and animals without excessive toxicity, irritation, allergic response or other problems and complications and are commensurate with a reasonable benefit/risk ratio.

The term "substantially pure" as used herein means that in certain preferred embodiments of the present invention, the crystal form structure of the compound of formula I is in substantially pure form with an HPLC purity or crystal form purity of substantially 90% or more (inclusive), preferably 95% or more, more preferably 98% or more, and most preferably 99.5% or more.

The "crystal form" or "crystal form substance" as used herein refers to crystal forms that have the same chemical composition but different spatial arrangements of the molecules, atoms and/or ions constituting the crystal. While crystal forms and crystal form substances have the same chemical composition, they differ in packing and geometric arrangements, and may exhibit different physical properties, such as melting point, shape, color, density, hardness, deformability, stability, solubility, dissolution rate and similar properties. Depending on their temperature-stability relationship, two polymorphs may be either monotropic or enantiotropic. For a monotropic system, the relative stability between two solid phases remains unchanged as the temperature is changed. In contrast, in an enantiotropic system, there exists a transition temperature at which the stability of the two phases is swapped ((Theory and Origin of Polymorphism in "Polymorphism in Pharmaceutical Solids" (1999) ISBN:)-8247-0237).

Samples of the crystal form of the present invention may be provided with substantially pure phase homogeneity, indicating the presence of a dominant amount of a single crystal form structure and optionally minor amounts of one or more other crystal form structures. The presence of more than one crystal form structures of the present invention in a sample may be determined by techniques such as X-ray powder diffraction (XRPD) or solid state nuclear magnetic resonance (SSNMR). For example, the presence of extra peaks in the comparison of an experimentally measured XRPD pattern (observed) with a simulated XRPD pattern (calculated) may indicate the presence of more than one crystal form structure in the sample. The simulated XRPD may be calculated according to single crystal X-ray data (see Smith, D.K., "A FORTRAN Program for Calculating X-Ray Powder Diffraction Patterns," Lawrence Radiation Laboratory, Livermore, California, UCRL-7196, April 1963; see also Yin. S., Scaringe, R.P., DiMarco, J., Galella, M and Gougoutas, J.Z., American Pharmceutical Review. 2003.6.2.80). Preferably, the crystal form structure has substantially pure phase homogeneity as shown by less than 10%, preferably less than 5 %, and more preferably less than 2% of the total peak area in the experimentally measured XRPD pattern arising from the extra peaks that are absent from the simulated XRPD pattern. Most preferred is a crystal form structure of the present invention having substantially pure phase homogeneity with less than 1% of the total peak area in the experimentally measured XRPD pattern arising from the extra peaks that are absent from the simulated XRPD pattern.

The various crystal form structures of the present invention described herein may be distinguishable from one another through the use of various analytical techniques known to those of ordinary skill in the art. Such techniques include, but are not limited to, solid state nuclear magnetic resonance (SSNMR) spectroscopy, X-ray powder diffraction (XRPD), differential scanning calorimetry (DSC) and/or thermogravimetric analysis (TGA).

The crystal form structures of the present invention may be prepared by a variety of methods, including for example, crystallization or recrystallization from a suitable solvent, sublimation, growth from a melt, solid state transformation from another phase, crystallization from a supercritical fluid, and jet spraying. Techniques for crystallization or recrystallization of crystal form structures from a solvent mixture include, for example, evaporation of the solvent, decreasing the temperature of the solvent mixture, crystal seeding a supersaturated solvent mixture of the molecule

and/or salt, freeze drying the solvent mixture, and addition of antisolvents (countersolvents) to the solvent mixture. High throughput crystallization techniques may be used to prepare crystal form structures, including polymorphs.

Drug crystals, including polymorphs, and preparation methods and characterization of the drug crystals are discussed in Solid-State Chemistry of Drugs, S.R. Byrn, R.R. Pfeiffer, and J.G. Stowell, 2nd Edition, SSCI, West Lafayette, Indiana, 1999.

Seed crystals can be added to any crystal form mixture to promote crystallization. As will be clear to the skilled, seed crystals are used as a means of controlling the growth of a particular crystal form structure or as a means of controlling the particle size distribution of the crystal form product. Accordingly, calculation of the amount of seed crystals needed depends on the size of the seed crystal available and the desired average size of a product particle as described, for example, in "Programmed cooling of batch crystallizers," J. W. Mullin and J. Nyvlt, Chemical Engineering Science, 1971, 26, 369-377. In general, seed crystals of small size are needed to effectively control the growth of crystals in this batch. Seed crystals of small size may be generated by sieving, milling or micronizing of larger crystals, or by micro-crystallization of solutions. Care should be taken that milling or micronizing of crystals does not result in any change in crystallinity from the desired crystal structure (i.e., change to amorphous or to another polymorphic form).

Crystal structures equivalent to the crystal structures disclosed or claimed herein may exhibit similar, yet non-identical, analytical characteristics within a reasonable range of error, depending on test conditions, purity, equipment and other common variables known to those skilled in the art. Accordingly, it will be apparent to those skilled in the art that various modifications and variations may be made in the present invention without departing from the scope and spirit of the present invention. Other embodiments of the present invention will be apparent to those skilled in the art from consideration of the specification and practice of the present invention disclosed herein. Applicants intend that the specification and examples be considered as exemplary, but not limiting the scope thereof.

The term "room temperature" or "RT" as used herein refers to an ambient temperature from 20°C to 25°C (68°F-77°F).

## 2. Experiment materials

The reagents used in the examples of the present invention were commercially available industrial grade or analytical grade reagents, and the compounds were synthesized according to the methods of the following examples.

## 3. Analysis Method

### 3.1. X-ray powder diffraction

Those of ordinary skill in the art will appreciate that an X-ray powder diffraction pattern may be obtained with a measurement error that is dependent on the measurement conditions used. In particular, it is generally known that intensities in an X-ray powder diffraction pattern may fluctuate depending on material conditions used. It should be further understood that relative intensities may also vary with experimental conditions and, accordingly, the exact intensities should not be taken into account. Additionally, a measurement error of diffraction angle for a conventional X-ray powder diffraction pattern is typically about 5% or less, and such degree of measurement error should be taken into account as pertaining to the aforementioned diffraction angles. Therefore, it is to be understood that the crystal structures of the present invention are not limited to the crystal structures that provide X-ray diffraction patterns completely identical to the X-ray powder diffraction patterns depicted in the drawings disclosed herein. Any crystal structures that provide X-ray powder diffraction patterns substantially identical to those disclosed in the drawings fall within the scope of the present invention. The ability to determine whether X-ray powder diffraction patterns are substantially identical is within the capabilities of those of ordinary skill in the art. Calibration was performed with other suitable standard known to those skilled in the art. The relative intensities, however, may change with the crystal size and shape.

The polymorphic forms of the compound of formula I were characterized by their X-ray powder diffraction patterns. Therefore, the X-ray powder diffraction patterns of the salt were collected on a Bruker D8 Advance X-ray powder diffractometer with a GADDS (general area diffraction detector system) CS operating in a reflection mode and using Cu K$\alpha$ radiation (1.54 Å). The tube voltage and current were respectively set as 40 kV and 25 mA for acquisition scan. The sample was scanned for a period of 180 seconds in the 2$\theta$ range of 4.0° to 40°. The diffractometer was calibrated using corundum standards for peak positions expressed in 2$\theta$. All analyses were performed at room temperature, typically 20°C-30°C. Data were acquired and integrated using DIFFRAC.COMMANDER. Diffraction patterns were analyzed usingjade6 software. XRPD samples were prepared by placing the sample on a monocrystalline silicon wafer and pressing the sample powder with a glass sheet or equivalent to ensure that the surface of the sample is flat and of an appropriate height. The sample holder was then placed into the Bruker XRPD instrument and X-ray powder diffraction patterns were collected using the instrument parameters described above. Measurement differences associated with the results of such X-ray powder diffraction analysis are due to a variety of factors including: (a) errors in sample preparation (e.g., sample height),

(b) instrument errors, (c) calibration differences, (d) operator errors (including those occurring in determining peak positions), and (e) properties of the substance (e.g., preferred orientation errors). Calibration errors and sample height errors often cause all peaks to be shifted in the same direction. In general, this calibration factor will allow the measured peak position consistent with the expected peak position and within the expected range of 2θ value ± 0.2°.

The 2θ (º) values and intensity values (as % of the highest peak) of each polymorphic form obtained in the examples of the present invention are listed in Table 1-Table 6.

### 3.2. Differential scanning calorimetry

Differential scanning calorimetry (DSC) experiments were performed on a NETZSCH DSC 214 polyma instrument. The sample (about 1-6 milligrams) was weighed in an aluminum pan and recorded accurately to one hundredth of a milligram, and transferred to the DSC. The instrument was purged with nitrogen gas at 40 mL/min. Data were collected between 25°C and 300°C at a heating rate of 10°C/min. The plot was made with the endothermic peaks pointing down. However, those skilled in the art will note that in DSC measurements there is a certain degree of variability in actual measured onset and peak temperatures, depending on heating rate, crystal shape and purity, and other measurement parameters.

Specific examples and preparation method examples provided below will further illustrate specific aspects of embodiments of the present invention. The scope of the following examples is not intended to limit the scope of the present invention in any way.

### Example 1

40 mg of the free base of compound 3 was weighed, 10 mL of methanol was added, the resulting mixture was warmed to 60°C for dissolution, substantially dissolved to clarification, filtered, stirred at room temperature overnight, and filtered, and the solid was dried under vacuum at 50°C for 24 h to obtain (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone free base crystal form A.

The resulting free base crystal form A of compound 3 was analyzed by X-ray powder diffractometer (XRPD) (model BRUKER D8 ADVANCE) and differential scanning calorimeter (DSC) (model NETZSCH DSC 214 polyma) respectively. The XRPD analysis method parameters are as follows: CuK ray (40 kV, 25 mA), scanning step size: 0.02°/S (2θ value), scanning speed: 12°/min (2θ value), scanning range: 4° to 40° (2θ value). The DSC analysis method parameters are as follows: temperature range: 25°C to 300°C, scanning rate: 10°C per minute, protective gas: nitrogen (flow rate: 40 ml/min). The XRPD analysis pattern is as shown in FIG. 1, and the DSC analysis pattern is as shown in FIG. 7.

### Example 2

100 mg of the free base crystal form A of compound 3 was weighted, 2 mL of ethyl acetate was added, and to which was added hydrogen chloride in methanol (1 mol/L) in a 1 : 1.2 molar ratio. The resulting mixture was turbid, stirred at room temperature for 24 hours, and then filtered, and the solid was dried under vacuum at 50°C overnight to obtain compound hydrochloride as a solid, which had an XRPD pattern as shown in FIG. 2.

### Example 3

50 mg of the free base crystal form A of compound 3 was weighted, 400 μL of tetrahydrofuran was added, and to which was added methanesulfonic acid in methanol (1 mol/L) in a 1 : 1.2 molar ratio. The resulting clear solution was open at room temperature to evaporate the solvent to obtain a gel, and to which was added 400 μL of ethyl acetate. The resulting mixture was stirred at room temperature for 8 hours, and then filtered. The resulting solid was dried under vacuum at 50°C for 24 h to obtain compound methanesulfonate as a solid, which had an XRPD pattern as shown in FIG. 3 below.

### Example 4

50 mg of the free base crystal form A of compound 3 was weighted, 400 μL of tetrahydrofuran was added, and to which was added nitric acid in methanol (1 mol/L) in a 1 : 1.2 molar ratio. The resulting clear solution was open at room temperature to evaporate the solvent to obtain a gel, and to which was added 400 μL of ethyl acetate. The resulting mixture was stirred at room temperature for 8 hours, and then filtered. The resulting solid was dried under vacuum at 50°C for 24 h to obtain compound nitrate as a solid, which had an XRPD pattern as shown in FIG. 4 below.

**Example 5**

100 mg of the free base crystal form A of compound 3 was weighted, 2 mL of ethyl acetate was added, and to which was added sulfuric acid in methanol (1 mol/L) in a 1 : 1.2 molar ratio. The resulting mixture was turbid, stirred at room temperature for 24 hours, and then filtered, and the solid was dried under vacuum at 50°C overnight to obtain compound I sulfate as a solid, which had an XRPD pattern as shown in FIG. 5.

**Example 6**

100 mg of the free base crystal form A of compound 3 was weighted, 2 mL of ethyl acetate was added, and to which was added hydrobromic acid in methanol (1 mol/L) in a 1 : 1.2 molar ratio. The resulting mixture was turbid, stirred at room temperature for 24 hours, and then filtered, and the solid was dried under vacuum at 50°C overnight to obtain compound hydrobromide as a solid, which had an XRPD pattern as shown in FIG. 6.

**Example 7: Experimental study on stability of sample of crystal form A**

The free base crystal form A of compound 3 of the present invention was taken to prepare a sample, the sample was placed under the conditions of illumination 5000lx, high temperature 60°C, high humidity 92.5% RH, and high temperature and high humidity 50°C 75% RH, the 5-day stability and 10-day stability were investigated, a HPLC external standard method was used to determine the content, and a chromatographic peak area normalization method was used to calculate the change in related substances. The experimental results are as shown in Table 7:

**Table 7: Experimental results of stability of sample of crystal form A**

| | | | |
|---|---|---|---|
| Illumination | 5 days | % Increase in total impurity | < 0.02 |
| | | % Maximum increase in single impurity | 0.03 |
| | 10 days | % Increase in total impurity | < 0.02 |
| | | % Maximum increase in single impurity | 0.05 |
| High temperature | 5 days | % Increase in total impurity | 0.12 |
| | | % Maximum increase in single impurity | 0.07 |
| | 10 days | % Increase in total impurity | < 0.02 |
| | | % Maximum increase in single impurity | 0.07 |
| High humidity | 5 days | % Increase in total impurity | < 0.02 |
| | | % Maximum increase in single impurity | 0.06 |
| | 10 days | % Increase in total impurity | < 0.02 |
| | | % Maximum increase in single impurity | 0.07 |
| High temperature and high humidity | 5 days | % Increase in total impurity | < 0.02 |
| | | % Maximum increase in single impurity | 0.03 |
| | 10 days | % Increase in total impurity | < 0.02 |
| | | % Maximum increase in single impurity | < 0.02 |
| Conclusion: After being placed under the conditions of illumination, high temperature and/or high humidity for 10 days, the free base crystal form A has no significant increase in the total amount of impurities and single impurities, indicating good stability. | | | |

**Example 8: Experimental study on hygroscopicity of sample of crystal form A**

The free base crystal form A of compound 3 was placed in saturated water vapor at different relative humidities to allow the compound to reach a dynamic equilibrium with the water vapor, and the percentage of weight gain after moisture absorption of the compound after equilibrium was calculated.

Conclusion: The free base crystal form A has a weight gain after moisture absorption of about 0.2720% under the condition of RH 80%, indicating weak hygroscopicity. After 2 cycles of moisture absorption and desorption under the

condition of 0-95% relative humidity, there is no change in the XRPD spectrum of the free base crystal form A, i.e., no crystal form transformation.

Finally, it should be noted that the above examples are only used to illustrate the technical solutions of the present invention and not to limit the present invention. Although the present invention has been described in detail with reference to the preferred examples, those of ordinary skill in the art should understand that modifications or equivalent substitutions may be made to the technical solutions of the present invention without departing from the spirit and scope of the technical solutions of the present invention, which shall be covered by the claims of the present invention.

## Claims

1. A crystal form or an acidic salt of a compound as represented by general formula (I):

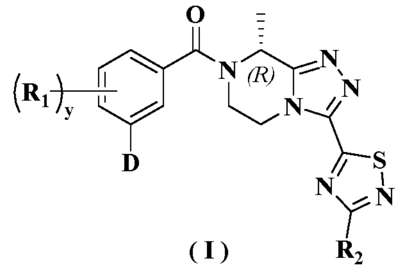

( I )

wherein:

$R_1$ is selected from hydrogen, deuterium or halogen; preferably hydrogen, deuterium, fluorine, chlorine or bromine;

$R_2$ is selected from hydrogen, deuterium, halogen, $C_{1-6}$ alkyl, $C_{1-6}$ deuteroalkyl, or 3- to 6-membered heterocyclyl; the $C_{1-6}$ alkyl, $C_{1-6}$ deuteroalkyl and 3- to 6-membered heterocyclyl are optionally further substituted by one or more substituents selected from deuterium and halogen; preferably hydrogen, deuterium, fluorine, chlorine, bromine, methyl, deuterated methyl, azetidinyl, tetrahydropyrrolyl,

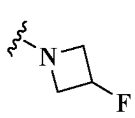 or 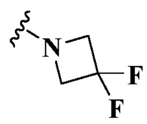 ;

y is 0, 1, 2, 3, 4 or 5.

2. The crystal form or acidic salt according to claim 1, wherein the compound has the following specific structure:

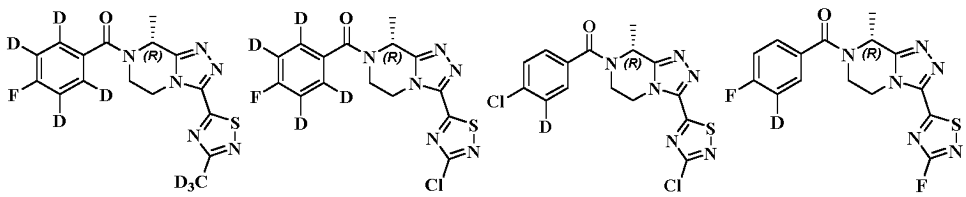

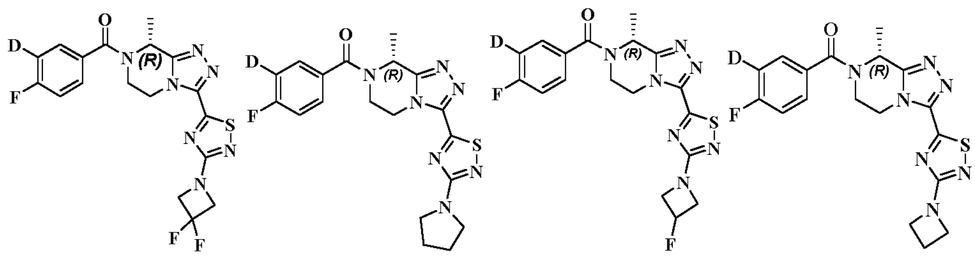

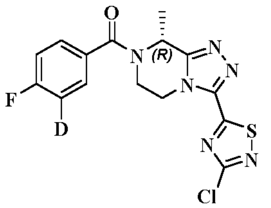

**3.** The crystal form according to claim 1 or 2, wherein the crystal form is a free base crystal form of compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d) methanone,

the X-ray powder diffraction pattern thereof has a diffraction peak at 2θ of 9.3 ± 0.2°, or has a diffraction peak at 2θ of 10.0 ± 0.2°, or has a diffraction peak at 2θ of 12.0 ± 0.2°, or has a diffraction peak at 2θ of 12.8 ± 0.2°, or has a diffraction peak at 2θ of 16.8 ± 0.2°, or has a diffraction peak at 2θ of 18.6 ± 0.2°, or has a diffraction peak at 2θ of 19.2 ± 0.2°, or has a diffraction peak at 2θ of 19.8 ± 0.2°, or has a diffraction peak at 2θ of 20.2 ± 0.2°, or has a diffraction peak at 2θ of 21.8 ± 0.2°, or has a diffraction peak at 2θ of 22.5 ± 0.2°, or has a diffraction peak at 2θ of 22.8 ± 0.2°, or has a diffraction peak at 2θ of 24.2 ± 0.2°, or has a diffraction peak at 2θ of 25.4 ± 0.2°, or has a diffraction peak at 2θ of 27.5 ± 0.2°, or has a diffraction peak at 2θ of 27.8 ± 0.2°, or has a diffraction peak at 2θ of 28.9 ± 0.2°; preferably, the X-ray powder diffraction pattern thereof optionally has a diffraction peak at 2, 4, 6, 8 or 10 of the above-mentioned 2θ;
preferably, the X-ray powder diffraction pattern thereof has at least a diffraction peak at one or more of 2θ of 16.8 ± 0.2°, 18.6 ± 0.2°, 19.9 ± 0.2° or 20.2 ± 0.2°; preferably, the X-ray powder diffraction pattern thereof has a diffraction peak at 2 to 4 of the above-mentioned 2θ, more preferably, at 3 or 4 of the above-mentioned 2θ, and most preferably, at 4 of the above-mentioned 2θ; optionally, the X-ray powder diffraction pattern thereof further has a diffraction peak at one or more of 2θ of 9.3 ± 0.2°, 10.0 ± 0.2°, 12.0 ± 0.2°, 12.8 ± 0.2° or 22.5 ± 0.2°, preferably at 2, 3, 4 or 5 of the above-mentioned 2θ; for example,
9.3 ± 0.2°, 10.0 ± 0.2°, 16.8 ± 0.2°, 18.6 ± 0.2°;
10.0 ± 0.2°, 12.0 ± 0.2°, 16.8 ± 0.2°, 18.6 ± 0.2°;
12.0 ± 0.2°, 12.8 ± 0.2°, 16.8 ± 0.2°, 18.6 ± 0.2°;
12.8 ± 0.2°, 22.5 ± 0.2°, 16.8 ± 0.2°, 18.6 ± 0.2°;
9.3 ± 0.2°, 10.0 ± 0.2°, 18.6 ± 0.2°, 19.8 ± 0.2°;
10.0 ± 0.2°, 12.0 ± 0.2°, 18.6 ± 0.2°, 19.8 ± 0.2°;
12.0 ± 0.2°, 12.8 ± 0.2°, 18.6 ± 0.2°, 19.8 ± 0.2°;
12.8 ± 0.2°, 22.5 ± 0.2°, 18.6 ± 0.2°, 19.8 ± 0.2°;
9.3 ± 0.2°, 10.0 ± 0.2°, 19.8 ± 0.2°, 20.2 ± 0.2°;
10.0 ± 0.2°, 12.0 ± 0.2°, 19.8 ± 0.2°, 20.2 ± 0.2°;
12.0 ± 0.2°, 12.8 ± 0.2°, 19.8 ± 0.2°, 20.2 ± 0.2°;
12.812.8 ± 0.2°, 22.5 ± 0.2°, 19.8 ± 0.2°, 20.2 ± 0.2°;
9.34 ± 0.2°, 10.0 ± 0.2°, 16.8 ± 0.2°, 18.6 ± 0.2°, 19.8 ± 0.2°;
10.0 ± 0.2°, 12.0 ± 0.2°, 16.8 ± 0.2°, 18.6 ± 0.2°, 19.8 ± 0.2°;
12.0 ± 0.2°, 12.8 ± 0.2°, 16.8 ± 0.2°, 18.6 ± 0.2°, 19.8 ± 0.2°;
12.8 ± 0.2°, 22.5 ± 0.2°, 16.8 ± 0.2°, 18.6 ± 0.2°, 19.8 ± 0.2°;
9.34 ± 0.2°, 10.0 ± 0.2°, 18.6 ± 0.2°, 19.8 ± 0.2°, 20.2 ± 0.2°;
10.0 ± 0.2°, 12.0 ± 0.2°, 18.6 ± 0.2°, 19.8 ± 0.2°, 20.2 ± 0.2°;
12.0 ± 0.2°, 12.8 ± 0.2°, 18.6 ± 0.2°, 19.8 ± 0.2°, 20.2 ± 0.2°;
12.8 ± 0.2°, 22.5 ± 0.2°, 18.6 ± 0.2°, 19.8 ± 0.2°, 20.2 ± 0.2°;
9.3 ± 0.2°, 10.0 ± 0.2°, 16.8 ± 0.2°, 18.6 ± 0.2°, 19.8 ± 0.2°, 20.2 ± 0.2°;
10.0 ± 0.2°, 12.0 ± 0.2°, 16.8 ± 0.2°, 18.6 ± 0.2°, 19.8 ± 0.2°, 20.2 ± 0.2°;
12.0 ± 0.2°, 12.8 ± 0.2°, 16.8 ± 0.2°, 18.6 ± 0.2°, 19.8 ± 0.2°, 20.2 ± 0.2°;
12.8 ± 0.2°, 22.5 ± 0.2°, 16.8 ± 0.2°, 18.6 ± 0.2°, 19.8 ± 0.2°, 20.2 ± 0.2°;
9.3 ± 0.2°, 10.0 ± 0.2°, 12.0 ± 0.2°, 16.8 ± 0.2°, 18.6 ± 0.2°, 19.8 ± 0.2°, 20.2 ± 0.2°;
10.0 ± 0.2°, 12.0 ± 0.2°, 12.8 ± 0.2°, 16.8 ± 0.2°, 18.6 ± 0.2°, 19.8 ± 0.2°, 20.2 ± 0.2°;
12.0 ± 0.2°, 12.8 ± 0.2°, 22.5 ± 0.2°, 16.8 ± 0.2°, 18.6 ± 0.2°, 19.8 ± 0.2°, 20.2 ± 0.2°;
9.3 ± 0.2°, 10.0 ± 0.2°, 12.0 ± 0.2°, 12.8 ± 0.2°, 16.8 ± 0.2°, 18.6 ± 0.2°, 19.8 ± 0.2°, 20.2 ± 0.2°;
10.0 ± 0.2°, 12.0 ± 0.2°, 12.8 ± 0.2°, 22.5 ± 0.2°, 16.8 ± 0.2°, 18.6 ± 0.2°, 19.8 ± 0.2°, 20.2 ± 0.2°;
9.3 ± 0.2°, 10.0 ± 0.2°, 12.0 ± 0.2°, 12.8 ± 0.2°, 22.5 ± 0.2°, 16.8 ± 0.2°, 18.6 ± 0.2°, 19.8 ± 0.2°, 20.2 ± 0.2°;
more preferably, the X-ray powder diffraction pattern thereof optionally has a diffraction peak at one or more of 2θ

of 15.8 ± 0.2°, 24.2 ± 0.2°, 25.4 ± 0.2°, 27.5 ± 0.2°, 27.8 ± 0.2° or 28.9 ± 0.2°; preferably, the X-ray powder diffraction pattern thereof has at least a diffraction peak at any 2 to 4 of the above-mentioned 2θ, or at any 5 or 6 of the above-mentioned 2θ, and further preferably, at any 4 or 6 of the above-mentioned 2θ; for example,

15.8 ± 0.2°, 24.2 ± 0.2°, 25.4 ± 0.2°, 27.5 ± 0.2°;
24.2 ± 0.2°, 25.4 ± 0.2°, 27.5 ± 0.2°, 27.8 ± 0.2°;
25.4 ± 0.2°, 27.5 ± 0.2°, 27.8 ± 0.2°, 28.9 ± 0.2°;
15.8 ± 0.2°, 24.2 ± 0.2°, 27.5 ± 0.2°, 27.8 ± 0.2°;
15.8 ± 0.2°, 24.2 ± 0.2°, 27.8 ± 0.2°, 28.9 ± 0.2°;
15.8 ± 0.2°, 24.2 ± 0.2°, 25.4 ± 0.2°, 28.9 ± 0.2°;
15.8 ± 0.2°, 24.2 ± 0.2°, 25.4 ± 0.2°, 27.5 ± 0.2°, 27.8 ± 0.2° or 28.9 ± 0.2°;

further preferably, the X-ray powder diffraction pattern thereof is basically as shown in FIG. 1, and the DSC pattern thereof is basically as shown in FIG. 7.

**4.** The acidic salt according to claim 1 or 2, wherein the acid in the acidic salt is selected from an inorganic acid or an organic acid, wherein the inorganic acid is selected from hydrochloric acid, sulfuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexane aminosulfonic acid, camphor sulfonic acid, aspartic acid, camphor acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, ethane-1,2-disulfonic acid, ethanesulfonic acid, formic acid, fumaric acid, galactonic acid, gentisuric acid, glutaric acid, 2-ketoglutaric acid, glycolic acid, benzoylglycine, hydroxyethyl sulfonic acid, lactobionic acid, ascorbic acid, aspartic acid, lauric acid, camphor acid, maleic acid, malonic acid, methanesulfonic acid, 1,5-naphthalene disulfonic acid, naphthalene-2-sulfonic acid, nicotinic acid, oleic acid, whey acid, oxalic acid, palmitic acid, pamoic acid, propanoic acid, salicylic acid, 4-aminosalicylic acid, sebacic acid, stearic acid, succinic acid, thiocyanic acid, pamoic acid, formic acid, undecylenoic acid, trifluoroacetic acid, benzenesulfonic acid, p-toluenesulfonic acid or L-malic acid; preferably hydrochloric acid, methanesulfonic acid, nitric acid, sulfuric acid or hydrobromic acid.

**5.** The acidic salt according to claim 4, wherein the acidic salt is an acidic salt of (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, preferably a crystal form of the acidic salt.

**6.** A crystal form of the acidic salt according to claim 5, wherein the crystal form is a hydrochloride crystal form, and the X-ray powder diffraction pattern thereof has a diffraction peak at 2θ of 8.3 ± 0.2°, or has a diffraction peak at 2θ of 10.1 ± 0.2°, or has a diffraction peak at 2θ of 15.0 ± 0.2°, or has a diffraction peak at 2θ of 16.5 ± 0.2°, or has a diffraction peak at 2θ of 16.0 ± 0.2°, or has a diffraction peak at 2θ of 17.0 ± 0.2°, or has a diffraction peak at 2θ of 17.7 ± 0.2°, or has a diffraction peak at 2θ of 21.5 ± 0.2°, or has a diffraction peak at 2θ of 22.0 ± 0.2°, or has a diffraction peak at 2θ of 24.4 ± 0.2°, or has a diffraction peak at 2θ of 24.7 ± 0.2°, or has a diffraction peak at 2θ of 27.4 ± 0.2°, or has a diffraction peak at 2θ of 30.9 ± 0.2°, or has a diffraction peak at 2θ of 32.2 ± 0.2°; preferably, the X-ray powder diffraction pattern thereof optionally has a diffraction peak at 2, 4, 6, 8 or 10 of the above-mentioned 2θ; preferably, the X-ray powder diffraction pattern thereof has at least a diffraction peak at one or more of 2θ of 8.3 ± 0.2°, 15.0 ± 0.2° or 16.5 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 10.11 ± 0.2°, 16.0 ± 0.2°, 17.0 ± 0.2°, 17.7 ± 0.2° or 21.5 ± 0.2°, preferably at 2, 3, 4 or 5 of the above-mentioned 2θ; for example,

8.3 ± 0.2°, 15.0 ± 0.2°, 10.1 ± 0.2°, 16.0 ± 0.2°;
8.3 ± 0.2°, 15.0 ± 0.2°, 10.1 ± 0.2°, 17.0 ± 0.2°;
8.3 ± 0.2°, 15.0 ± 0.2°, 10.1 ± 0.2°, 17.7 ± 0.2°;
8.3 ± 0.2°, 15.0 ± 0.2°, 10.1 ± 0.2°, 21.5 ± 0.2°;
8.3 ± 0.2°, 15.0 ± 0.2°, 16.0 ± 0.2°, 17.0 ± 0.2°;
8.3 ± 0.2°, 15.0 ± 0.2°, 16.0 ± 0.2°, 17.7 ± 0.2°;
8.3 ± 0.2°, 15.0 ± 0.2°, 16.0 ± 0.2°, 21.5 ± 0.2°;
8.3 ± 0.2°, 15.0 ± 0.2°, 17.0 ± 0.2°, 17.7 ± 0.2°;
8.3 ± 0.2°, 15.0 ± 0.2°, 17.0 ± 0.2°, 21.5 ± 0.2°;
8.3 ± 0.2°, 15.0 ± 0.2°, 16.5 ± 0.2°, 10.1 ± 0.2°, 16.0 ± 0.2°;
8.3 ± 0.2°, 15.0 ± 0.2°, 16.5 ± 0.2°, 10.1 ± 0.2°, 17.0 ± 0.2°;

8.3 ± 0.2°, 15.0 ± 0.2°, 16.5 ± 0.2°, 10.1 ± 0.2°, 17.7 ± 0.2°;
8.3 ± 0.2°, 15.0 ± 0.2°, 16.5 ± 0.2°, 10.1 ± 0.2°, 21.5 ± 0.2°;
8.3 ± 0.2°, 15.0 ± 0.2°, 16.5 ± 0.2°, 16.0 ± 0.2°, 17.0 ± 0.2°;
8.3 ± 0.2°, 15.0 ± 0.2°, 16.5 ± 0.2°, 16.0 ± 0.2°, 17.7 ± 0.2°;
8.3 ± 0.2°, 15.0 ± 0.2°, 16.5 ± 0.2°, 16.0 ± 0.2°, 21.5 ± 0.2°;
8.3 ± 0.2°, 15.0 ± 0.2°, 16.5 ± 0.2°, 17.0 ± 0.2°, 17.7 ± 0.2°;
8.3 ± 0.2°, 15.0 ± 0.2°, 16.5 ± 0.2°, 17.0 ± 0.2°, 21.5 ± 0.2°;
8.3 ± 0.2°, 16.5 ± 0.2°, 10.1 ± 0.2°, 16.0 ± 0.2°, 17.0 ± 0.2°;
8.3 ± 0.2°, 16.5 ± 0.2°, 10.1 ± 0.2°, 17.0 ± 0.2°, 17.7 ± 0.2°;
8.3 ± 0.2°, 16.5 ± 0.2°, 10.1 ± 0.2°, 17.7 ± 0.2°, 21.5 ± 0.2°;
8.3 ± 0.2°, 16.5 ± 0.2°, 10.1 ± 0.2°, 16.0 ± 0.2°, 21.5 ± 0.2°;
8.3 ± 0.2°, 16.5 ± 0.2°, 16.0 ± 0.2°, 17.0 ± 0.2°, 17.7 ± 0.2°;
8.3 ± 0.2°, 16.5 ± 0.2°, 16.0 ± 0.2°, 17.7 ± 0.2°, 21.5 ± 0.2°;
8.3 ± 0.2°, 16.5 ± 0.2°, 17.0 ± 0.2°, 17.7 ± 0.2°, 21.5 ± 0.2°;
15.0 ± 0.2°, 16.5 ± 0.2°, 10.1 ± 0.2°, 16.0 ± 0.2°, 17.0 ± 0.2°;
15.0 ± 0.2°, 16.5 ± 0.2°, 16.0 ± 0.2°, 17.0 ± 0.2°, 17.7 ± 0.2°;
15.0 ± 0.2°, 16.5 ± 0.2°, 17.0 ± 0.2°, 17.7 ± 0.2°, 21.5 ± 0.2°;
15.0 ± 0.2°, 16.5 ± 0.2°, 10.1 ± 0.2°, 17.0 ± 0.2°, 17.7 ± 0.2°;
15.0 ± 0.2°, 16.5 ± 0.2°, 10.1 ± 0.2°, 17.7 ± 0.2°, 21.5 ± 0.2°;
15.0 ± 0.2°, 16.5 ± 0.2°, 10.1 ± 0.2°, 16.0 ± 0.2°, 17.7 ± 0.2°;
15.0 ± 0.2°, 16.5 ± 0.2°, 10.1 ± 0.2°, 16.0 ± 0.2°, 21.5 ± 0.2°;
15.0 ± 0.2°, 16.5 ± 0.2°, 16.0 ± 0.2°, 17.7 ± 0.2°, 21.5 ± 0.2°;
8.3 ± 0.2°, 15.0 ± 0.2°, 16.5 ± 0.2°, 10.1 ± 0.2°, 16.0 ± 0.2°, 17.0 ± 0.2°;
8.3 ± 0.2°, 15.0 ± 0.2°, 16.5 ± 0.2°, 16.0 ± 0.2°, 17.0 ± 0.2°, 17.7 ± 0.2°;
8.3 ± 0.2°, 15.0 ± 0.2°, 16.5 ± 0.2°, 17.0 ± 0.2°, 17.7 ± 0.2°, 21.5 ± 0.2°;
8.3 ± 0.2°, 15.0 ± 0.2°, 16.5 ± 0.2°, 10.1 ± 0.2°, 17.0 ± 0.2°, 17.7 ± 0.2°;
8.3 ± 0.2°, 15.0 ± 0.2°, 16.5 ± 0.2°, 10.1 ± 0.2°, 17.7 ± 0.2°, 21.5 ± 0.2°;
8.3 ± 0.2°, 15.0 ± 0.2°, 16.5 ± 0.2°, 16.0 ± 0.2°, 17.7 ± 0.2°, 21.5 ± 0.2°;
8.3 ± 0.2°, 15.0 ± 0.2°, 16.5 ± 0.2°, 10.1 ± 0.2°, 16.0 ± 0.2°, 17.0 ± 0.2°, 17.7 ± 0.2°;
8.3 ± 0.2°, 15.0 ± 0.2°, 16.5 ± 0.2°, 16.0 ± 0.2°, 17.0 ± 0.2°, 17.7 ± 0.2°, 21.5 ± 0.2°;
8.3 ± 0.2°, 15.0 ± 0.2°, 16.5 ± 0.2°, 10.1 ± 0.2°, 17.0 ± 0.2°, 17.7 ± 0.2° or 21.5 ± 0.2°;
8.3 ± 0.2°, 15.0 ± 0.2°, 16.5 ± 0.2°, 10.1 ± 0.2°, 16.0 ± 0.2°, 17.7 ± 0.2°, 21.5 ± 0.2°;
8.3 ± 0.2°, 15.0 ± 0.2°, 16.5 ± 0.2°, 10.1 ± 0.2°, 16.0 ± 0.2°, 17.0 ± 0.2°, 21.5 ± 0.2°;
more preferably, the X-ray powder diffraction pattern thereof optionally has a diffraction peak at one or more of 2θ of 24.4 ± 0.2°, 24.7 ± 0.2°, 25.2 ± 0.2°, 27.4 ± 0.2°, 28.5 ± 0.2°, 30.2 ± 0.2° or 32.2 ± 0.2°; preferably, the X-ray powder diffraction pattern thereof has at least a diffraction peak at any 2 to 4 of the above-mentioned 2θ, or at any 5 or 6 of the above-mentioned 2θ, and further preferably, at any 4 or 6 of the above-mentioned 2θ; for example,
24.4 ± 0.2°, 24.7 ± 0.2°, 25.2 ± 0.2°, 27.4 ± 0.2°;
24.4 ± 0.2°, 25.2 ± 0.2°, 27.4 ± 0.2°, 28.5 ± 0.2°;
24.4 ± 0.2°, 27.4 ± 0.2°, 28.5 ± 0.2°, 30.2 ± 0.2°;
24.4 ± 0.2°, 28.5 ± 0.2°, 30.2 ± 0.2°, 32.2 ± 0.2°;
24.7 ± 0.2°, 25.2 ± 0.2°, 27.4 ± 0.2°, 28.5 ± 0.2°;
24.7 ± 0.2°, 27.4 ± 0.2°, 28.5 ± 0.2°, 30.2 ± 0.2°;
24.7 ± 0.2°, 28.5 ± 0.2°, 30.2 ± 0.2°, 32.2 ± 0.2°;
25.2 ± 0.2°, 27.4 ± 0.2°, 28.5 ± 0.2°, 30.2 ± 0.2°;
25.2 ± 0.2°, 28.5 ± 0.2°, 30.2 ± 0.2°, 32.2 ± 0.2°;
27.4 ± 0.2°, 28.5 ± 0.2°, 30.2 ± 0.2°, 32.2 ± 0.2°;
24.4 ± 0.2°, 24.7 ± 0.2°, 27.4 ± 0.2°, 28.5 ± 0.2°;
24.4 ± 0.2°, 24.7 ± 0.2°, 28.5 ± 0.2°, 30.2 ± 0.2°;
24.4 ± 0.2°, 24.7 ± 0.2°, 30.2 ± 0.2°, 32.2 ± 0.2°;
24.4 ± 0.2°, 24.7 ± 0.2°, 25.2 ± 0.2°, 27.4 ± 0.2°, 28.5 ± 0.2°, 30.2 ± 0.2°;
24.7 ± 0.2°, 25.2 ± 0.2°, 27.4 ± 0.2°, 28.5 ± 0.2°, 30.2 ± 0.2°, 32.2 ± 0.2°;
24.4 ± 0.2°, 25.2 ± 0.2°, 27.4 ± 0.2°, 28.5 ± 0.2°, 30.2 ± 0.2°, 32.2 ± 0.2°;
24.4 ± 0.2°, 24.7 ± 0.2°, 27.4 ± 0.2°, 28.5 ± 0.2°, 30.2 ± 0.2°, 32.2 ± 0.2°;
24.4 ± 0.2°, 24.7 ± 0.2°, 25.2 ± 0.2°, 28.5 ± 0.2°, 30.2 ± 0.2°, 32.2 ± 0.2°;
24.4 ± 0.2°, 24.7 ± 0.2°, 25.2 ± 0.2°, 27.4 ± 0.2°, 30.2 ± 0.2°, 32.2 ± 0.2°;
24.4 ± 0.2°, 24.7 ± 0.2°, 25.2 ± 0.2°, 27.4 ± 0.2°, 28.5 ± 0.2°, 32.2 ± 0.2°;
further preferably, the X-ray powder diffraction pattern thereof is basically as shown in FIG. 2.

**7.** A crystal form of the acidic salt according to claim 5, wherein the crystal form is a methanesulfonate crystal form, and the X-ray powder diffraction pattern thereof has a diffraction peak at 2θ of 7.1 ± 0.2°, or has a diffraction peak at 2θ of 10.1 ± 0.2°, or has a diffraction peak at 2θ of 10.5 ± 0.2°, or has a diffraction peak at 2θ of 12.0 ± 0.2°, or has a diffraction peak at 2θ of 13.2 ± 0.2°, or has a diffraction peak at 2θ of 16.9 ± 0.2°, or has a diffraction peak at 2θ of 18.7 ± 0.2°, or has a diffraction peak at 2θ of 21.1 ± 0.2°, or has a diffraction peak at 2θ of 16.8 ± 0.2°, or has a diffraction peak at 2θ of 18.6 ± 0.2°, or has a diffraction peak at 2θ of 19.7 ± 0.2°, or has a diffraction peak at 2θ of 21.5 ± 0.2°, or has a diffraction peak at 2θ of 21.7 ± 0.2°, or has a diffraction peak at 2θ of 22.5 ± 0.2°, or has a diffraction peak at 2θ of 23.3 ± 0.2°, or has a diffraction peak at 2θ of 25.5 ± 0.2°, or has a diffraction peak at 2θ of 28.0 ± 0.2°; preferably, the X-ray powder diffraction pattern thereof optionally has a diffraction peak at 2, 4, 6, 8 or 10 of the above-mentioned 2θ;

preferably, the X-ray powder diffraction pattern thereof has at least a diffraction peak at one or more of 2θ of 7.1 ± 0.2°, 10.1 ± 0.2° or 10.5 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 12.0 ± 0.2°, 13.2 ± 0.2°, 16.9 ± 0.2°, 18.7 ± 0.2° or 21.1 ± 0.2°, preferably at 2, 3, 4 or 5 of the above-mentioned 2θ; for example,
7.1 ± 0.2°, 10.1 ± 0.2°, 12.0 ± 0.2°, 13.2 ± 0.2°;
7.1 ± 0.2°, 10.1 ± 0.2°, 13.2 ± 0.2°, 16.9 ± 0.2°;
7.1 ± 0.2°, 10.1 ± 0.2°, 16.9 ± 0.2°, 18.7 ± 0.2°;
7.1 ± 0.2°, 10.1 ± 0.2°, 18.7 ± 0.2°, 21.1 ± 0.2°;
7.1 ± 0.2°, 10.1 ± 0.2°, 12.0 ± 0.2°, 16.9 ± 0.2°;
7.1±0.2°, 10.1 ± 0.2°, 12.0 ± 0.2°, 18.7 ± 0.2°;
7.1 ± 0.2°, 10.1 ± 0.2°, 12.0 ± 0.2°, 21.1 ± 0.2°;
7.1 ± 0.2°, 10.1 ± 0.2°, 13.2 ± 0.2°, 18.7 ± 0.2°;
7.1 ± 0.2°, 10.1 ± 0.2°, 13.2 ± 0.2°, 21.1 ± 0.2°;
7.1 ± 0.2°, 10.1 ± 0.2°, 16.9 ± 0.2°, 21.1 ± 0.2°;
7.1 ± 0.2°, 10.1 ± 0.2°, 10.5 ± 0.2°, 12.0 ± 0.2°, 13.2 ± 0.2°;
7.1 ± 0.2°, 10.1 ± 0.2°, 10.5 ± 0.2°, 13.2 ± 0.2°, 16.9 ± 0.2°;
7.1 ± 0.2°, 10.1 ± 0.2°, 10.5 ± 0.2°, 16.9 ± 0.2°, 18.7 ± 0.2°;
7.1 ± 0.2°, 10.1 ± 0.2°, 10.5 ± 0.2°, 18.7 ± 0.2°, 21.1 ± 0.2°;
7.1 ± 0.2°, 10.1 ± 0.2°, 10.5 ± 0.2°, 12.0 ± 0.2°, 16.9 ± 0.2°;
7.1 ± 0.2°, 10.1 ± 0.2°, 10.5 ± 0.2°, 12.0 ± 0.2°, 18.7 ± 0.2°;
7.1 ± 0.2°, 10.1 ± 0.2°, 10.5 ± 0.2°, 12.0 ± 0.2°, 21.1 ± 0.2°;
7.1 ± 0.2°, 10.1 ± 0.2°, 10.5 ± 0.2°, 13.2 ± 0.2°, 18.7 ± 0.2°;
7.1 ± 0.2°, 10.1 ± 0.2°, 10.5 ± 0.2°, 13.2 ± 0.2°, 21.1 ± 0.2°;
7.1 ± 0.2°, 10.1 ± 0.2°, 10.5 ± 0.2°, 16.9 ± 0.2°, 21.1 ± 0.2°;
7.1 ± 0.2°, 10.1 ± 0.2°, 10.5 ± 0.2°, 12.0 ± 0.2°, 13.2 ± 0.2°, 16.9 ± 0.2°;
7.1 ± 0.2°, 10.1 ± 0.2°, 10.5 ± 0.2°, 13.2 ± 0.2°, 16.9 ± 0.2°, 18.7 ± 0.2°;
7.1 ± 0.2°, 10.1 ± 0.2°, 10.5 ± 0.2°, 16.9 ± 0.2°, 18.7 ± 0.2°, 21.1 ± 0.2°;
7.1 ± 0.2°, 10.1 ± 0.2°, 10.5 ± 0.2°, 12.0 ± 0.2°, 16.9 ± 0.2°, 18.7 ± 0.2°;
7.1 ± 0.2°, 10.1 ± 0.2°, 10.5 ± 0.2°, 12.0 ± 0.2°, 18.7 ± 0.2°, 21.1 ± 0.2°;
7.1 ± 0.2°, 10.1 ± 0.2°, 10.5 ± 0.2°, 13.2 ± 0.2°, 18.7 ± 0.2°, 21.1 ± 0.2°;
7.1 ± 0.2°, 10.1 ± 0.2°, 10.5 ± 0.2°, 12.0 ± 0.2°, 13.2 ± 0.2°, 16.9 ± 0.2°, 18.7 ± 0.2°;
7.1 ± 0.2°, 10.1 ± 0.2°, 10.5 ± 0.2°, 13.2 ± 0.2°, 16.9 ± 0.2°, 18.7 ± 0.2°, 21.1 ± 0.2°;
7.1 ± 0.2°, 10.1 ± 0.2°, 10.5 ± 0.2°, 12.0 ± 0.2°, 16.9 ± 0.2°, 18.7 ± 0.2°, 21.1 ± 0.2°;
7.1 ± 0.2°, 10.1 ± 0.2°, 10.5 ± 0.2°, 12.0 ± 0.2°, 13.2 ± 0.2°, 18.7 ± 0.2°, 21.1 ± 0.2°;
7.1 ± 0.2°, 10.1 ± 0.2°, 10.5 ± 0.2°, 12.0 ± 0.2°, 13.2 ± 0.2°, 16.9 ± 0.2°, 18.7 ± 0.2°, 21.1 ± 0.2°;
more preferably, the X-ray powder diffraction pattern thereof optionally has a diffraction peak at one or more of 2θ of 16.8 ± 0.2°, 18.6 ± 0.2°, 19.7 ± 0.2°, 21.5 ± 0.2°, 21.7 ± 0.2°, 22.5 ± 0.2°, 23.3 ± 0.2°, 25.5 ± 0.2° or 28.0 ± 0.2°; preferably, the X-ray powder diffraction pattern thereof has at least a diffraction peak at any 2 to 4 of the above-mentioned 2θ, or at any 5 or 6 of the above-mentioned 2θ, or at any 7 to 9 of the above-mentioned 2θ, and further preferably, at any 4, 6 or 8 of the above-mentioned 2θ; for example,
16.8 ± 0.2°, 18.6 ± 0.2°, 19.7 ± 0.2°, 21.5 ± 0.2°;
18.6 ± 0.2°, 19.7 ± 0.2°, 21.5 ± 0.2°, 21.7 ± 0.2°;
19.7 ± 0.2°, 21.5 ± 0.2°, 21.7 ± 0.2°, 22.5 ± 0.2°;
21.5 ± 0.2°, 21.7 ± 0.2°, 22.5 ± 0.2°, 23.3 ± 0.2°;
21.7 ± 0.2°, 22.5 ± 0.2°, 23.3 ± 0.2°, 25.5 ± 0.2°;
22.5 ± 0.2°, 23.3 ± 0.2°, 25.5 ± 0.2°, 28.0 ± 0.2°;
16.8 ± 0.2°, 19.7 ± 0.2°, 21.5 ± 0.2°, 21.7 ± 0.2°;

16.8 ± 0.2°, 21.5 ± 0.2°, 21.7 ± 0.2°, 22.5 ± 0.2°;
16.8 ± 0.2°, 21.7 ± 0.2°, 22.5 ± 0.2°, 23.3 ± 0.2°;
16.8 ± 0.2°, 22.5 ± 0.2°, 23.3 ± 0.2°, 25.5 ± 0.2°;
16.8 ± 0.2°, 23.3 ± 0.2°, 25.5 ± 0.2°, 28.0 ± 0.2°;
16.8 ± 0.2°, 18.6 ± 0.2°, 21.5 ± 0.2°, 21.7 ± 0.2°;
16.8 ± 0.2°, 18.6 ± 0.2°, 21.7 ± 0.2°, 22.5 ± 0.2°;
16.8 ± 0.2°, 18.6 ± 0.2°, 22.5 ± 0.2°, 23.3 ± 0.2°;
16.8 ± 0.2°, 18.6 ± 0.2°, 23.3 ± 0.2°, 25.5 ± 0.2°;
16.8 ± 0.2°, 18.6 ± 0.2°, 25.5 ± 0.2°, 28.0 ± 0.2°;
16.8 ± 0.2°, 18.6 ± 0.2°, 19.7 ± 0.2°, 21.7 ± 0.2°;
16.8 ± 0.2°, 18.6 ± 0.2°, 19.7 ± 0.2°, 22.5 ± 0.2°;
16.8 ± 0.2°, 18.6 ± 0.2°, 19.7 ± 0.2°, 23.3 ± 0.2°;
16.8 ± 0.2°, 18.6 ± 0.2°, 19.7 ± 0.2°, 25.5 ± 0.2°;
16.8 ± 0.2°, 18.6 ± 0.2°, 19.7 ± 0.2°, 28.0 ± 0.2°;
18.6 ± 0.2°, 19.7 ± 0.2°, 21.7 ± 0.2°, 22.5 ± 0.2°;
18.6 ± 0.2°, 19.7 ± 0.2°, 22.5 ± 0.2°, 23.3 ± 0.2°;
18.6 ± 0.2°, 19.7 ± 0.2°, 23.3 ± 0.2°, 25.5 ± 0.2°;
18.6 ± 0.2°, 19.7 ± 0.2°, 25.5 ± 0.2°, 28.0 ± 0.2°;
19.7 ± 0.2°, 21.5 ± 0.2°, 22.5 ± 0.2°, 23.3 ± 0.2°;
19.7 ± 0.2°, 21.5 ± 0.2°, 23.3 ± 0.2°, 25.5 ± 0.2°;
19.7 ± 0.2°, 21.5 ± 0.2°, 25.5 ± 0.2°, 28.0 ± 0.2°;
21.5 ± 0.2°, 21.7 ± 0.2°, 23.3 ± 0.2°, 25.5 ± 0.2°;
21.5 ± 0.2°, 21.7 ± 0.2°, 25.5 ± 0.2°, 28.0 ± 0.2°;
16.8 ± 0.2°, 18.6 ± 0.2°, 19.7 ± 0.2°, 21.5 ± 0.2°, 21.7 ± 0.2°, 22.5 ± 0.2°;
18.6 ± 0.2°, 19.7 ± 0.2°, 21.5 ± 0.2°, 21.7 ± 0.2°, 22.5 ± 0.2°, 23.3 ± 0.2°;
19.7 ± 0.2°, 21.5 ± 0.2°, 21.7 ± 0.2°, 22.5 ± 0.2°, 23.3 ± 0.2°, 25.5 ± 0.2°;
21.5 ± 0.2°, 21.7 ± 0.2°, 22.5 ± 0.2°, 23.3 ± 0.2°, 25.5 ± 0.2°, 28.0 ± 0.2°;
16.8 ± 0.2°, 19.7 ± 0.2°, 21.5 ± 0.2°, 21.7 ± 0.2°, 22.5 ± 0.2°, 23.3 ± 0.2°;
16.8 ± 0.2°, 21.5 ± 0.2°, 21.7 ± 0.2°, 22.5 ± 0.2°, 23.3 ± 0.2°, 25.5 ± 0.2°;
16.8 ± 0.2°, 21.7 ± 0.2°, 22.5 ± 0.2°, 23.3 ± 0.2°, 25.5 ± 0.2°, 28.0 ± 0.2°;
16.8 ± 0.2°, 18.6 ± 0.2°, 21.5 ± 0.2°, 21.7 ± 0.2°, 22.5 ± 0.2°, 23.3 ± 0.2°;
16.8 ± 0.2°, 18.6 ± 0.2°, 21.7 ± 0.2°, 22.5 ± 0.2°, 23.3 ± 0.2°, 25.5 ± 0.2°;
16.8 ± 0.2°, 18.6 ± 0.2°, 22.5 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.7 ± 0.2°, 22.5 ± 0.2°, 23.3 ± 0.2°;
16.8 ± 0.2°, 18.6 ± 0.2°, 19.7 ± 0.2°, 22.5 ± 0.2°, 23.3 ± 0.2°, 25.5 ± 0.2°;
16.8 ± 0.2°, 18.6 ± 0.2°, 19.7 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.5 ± 0.2°, 22.5 ± 0.2°, 23.3 ± 0.2°;
16.8 ± 0.2°, 18.6 ± 0.2°, 19.7 ± 0.2°, 21.5 ± 0.2°, 23.3 ± 0.2°, 25.5 ± 0.2°;
16.8 ± 0.2°, 18.6 ± 0.2°, 19.7 ± 0.2°, 21.5 ± 0.2°, 25.5 ± 0.2°, 28.0 ± 0.2°;
16.8 ± 0.2°, 18.6 ± 0.2°, 19.7 ± 0.2°, 21.5 ± 0.2°, 21.7 ± 0.2°, 23.3 ± 0.2°;
16.8 ± 0.2°, 18.6 ± 0.2°, 19.7 ± 0.2°, 21.5 ± 0.2°, 21.7 ± 0.2°, 25.5 ± 0.2°;
16.8 ± 0.2°, 18.6 ± 0.2°, 19.7 ± 0.2°, 21.5 ± 0.2°, 21.7 ± 0.2°, 28.0 ± 0.2°;
18.6 ± 0.2°, 19.7 ± 0.2°, 21.7 ± 0.2°, 22.5 ± 0.2°, 23.3 ± 0.2°, 25.5 ± 0.2°;
18.6 ± 0.2°, 19.7 ± 0.2°, 22.5 ± 0.2°, 23.3 ± 0.2°, 25.5 ± 0.2°, 28.0 ± 0.2°;
19.7 ± 0.2°, 21.5 ± 0.2°, 22.5 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.5 ± 0.2°, 21.7 ± 0.2°, 22.5 ± 0.2°, 23.3 ± 0.2°, 25.5 ± 0.2°;
18.6 ± 0.2°, 19.7 ± 0.2°, 21.5 ± 0.2°, 21.7 ± 0.2°, 22.5 ± 0.2°, 23.3 ± 0.2°, 25.5 ± 0.2°, 28.0 ± 0.2°;
16.8 ± 0.2°, 18.6 ± 0.2°, 21.5 ± 0.2°, 21.7 ± 0.2°, 22.5 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.7 ± 0.2°, 22.5 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.5 ± 0.2°, 22.5 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.5 ± 0.2°, 21.7 ± 0.2°, 23.3 ± 0.2°, 25.5 ± 0.2°, 28.0 ± 0.2°;
16.8 ± 0.2°, 18.6 ± 0.2°, 19.7 ± 0.2°, 21.5 ± 0.2°, 21.7 ± 0.2°, 22.5 ± 0.2°, 25.5 ± 0.2°, 28.0 ± 0.2°;
further preferably, the X-ray powder diffraction pattern thereof is basically as shown in FIG. 3.

**8.** A crystal form of the acidic salt according to claim 5, wherein the crystal form is a nitrate crystal form, and the X-ray powder diffraction pattern thereof has a diffraction peak at 2θ of 9.3 ± 0.2°, or has a diffraction peak at 2θ of 11.9 ± 0.2°, or has a diffraction peak at 2θ of 12.5 ± 0.2°, or has a diffraction peak at 2θ of 14.3 ± 0.2°, or has a diffraction peak at 2θ of 14.6 ± 0.2°, or has a diffraction peak at 2θ of 16.2 ± 0.2°, or has a diffraction peak at 2θ of 16.7 ± 0.2°, or has a

diffraction peak at 2θ of 17.9 ± 0.2°, or has a diffraction peak at 2θ of 18.7 ± 0.2°, or has a diffraction peak at 2θ of 22.9 ± 0.2°, or has a diffraction peak at 2θ of 25.1 ± 0.2°, or has a diffraction peak at 2θ of 25.9 ± 0.2°, or has a diffraction peak at 2θ of 26.6 ± 0.2°, or has a diffraction peak at 2θ of 28.2 ± 0.2°, or has a diffraction peak at 2θ of 28.6 ± 0.2°; preferably, the X-ray powder diffraction pattern thereof optionally has a diffraction peak at 2, 4, 6, 8 or 10 of the above-mentioned 2θ;

preferably, the X-ray powder diffraction pattern thereof has at least a diffraction peak at one or more of 2θ of 9.3 ± 0.2°, 11.9 ± 0.2° or 12.5 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 14.3 ± 0.2°, 14.6 ± 0.2°, 16.2 ± 0.2°, 16.7 ± 0.2° or 17.9 ± 0.2°, preferably at 2, 3, 4 or 5 of the above-mentioned 2θ; for example,
9.3 ± 0.2°, 11.9 ± 0.2°, 14.3 ± 0.2°, 14.6 ± 0.2°;
9.3 ± 0.2°, 11.9 ± 0.2°, 14.6 ± 0.2°, 16.2 ± 0.2°;
9.3 ± 0.2°, 11.9 ± 0.2°, 16.2 ± 0.2°, 16.7 ± 0.2°;
9.3 ± 0.2°, 11.9 ± 0.2°, 16.7 ± 0.2°, 17.9 ± 0.2°;
9.3 ± 0.2°, 11.9 ± 0.2°, 14.3 ± 0.2°, 16.2 ± 0.2°;
9.3 ± 0.2°, 11.9 ± 0.2°, 14.3 ± 0.2°, 16.7 ± 0.2°;
9.3 ± 0.2°, 11.9 ± 0.2°, 14.3 ± 0.2°, 17.9 ± 0.2°;
9.3 ± 0.2°, 11.9 ± 0.2°, 14.6 ± 0.2°, 16.7 ± 0.2°;
9.3 ± 0.2°, 11.9 ± 0.2°, 14.6 ± 0.2°, 17.9 ± 0.2°;
9.3 ± 0.2°, 11.9 ± 0.2°, 16.2 ± 0.2°, 17.9 ± 0.2°;
9.3 ± 0.2°, 11.9 ± 0.2°, 12.5 ± 0.2°, 14.3 ± 0.2°, 14.6 ± 0.2°;
9.3 ± 0.2°, 11.9 ± 0.2°, 12.5 ± 0.2°, 14.6 ± 0.2°, 16.2 ± 0.2°;
9.3 ± 0.2°, 11.9 ± 0.2°, 12.5 ± 0.2°, 16.2 ± 0.2°, 16.7 ± 0.2°;
9.3 ± 0.2°, 11.9 ± 0.2°, 12.5 ± 0.2°, 16.7 ± 0.2°, 17.9 ± 0.2°;
9.3 ± 0.2°, 11.9 ± 0.2°, 12.5 ± 0.2°, 14.3 ± 0.2°, 16.2 ± 0.2°;
9.3 ± 0.2°, 11.9 ± 0.2°, 12.5 ± 0.2°, 14.3 ± 0.2°, 16.7 ± 0.2°;
9.3 ± 0.2°, 11.9 ± 0.2°, 12.5 ± 0.2°, 14.3 ± 0.2°, 17.9 ± 0.2°;
9.3 ± 0.2°, 11.9 ± 0.2°, 12.5 ± 0.2°, 14.6 ± 0.2°, 16.7 ± 0.2°;
9.3 ± 0.2°, 11.9 ± 0.2°, 12.5 ± 0.2°, 14.6 ± 0.2°, 17.9 ± 0.2°;
9.3 ± 0.2°, 11.9 ± 0.2°, 12.5 ± 0.2°, 16.2 ± 0.2°, 17.9 ± 0.2°;
9.3 ± 0.2°, 11.9 ± 0.2°, 12.5 ± 0.2°, 14.3 ± 0.2°, 14.6 ± 0.2°, 16.2 ± 0.2°;
9.3 ± 0.2°, 11.9 ± 0.2°, 12.5 ± 0.2°, 14.6 ± 0.2°, 16.2 ± 0.2°, 16.7 ± 0.2°;
9.3 ± 0.2°, 11.9 ± 0.2°, 12.5 ± 0.2°, 16.2 ± 0.2°, 16.7 ± 0.2°, 17.9 ± 0.2°;
9.3 ± 0.2°, 11.9 ± 0.2°, 12.5 ± 0.2°, 14.3 ± 0.2°, 16.2 ± 0.2°, 16.7 ± 0.2°;
9.3 ± 0.2°, 11.9 ± 0.2°, 12.5 ± 0.2°, 14.3 ± 0.2°, 16.7 ± 0.2°, 17.9 ± 0.2°;
9.3 ± 0.2°, 11.9 ± 0.2°, 12.5 ± 0.2°, 14.6 ± 0.2°, 16.7 ± 0.2°, 17.9 ± 0.2°;
9.3 ± 0.2°, 11.9 ± 0.2°, 12.5 ± 0.2°, 14.3 ± 0.2°, 14.6 ± 0.2°, 16.7 ± 0.2°;
9.3 ± 0.2°, 11.9 ± 0.2°, 12.5 ± 0.2°, 14.3 ± 0.2°, 14.6 ± 0.2°, 17.9 ± 0.2°;
9.3 ± 0.2°, 11.9 ± 0.2°, 12.5 ± 0.2°, 14.3 ± 0.2°, 14.6 ± 0.2°, 16.2 ± 0.2°, 16.7 ± 0.2°;
9.3 ± 0.2°, 11.9 ± 0.2°, 12.5 ± 0.2°, 14.6 ± 0.2°, 16.2 ± 0.2°, 16.7 ± 0.2°, 17.9 ± 0.2°;
9.3 ± 0.2°, 11.9 ± 0.2°, 12.5 ± 0.2°, 14.3 ± 0.2°, 16.2 ± 0.2°, 16.7 ± 0.2°, 17.9 ± 0.2°;
9.3 ± 0.2°, 11.9 ± 0.2°, 12.5 ± 0.2°, 14.3 ± 0.2°, 14.6 ± 0.2°, 16.7 ± 0.2° or 17.9 ± 0.2°;
9.3 ± 0.2°, 11.9 ± 0.2°, 12.5 ± 0.2°, 14.3 ± 0.2°, 14.6 ± 0.2°, 16.2 ± 0.2°, 17.9 ± 0.2°;
9.3 ± 0.2°, 11.9 ± 0.2°, 12.5 ± 0.2°, 14.3 ± 0.2°, 14.6 ± 0.2°, 16.2 ± 0.2°, 16.7 ± 0.2°, 17.9 ± 0.2°;
more preferably, the X-ray powder diffraction pattern thereof optionally has a diffraction peak at one or more of 2θ of 18.7 ± 0.2°, 22.9 ± 0.2°, 25.1 ± 0.2°, 25.9 ± 0.2°, 26.6 ± 0.2°, 28.2 ± 0.2° or 28.6 ± 0.2°; preferably, the X-ray powder diffraction pattern thereof has at least a diffraction peak at any 2 to 4 of the above-mentioned 2θ, or at any 5 to 7 of the above-mentioned 2θ, and further preferably, at any 4 or 6 of the above-mentioned 2θ; for example,
18.7 ± 0.2°, 22.9 ± 0.2°, 25.1 ± 0.2°, 25.9 ± 0.2°;
18.7 ± 0.2°, 25.1 ± 0.2°, 25.9 ± 0.2°, 26.6 ± 0.2°;
18.7 ± 0.2°, 25.9 ± 0.2°, 26.6 ± 0.2°, 28.2 ± 0.2°;
18.7 ± 0.2°, 26.6 ± 0.2°, 28.2 ± 0.2°, 28.6 ± 0.2°;
18.7 ± 0.2°, 22.9 ± 0.2°, 25.9 ± 0.2°, 26.6 ± 0.2°;
18.7 ± 0.2°, 22.9 ± 0.2°, 26.6 ± 0.2°, 28.2 ± 0.2°;
18.7 ± 0.2°, 22.9 ± 0.2°, 28.2 ± 0.2°, 28.6 ± 0.2°;
18.7 ± 0.2°, 22.9 ± 0.2°, 25.1 ± 0.2°, 26.6 ± 0.2°;
18.7 ± 0.2°, 22.9 ± 0.2°, 25.1 ± 0.2°, 28.2 ± 0.2°;

18.7 ± 0.2°, 22.9 ± 0.2°, 25.1 ± 0.2°, 28.6 ± 0.2°;
22.9 ± 0.2°, 25.1 ± 0.2°, 25.9 ± 0.2°, 26.6 ± 0.2°;
22.9 ± 0.2°, 25.9 ± 0.2°, 26.6 ± 0.2°, 28.2 ± 0.2°;
22.9 ± 0.2°, 26.6 ± 0.2°, 28.2 ± 0.2°, 28.6 ± 0.2°;
25.1 ± 0.2°, 25.9 ± 0.2°, 26.6 ± 0.2°, 28.2 ± 0.2°;
25.1 ± 0.2°, 26.6 ± 0.2°, 28.2 ± 0.2°, 28.6 ± 0.2°;
25.9 ± 0.2°, 26.6 ± 0.2°, 28.2 ± 0.2°, 28.6 ± 0.2°;
18.7 ± 0.2°, 22.9 ± 0.2°, 25.1 ± 0.2°, 25.9 ± 0.2°, 26.6 ± 0.2°, 28.2 ± 0.2°;
22.9 ± 0.2°, 25.1 ± 0.2°, 25.9 ± 0.2°, 26.6 ± 0.2°, 28.2 ± 0.2°, 28.6 ± 0.2°;
18.7 ± 0.2°, 25.1 ± 0.2°, 25.9 ± 0.2°, 26.6 ± 0.2°, 28.2 ± 0.2°, 28.6 ± 0.2°;
18.7 ± 0.2°, 22.9 ± 0.2°, 25.9 ± 0.2°, 26.6 ± 0.2°, 28.2 ± 0.2°, 28.6 ± 0.2°;
18.7 ± 0.2°, 22.9 ± 0.2°, 25.1 ± 0.2°, 26.6 ± 0.2°, 28.2 ± 0.2°, 28.6 ± 0.2°;
18.7 ± 0.2°, 22.9 ± 0.2°, 25.1 ± 0.2°, 25.9 ± 0.2°, 28.2 ± 0.2°, 28.6 ± 0.2°;
18.7 ± 0.2°, 22.9 ± 0.2°, 25.1 ± 0.2°, 25.9 ± 0.2°, 26.6 ± 0.2°, 28.6 ± 0.2°;
further preferably, the X-ray powder diffraction pattern thereof is basically as shown in FIG. 4.

**9.** A crystal form of the acidic salt according to claim 5, wherein the crystal form is a sulfate crystal form, and the X-ray powder diffraction pattern thereof has a diffraction peak at 2θ of 7.1 ± 0.2°, or has a diffraction peak at 2θ of 13.4 ± 0.2°, or has a diffraction peak at 2θ of 14.2 ± 0.2°, or has a diffraction peak at 2θ of 15.5 ± 0.2°, or has a diffraction peak at 2θ of 16.3 ± 0.2°, or has a diffraction peak at 2θ of 17.4 ± 0.2°, or has a diffraction peak at 2θ of 17.7°, or has a diffraction peak at 2θ of 18.0 ± 0.2°, or has a diffraction peak at 2θ of 19.8 ± 0.2°, or has a diffraction peak at 2θ of 20.1 ± 0.2°; preferably, the X-ray powder diffraction pattern thereof optionally has a diffraction peak at 2, 4, 6, 8 or 10 of the above-mentioned 2θ;

preferably, the X-ray powder diffraction pattern thereof has at least a diffraction peak at one or more of 2θ of 7.1 ± 0.2°, 16.3 ± 0.2° or 18.0 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 13.4 ± 0.2°, 14.2 ± 0.2°, 15.5 ± 0.2°, 17.4 ± 0.2° or 17.7 ± 0.2°, preferably at 2, 3, 4 or 5 of the above-mentioned 2θ; for example,
7.1 ± 0.2°, 16.3 ± 0.2°, 13.4 ± 0.2°, 14.2 ± 0.2°;
7.1 ± 0.2°, 16.3 ± 0.2°, 14.2 ± 0.2°, 15.5 ± 0.2°;
7.1 ± 0.2°, 16.3 ± 0.2°, 15.5 ± 0.2°, 17.4 ± 0.2°;
7.1 ± 0.2°, 16.3 ± 0.2°, 17.4 ± 0.2°, 17.7 ± 0.2°;
7.1 ± 0.2°, 16.3 ± 0.2°, 13.4 ± 0.2°, 15.5 ± 0.2°;
7.1 ± 0.2°, 16.3 ± 0.2°, 13.4 ± 0.2°, 17.4 ± 0.2°;
7.1 ± 0.2°, 16.3 ± 0.2°, 13.4 ± 0.2°, 17.7 ± 0.2°;
7.1 ± 0.2°, 16.3 ± 0.2°, 14.2 ± 0.2°, 17.4 ± 0.2°;
7.1 ± 0.2°, 16.3 ± 0.2°, 14.2 ± 0.2°, 17.7 ± 0.2°;
7.1 ± 0.2°, 16.3 ± 0.2°, 15.5 ± 0.2°, 17.7 ± 0.2°;
7.1 ± 0.2°, 13.4 ± 0.2°, 14.2 ± 0.2°, 18.0 ± 0.2°;
7.1 ± 0.2°, 14.2 ± 0.2°, 15.5 ± 0.2°, 18.0 ± 0.2°;
7.1 ± 0.2°, 15.5 ± 0.2°, 17.4 ± 0.2°, 18.0 ± 0.2°;
7.1 ± 0.2°, 17.4 ± 0.2°, 17.7 ± 0.2°, 18.0 ± 0.2°;
7.1 ± 0.2°, 13.4 ± 0.2°, 15.5 ± 0.2°, 18.0 ± 0.2°;
7.1 ± 0.2°, 13.4 ± 0.2°, 17.4 ± 0.2°, 18.0 ± 0.2°;
7.1 ± 0.2°, 13.4 ± 0.2°, 17.7 ± 0.2°, 18.0 ± 0.2°;
7.1 ± 0.2°, 14.2 ± 0.2°, 17.4 ± 0.2°, 18.0 ± 0.2°;
7.1 ± 0.2°, 14.2 ± 0.2°, 17.7 ± 0.2°, 18.0 ± 0.2°;
7.1 ± 0.2°, 15.5 ± 0.2°, 17.7 ± 0.2°, 18.0 ± 0.2°;
16.3 ± 0.2°, 13.4 ± 0.2°, 14.2 ± 0.2°, 18.0 ± 0.2°;
16.3 ± 0.2°, 14.2 ± 0.2°, 15.5 ± 0.2°, 18.0 ± 0.2°;
16.3 ± 0.2°, 15.5 ± 0.2°, 17.4 ± 0.2°, 18.0 ± 0.2°;
16.3 ± 0.2°, 17.4 ± 0.2°, 17.7 ± 0.2°, 18.0 ± 0.2°;
16.3 ± 0.2°, 13.4 ± 0.2°, 15.5 ± 0.2°, 18.0 ± 0.2°;
16.3 ± 0.2°, 13.4 ± 0.2°, 17.4 ± 0.2°, 18.0 ± 0.2°;
16.3 ± 0.2°, 13.4 ± 0.2°, 17.7 ± 0.2°, 18.0 ± 0.2°;
16.3 ± 0.2°, 14.2 ± 0.2°, 17.4 ± 0.2°, 18.0 ± 0.2°;
16.3 ± 0.2°, 14.2 ± 0.2°, 17.7 ± 0.2°, 18.0 ± 0.2°;

16.3 ± 0.2°, 15.5 ± 0.2°, 17.7 ± 0.2°, 18.0 ± 0.2°;
7.1 ± 0.2°, 16.3 ± 0.2°, 13.4 ± 0.2°, 14.2 ± 0.2°, 18.0 ± 0.2°;
7.1 ± 0.2°, 16.3 ± 0.2°, 14.2 ± 0.2°, 15.5 ± 0.2°, 18.0 ± 0.2°;
7.1 ± 0.2°, 16.3 ± 0.2°, 15.5 ± 0.2°, 17.4 ± 0.2°, 18.0 ± 0.2°;
7.1 ± 0.2°, 16.3 ± 0.2°, 17.4 ± 0.2°, 17.7 ± 0.2°, 18.0 ± 0.2°;
7.1 ± 0.2°, 16.3 ± 0.2°, 13.4 ± 0.2°, 15.5 ± 0.2°, 18.0 ± 0.2°;
7.1 ± 0.2°, 16.3 ± 0.2°, 13.4 ± 0.2°, 17.4 ± 0.2°, 18.0 ± 0.2°;
7.1 ± 0.2°, 16.3 ± 0.2°, 13.4 ± 0.2°, 17.7 ± 0.2°, 18.0 ± 0.2°;
7.1 ± 0.2°, 16.3 ± 0.2°, 14.2 ± 0.2°, 17.4 ± 0.2°, 18.0 ± 0.2°;
7.1 ± 0.2°, 16.3 ± 0.2°, 14.2 ± 0.2°, 17.7 ± 0.2°, 18.0 ± 0.2°;
7.1 ± 0.2°, 16.3 ± 0.2°, 15.5 ± 0.2°, 17.7 ± 0.2°, 18.0 ± 0.2°;
7.1 ± 0.2°, 16.3 ± 0.2°, 13.4 ± 0.2°, 14.2 ± 0.2°, 15.5 ± 0.2°, 18.0 ± 0.2°;
7.1 ± 0.2°, 16.3 ± 0.2°, 14.2 ± 0.2°, 15.5 ± 0.2°, 17.4 ± 0.2°, 18.0 ± 0.2°;
7.1 ± 0.2°, 16.3 ± 0.2°, 15.5 ± 0.2°, 17.4 ± 0.2°, 17.7 ± 0.2°, 18.0 ± 0.2°;
7.1 ± 0.2°, 16.3 ± 0.2°, 13.4 ± 0.2°, 15.5 ± 0.2°, 17.4 ± 0.2°, 18.0 ± 0.2°;
7.1 ± 0.2°, 16.3 ± 0.2°, 13.4 ± 0.2°, 15.5 ± 0.2°, 17.7 ± 0.2°, 18.0 ± 0.2°;
7.1 ± 0.2°, 16.3 ± 0.2°, 13.4 ± 0.2°, 14.2 ± 0.2°, 17.4 ± 0.2°, 18.0 ± 0.2°;
7.1 ± 0.2°, 16.3 ± 0.2°, 13.4 ± 0.2°, 14.2 ± 0.2°, 17.7 ± 0.2°, 18.0 ± 0.2°;
7.1 ± 0.2°, 16.3 ± 0.2°, 14.2 ± 0.2°, 15.5 ± 0.2°, 17.7 ± 0.2°, 18.0 ± 0.2°;
7.1 ± 0.2°, 16.3 ± 0.2°, 13.4 ± 0.2°, 14.2 ± 0.2°, 15.5 ± 0.2°, 17.4 ± 0.2°, 18.0 ± 0.2°;
7.1 ± 0.2°, 16.3 ± 0.2°, 14.2 ± 0.2°, 15.5 ± 0.2°, 17.4 ± 0.2°, 17.7 ± 0.2°, 18.0 ± 0.2°;
7.1 ± 0.2°, 16.3 ± 0.2°, 13.4 ± 0.2°, 15.5 ± 0.2°, 17.4 ± 0.2°, 17.7 ± 0.2°, 18.0 ± 0.2°;
7.1 ± 0.2°, 16.3 ± 0.2°, 13.4 ± 0.2°, 14.2 ± 0.2°, 17.4 ± 0.2°, 17.7 ± 0.2°, 18.0 ± 0.2°;
7.1 ± 0.2°, 16.3 ± 0.2°, 13.4 ± 0.2°, 14.2 ± 0.2°, 15.5 ± 0.2°, 17.7 ± 0.2°, 18.0 ± 0.2°;
7.1 ± 0.2°, 13.4 ± 0.2°, 14.2 ± 0.2°, 15.5 ± 0.2°, 16.3 ± 0.2°, 17.4 ± 0.2°, 17.7 ± 0.2°, 18.0 ± 0.2°;
more preferably, the X-ray powder diffraction pattern thereof optionally has a diffraction peak at one or more of 2θ of 19.8 ± 0.2°, 20.1 ± 0.2°, 21.0 ± 0.2°, 21.2 ± 0.2°, 22.6 ± 0.2°, 24.0 ± 0.2°, 24.8 ± 0.2°, 25.4 ± 0.2°, 26.1 ± 0.2°, 27.9 ± 0.2° or 28.6 ± 0.2°; preferably, the X-ray powder diffraction pattern thereof has at least a diffraction peak at any 2 to 4 of the above-mentioned 2θ, or at any 5 to 7 of the above-mentioned 2θ, and further preferably, at any 4 or 6 of the above-mentioned 2θ; for example,
19.8 ± 0.2°, 20.1 ± 0.2°, 21.0 ± 0.2°, 21.2 ± 0.2°;
20.1 ± 0.2°, 21.0 ± 0.2°, 21.2 ± 0.2°, 22.6 ± 0.2°;
21.0 ± 0.2°, 21.2 ± 0.2°, 22.6 ± 0.2°, 24.0 ± 0.2°;
21.2 ± 0.2°, 22.6 ± 0.2°, 24.0 ± 0.2°, 24.8 ± 0.2°;
22.6 ± 0.2°, 24.0 ± 0.2°, 24.8 ± 0.2°, 25.4 ± 0.2°;
19.8 ± 0.2°, 20.1 ± 0.2°, 21.0 ± 0.2°, 21.2 ± 0.2°, 22.6 ± 0.2°, 24.0 ± 0.2°;
20.1 ± 0.2°, 21.0 ± 0.2°, 21.2 ± 0.2°, 22.6 ± 0.2°, 24.0 ± 0.2°, 24.8 ± 0.2°;
21.0 ± 0.2°, 21.2 ± 0.2°, 22.6 ± 0.2°, 24.0 ± 0.2°, 24.8 ± 0.2°, 25.4 ± 0.2°;
21.2 ± 0.2°, 22.6 ± 0.2°, 24.0 ± 0.2°, 24.8 ± 0.2°, 25.4 ± 0.2°, 26.1 ± 0.2°;
22.6 ± 0.2°, 24.0 ± 0.2°, 24.8 ± 0.2°, 25.4 ± 0.2°, 26.1 ± 0.2°, 27.9 ± 0.2°;
24.0 ± 0.2°, 24.8 ± 0.2°, 25.4 ± 0.2°, 26.1 ± 0.2°, 27.9 ± 0.2°, 28.6 ± 0.2°;
further preferably, the X-ray powder diffraction pattern thereof is basically as shown in FIG. 5.

**10.** A crystal form of the acidic salt according to claim 5, wherein the crystal form is a hydrobromide crystal form, and the X-ray powder diffraction pattern thereof has a diffraction peak at 2θ of 9.2 ± 0.2°, or has a diffraction peak at 2θ of 12.0 ± 0.2°, or has a diffraction peak at 2θ of 12.9 ± 0.2°, or has a diffraction peak at 2θ of 14.6 ± 0.2°, or has a diffraction peak at 2θ of 16.8 ± 0.2°, or has a diffraction peak at 2θ of 17.1 ± 0.2°, or has a diffraction peak at 2θ of 18.6 ± 0.2°, or has a diffraction peak at 2θ of 22.7 ± 0.2°, or has a diffraction peak at 2θ of 19.5 ± 0.2°, or has a diffraction peak at 2θ of 19.8 ± 0.2°, or has a diffraction peak at 2θ of 20.2 ± 0.2°, or has a diffraction peak at 2θ of 22.5 ± 0.2°, or has a diffraction peak at 2θ of 25.4 ± 0.2°, or has a diffraction peak at 2θ of 26.4 ± 0.2°, or has a diffraction peak at 2θ of 27.5 ± 0.2°, or has a diffraction peak at 2θ of 27.8 ± 0.2°, or has a diffraction peak at 2θ of 28.9 ± 0.2°, or has a diffraction peak at 2θ of 29.3 ± 0.2°, or has a diffraction peak at 2θ of 32.3 ± 0.2°; preferably, the X-ray powder diffraction pattern thereof optionally has a diffraction peak at 2, 4, 6, 8 or 10 of the above-mentioned 2θ;

preferably, the X-ray powder diffraction pattern thereof has at least a diffraction peak at one or more of 2θ of 9.2 ± 0.2°, 12.0 ± 0.2° or 12.9 ± 0.2°; preferably, the X-ray powder diffraction pattern thereof has a diffraction peak at 2 of the above-mentioned 2θ, more preferably, at 3 of the above-mentioned 2θ; optionally, the X-ray powder diffraction pattern thereof further has a diffraction peak at one or more of 2θ of 14.6 ± 0.2°, 16.8 ± 0.2°, 17.1 ±

0.2°, 18.6 ± 0.2° or 22.7 ± 0.2°, preferably at 2, 3, 4 or 5 of the above-mentioned 2θ; for example,
9.2 ± 0.2°, 12.0 ± 0.2°, 14.6 ± 0.2°, 16.8 ± 0.2°;
9.2 ± 0.2°, 12.0 ± 0.2°, 16.8 ± 0.2°, 17.1 ± 0.2°;
9.2 ± 0.2°, 12.0 ± 0.2°, 17.1 ± 0.2°, 18.6 ± 0.2°;
9.2 ± 0.2°, 12.0 ± 0.2°, 18.6 ± 0.2°, 22.7 ± 0.2°;
9.2 ± 0.2°, 12.0 ± 0.2°, 14.6 ± 0.2°, 17.1 ± 0.2°;
9.2 ± 0.2°, 12.0 ± 0.2°, 14.6 ± 0.2°, 18.6 ± 0.2°;
9.2 ± 0.2°, 12.0 ± 0.2°, 14.6 ± 0.2°, 22.7 ± 0.2°;
9.2 ± 0.2°, 12.0 ± 0.2°, 16.8 ± 0.2°, 18.6 ± 0.2°;
9.2 ± 0.2°, 12.0 ± 0.2°, 16.8 ± 0.2°, 22.7 ± 0.2°;
9.2 ± 0.2°, 12.0 ± 0.2°, 17.1 ± 0.2°, 22.7 ± 0.2°;
12.0 ± 0.2°, 12.9 ± 0.2°, 14.6 ± 0.2°, 16.8 ± 0.2°;
12.0 ± 0.2°, 12.9 ± 0.2°, 16.8 ± 0.2°, 17.1 ± 0.2°;
12.0 ± 0.2°, 12.9 ± 0.2°, 17.1 ± 0.2°, 18.6 ± 0.2°;
12.0 ± 0.2°, 12.9 ± 0.2°, 18.6 ± 0.2°, 22.7 ± 0.2°;
12.0 ± 0.2°, 12.9 ± 0.2°, 14.6 ± 0.2°, 17.1 ± 0.2°;
12.0 ± 0.2°, 12.9 ± 0.2°, 14.6 ± 0.2°, 18.6 ± 0.2°;
12.0 ± 0.2°, 12.9 ± 0.2°, 14.6 ± 0.2°, 22.7 ± 0.2°;
12.0 ± 0.2°, 12.9 ± 0.2°, 16.8 ± 0.2°, 18.6 ± 0.2°;
12.0 ± 0.2°, 12.9 ± 0.2°, 16.8 ± 0.2°, 22.7 ± 0.2°;
12.0 ± 0.2°, 12.9 ± 0.2°, 17.1 ± 0.2°, 22.7 ± 0.2°;
9.2 ± 0.2°, 12.9 ± 0.2°, 14.6 ± 0.2°, 16.8 ± 0.2°;
9.2 ± 0.2°, 12.9 ± 0.2°, 16.8 ± 0.2°, 17.1 ± 0.2°;
9.2 ± 0.2°, 12.9 ± 0.2°, 17.1 ± 0.2°, 18.6 ± 0.2°;
9.2 ± 0.2°, 12.9 ± 0.2°, 18.6 ± 0.2°, 22.7 ± 0.2°;
9.2 ± 0.2°, 12.9 ± 0.2°, 14.6 ± 0.2°, 17.1 ± 0.2°;
9.2 ± 0.2°, 12.9 ± 0.2°, 14.6 ± 0.2°, 18.6 ± 0.2°;
9.2 ± 0.2°, 12.9 ± 0.2°, 14.6 ± 0.2°, 22.7 ± 0.2°;
9.2 ± 0.2°, 12.9 ± 0.2°, 16.8 ± 0.2°, 18.6 ± 0.2°;
9.2 ± 0.2°, 12.9 ± 0.2°, 16.8 ± 0.2°, 22.7 ± 0.2°;
9.2 ± 0.2°, 12.9 ± 0.2°, 17.1 ± 0.2°, 22.7 ± 0.2°;
9.2 ± 0.2°, 12.0 ± 0.2°, 14.6 ± 0.2°, 16.8 ± 0.2°, 17.1 ± 0.2°;
9.2 ± 0.2°, 12.0 ± 0.2°, 16.8 ± 0.2°, 17.1 ± 0.2°, 18.6 ± 0.2°;
9.2 ± 0.2°, 12.0 ± 0.2°, 17.1 ± 0.2°, 18.6 ± 0.2°, 22.7 ± 0.2°;
9.2 ± 0.2°, 12.0 ± 0.2°, 14.6 ± 0.2°, 17.1 ± 0.2°, 18.6 ± 0.2°;
9.2 ± 0.2°, 12.9 ± 0.2°, 14.6 ± 0.2°, 16.8 ± 0.2°, 17.1 ± 0.2°;
9.2 ± 0.2°, 12.9 ± 0.2°, 16.8 ± 0.2°, 17.1 ± 0.2°, 18.6 ± 0.2°;
9.2 ± 0.2°, 12.9 ± 0.2°, 17.1 ± 0.2°, 18.6 ± 0.2°, 22.7 ± 0.2°;
9.2 ± 0.2°, 12.9 ± 0.2°, 14.6 ± 0.2°, 17.1 ± 0.2°, 18.6 ± 0.2°;
12.0 ± 0.2°, 12.9 ± 0.2°, 14.6 ± 0.2°, 16.8 ± 0.2°, 17.1 ± 0.2°;
12.0 ± 0.2°, 12.9 ± 0.2°, 16.8 ± 0.2°, 17.1 ± 0.2°, 18.6 ± 0.2°;
12.0 ± 0.2°, 12.9 ± 0.2°, 17.1 ± 0.2°, 18.6 ± 0.2°, 22.7 ± 0.2°;
12.0 ± 0.2°, 12.9 ± 0.2°, 14.6 ± 0.2°, 17.1 ± 0.2°, 18.6 ± 0.2°;
9.2 ± 0.2°, 12.0 ± 0.2°, 12.9 ± 0.2°, 14.6 ± 0.2°, 16.8 ± 0.2°, 17.1 ± 0.2°;
9.2 ± 0.2°, 12.0 ± 0.2°, 12.9 ± 0.2°, 16.8 ± 0.2°, 17.1 ± 0.2°, 18.6 ± 0.2°;
9.2 ± 0.2°, 12.0 ± 0.2°, 12.9 ± 0.2°, 17.1 ± 0.2°, 18.6 ± 0.2°, 22.7 ± 0.2°;
9.2 ± 0.2°, 12.0 ± 0.2°, 12.9 ± 0.2°, 14.6 ± 0.2°, 17.1 ± 0.2°, 18.6 ± 0.2°;
9.2 ± 0.2°, 12.0 ± 0.2°, 12.9 ± 0.2°, 14.6 ± 0.2°, 16.8 ± 0.2°, 17.1 ± 0.2°, 18.6 ± 0.2°;
9.2 ± 0.2°, 12.0 ± 0.2°, 12.9 ± 0.2°, 16.8 ± 0.2°, 17.1 ± 0.2°, 18.6 ± 0.2°, 22.7 ± 0.2°;
9.2 ± 0.2°, 12.0 ± 0.2°, 12.9 ± 0.2°, 14.6 ± 0.2°, 17.1 ± 0.2°, 18.6 ± 0.2°, 22.7 ± 0.2°;
9.2 ± 0.2°, 12.0 ± 0.2°, 12.9 ± 0.2°, 14.6 ± 0.2°, 16.8 ± 0.2°, 18.6 ± 0.2°, 22.7 ± 0.2°;
9.2 ± 0.2°, 12.0 ± 0.2°, 12.9 ± 0.2°, 14.6 ± 0.2°, 16.8 ± 0.2°, 17.1 ± 0.2°, 22.7 ± 0.2°;
9.2 ± 0.2°, 12.0 ± 0.2°, 12.9 ± 0.2°, 14.6 ± 0.2°, 16.8 ± 0.2°, 17.1 ± 0.2°, 18.6 ± 0.2°, 22.7 ± 0.2°;
more preferably, the X-ray powder diffraction pattern thereof optionally has a diffraction peak at one or more of 2θ of 19.5 ± 0.2°, 19.8 ± 0.2°, 20.2 ± 0.2°, 22.5 ± 0.2°, 25.4 ± 0.2°, 26.4 ± 0.2°, 27.5 ± 0.2°, 27.8 ± 0.2°, 28.9 ± 0.2°, 29.3 ± 0.2° or 32.3 ± 0.2°; preferably, the X-ray powder diffraction pattern thereof has at least a diffraction peak at any 2 to 4 of the above-mentioned 2θ, or at any 5 to 7 of the above-mentioned 2θ, and further preferably, at any 4 or 6 of the above-mentioned 2θ; for example,

19.5 ± 0.2°, 19.8 ± 0.2°, 20.2 ± 0.2°, 22.5 ± 0.2°;
19.8 ± 0.2°, 20.2 ± 0.2°, 22.5 ± 0.2°, 25.4 ± 0.2°;
20.2 ± 0.2°, 22.5 ± 0.2°, 25.4 ± 0.2°, 26.4 ± 0.2°;
22.5 ± 0.2°, 25.4 ± 0.2°, 26.4 ± 0.2°, 27.5 ± 0.2°;
25.4 ± 0.2°, 26.4 ± 0.2°, 27.5 ± 0.2°, 27.8 ± 0.2°;
26.4 ± 0.2°, 27.5 ± 0.2°, 27.8 ± 0.2°, 28.9 ± 0.2°;
27.5 ± 0.2°, 27.8 ± 0.2°, 28.9 ± 0.2°, 29.3 ± 0.2°;
27.8 ± 0.2°, 28.9 ± 0.2°, 29.3 ± 0.2°, 32.3 ± 0.2°;
19.5 ± 0.2°, 19.8 ± 0.2°, 20.2 ± 0.2°, 22.5 ± 0.2°, 25.4 ± 0.2°, 26.4 ± 0.2°;
19.8 ± 0.2°, 20.2 ± 0.2°, 22.5 ± 0.2°, 25.4 ± 0.2°, 26.4 ± 0.2°, 27.5 ± 0.2°;
20.2 ± 0.2°, 22.5 ± 0.2°, 25.4 ± 0.2°, 26.4 ± 0.2°, 27.5 ± 0.2°, 27.8 ± 0.2°;
22.5 ± 0.2°, 25.4 ± 0.2°, 26.4 ± 0.2°, 27.5 ± 0.2°, 27.8 ± 0.2°, 28.9 ± 0.2°;
25.4 ± 0.2°, 26.4 ± 0.2°, 27.5 ± 0.2°, 27.8 ± 0.2°, 28.9 ± 0.2°, 29.3 ± 0.2°;
26.4 ± 0.2°, 27.5 ± 0.2°, 27.8 ± 0.2°, 28.9 ± 0.2°, 29.3 ± 0.2°, 32.3 ± 0.2°;
further preferably, the crystal form is a hydrobromide crystal form of the compound (R)-(3-(3-chloro-1,2,4-thiadiazol-5-yl)-8-methyl-5,6-dihydro-[1,2,4]triazolo[4,3-a]pyrazin-7(8H)-yl)(4-fluorophenyl-3-d)methanone, and the X-ray powder diffraction pattern thereof is basically as shown in FIG. 6.

**11.** The acidic salt or crystal form thereof according to any one of claims 4-10, wherein the number of the acid is 0.2 to 3; preferably 0.2, 0.5, 1, 1.5, 2, 2.5 or 3; more preferably 0.5, 1, 2 or 3; further preferably 1.

**12.** The acidic salt or crystal form thereof according to any one of claims 4-11, wherein the salt is hydrate or anhydrate; when the salt is hydrate, the number of water molecules is 0.2 to 3; preferably 0.2, 0.5, 1, 1.5, 2, 2.5 or 3; more preferably 0.5, 1, 2 or 3.

**13.** A method for preparing the crystal form according to any one of claims 1-3, comprising

a) weighing an appropriate amount of the compound, dissolving same with an organic solvent to clarification,
b) stirring, filtering and drying same to obtain the target product;

wherein the organic solvent is selected from methanol, ethanol, n-propanol, isopropanol, n-butanol, acetonitrile, acetone, methyl ethyl ketone, tetrahydrofuran, dioxane, N,N-dimethylformamide, dimethyl sulfoxide, ethyl acetate, isopropyl acetate, dichloromethane, trichloroethane, carbon tetrachloride, methyl tert-butyl ether, isopropyl ether, benzene, toluene, xylene or a combination thereof.

**14.** A method for preparing the acidic salt or crystal form thereof according to any one of claims 4-12, wherein the method comprises the following steps:

a) weighing an appropriate amount of the free base crystal form of the compound and adding same to an organic solvent;
b) weighing an appropriate amount of the acid, dissolving same with the organic solvent, stirring and then filtering same or dissolving and evaporating same to obtain a gel, to which an antisolvent is added, stirring and filtering same, and drying same in vacuum and separating same to obtain the target product;

wherein:

the organic solvent is selected from methanol, ethanol, n-propanol, isopropanol, n-butanol, acetonitrile, acetone, methyl ethyl ketone, tetrahydrofuran, dioxane, N,N-dimethylformamide, dimethyl sulfoxide, ethyl acetate, isopropyl acetate, dichloromethane, trichloroethane, carbon tetrachloride, methyl tert-butyl ether, isopropyl ether, benzene, toluene, xylene or a combination thereof;
the antisolvent is selected from ethyl acetate, n-heptane, n-hexane, isooctane, pentane, cyclohexane, cyclopentane, diethyl ether or a combination thereof;
the acid is selected from an inorganic acid or an organic acid, wherein the inorganic acid is selected from hydrochloric acid, sulfuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetylaminobenzoic acid, 4-aminobenzoic acid, decanoic acid, hexanoic acid, octanoic acid, cinnamic acid, citric acid, cyclohexane aminosulfonic acid, camphor sulfonic acid, aspartic acid,

camphor acid, gluconic acid, glucuronic acid, glutamic acid, isoascorbic acid, lactic acid, malic acid, mandelic acid, pyroglutamic acid, tartaric acid, dodecyl sulfuric acid, dibenzoyl tartaric acid, ethane-1,2-disulfonic acid, ethanesulfonic acid, formic acid, fumaric acid, galactonic acid, gentisuric acid, glutaric acid, 2-ketoglutaric acid, glycolic acid, benzoylglycine, hydroxyethyl sulfonic acid, lactobionic acid, ascorbic acid, aspartic acid, lauric acid, camphor acid, maleic acid, malonic acid, methanesulfonic acid, 1,5-naphthalene disulfonic acid, naphthalene-2-sulfonic acid, nicotinic acid, oleic acid, whey acid, oxalic acid, palmitic acid, pamoic acid, propanoic acid, salicylic acid, 4-aminosalicylic acid, sebacic acid, stearic acid, succinic acid, thiocyanic acid, pamoic acid, formic acid, undecylenoic acid, trifluoroacetic acid, benzenesulfonic acid, p-toluenesulfonic acid or L-malic acid; preferably, the acid is selected from hydrochloric acid, methanesulfonic acid, nitric acid, sulfuric acid or hydrobromic acid.

**15.** A pharmaceutical composition comprising a therapeutically effective amount of the crystal form, or the acidic salt or crystal form thereof according to any one of claims 1-12, as well as a pharmaceutically acceptable carrier.

**16.** The crystal form, or the acidic salt or crystal form thereof according to any one of claims 1-12, as well as the pharmaceutical composition according to claim 15, wherein the pharmaceutical composition comprises a therapeutically effective amount of the crystal form, or the acidic salt or crystal form thereof, and the therapeutically effective amount includes 0.0001-99%, 0.0001-95%, 0.0001-90%, 0.0001-85%, 0.0001-80%, 0.0001-75%, 0.0001-70%, 0.001-60%, 0.001-55%, 0.01-50%, 0.01-40%, 0.01-30%, 0.01-20%, 0.01-10% or 0.01-5%.

**17.** Use of the crystal form, or the acidic salt or crystal form thereof according to any one of claims 1-12, as well as the pharmaceutical composition according to claim 15 in the preparation of NK3 inhibitor-related drugs, preferably in the preparation of drugs for the treatment and/or prevention of related diseases such as psychiatric disorders, cognitive impairment, Parkinson's disease, Alzheimer's disease, attention deficit hyperactivity disorder, pain, convulsions, obesity, inflammatory diseases, vomiting, preeclampsia, airway related diseases, reproductive disorders, hormone dependent diseases, or gynecological disease; further preferably, in the preparation of drugs for the treatment and/or prevention of diseases related to menopausal syndrome, which includes hot flashes, sweating, palpitations, dizziness and obesity.

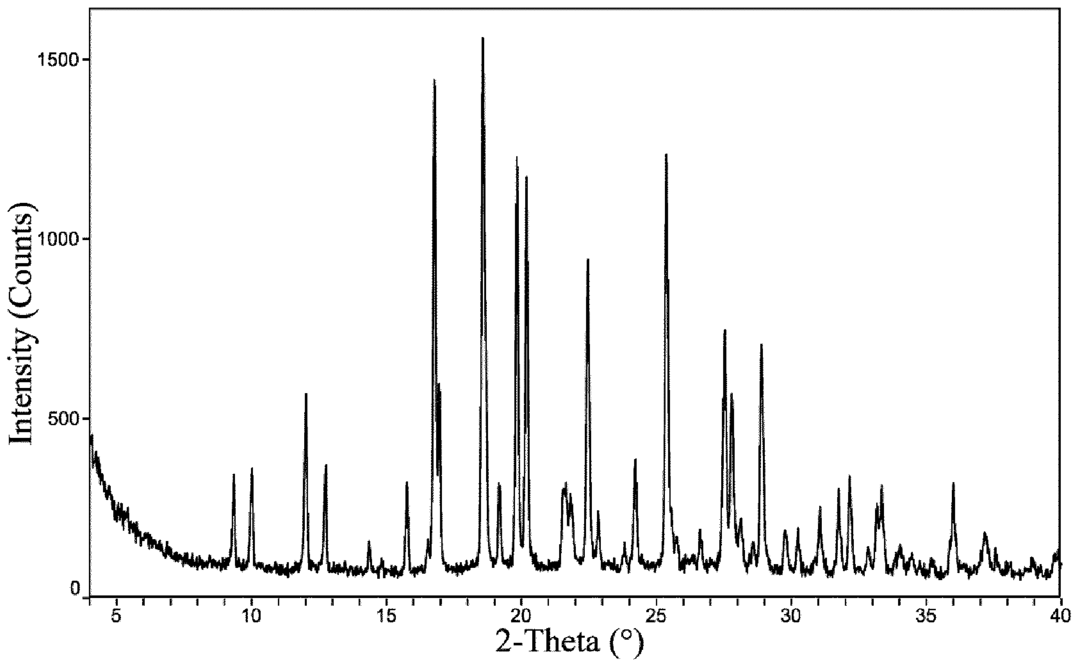


Figure 1

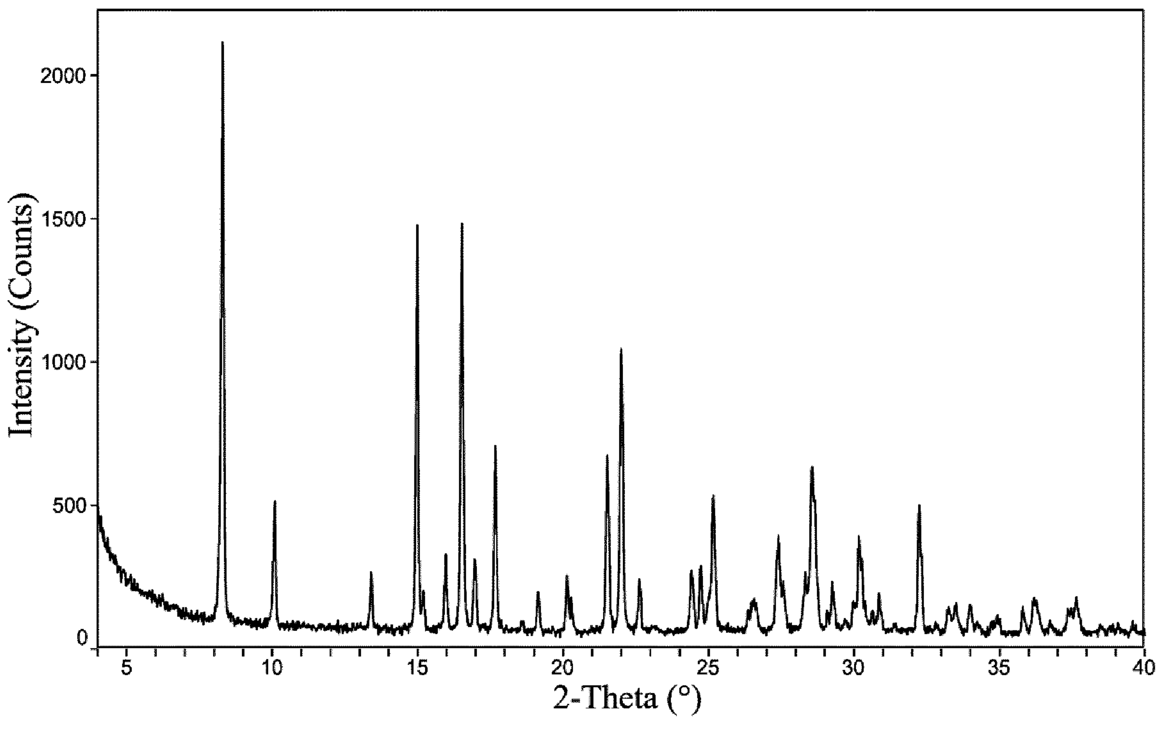


Figure 2

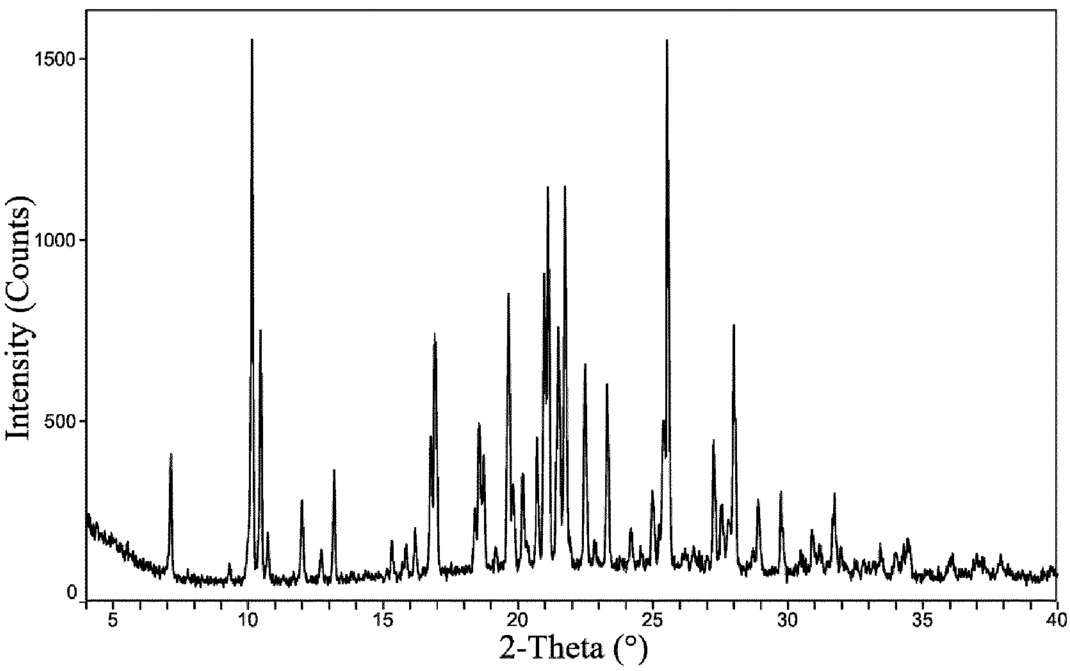


Figure 3

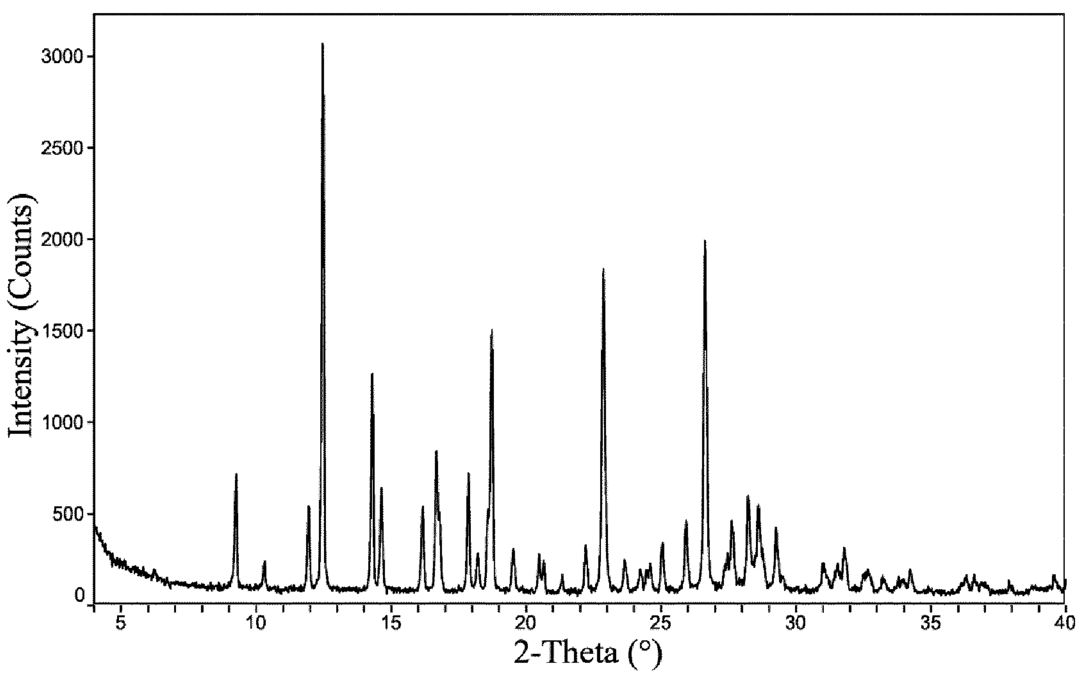


Figure 4

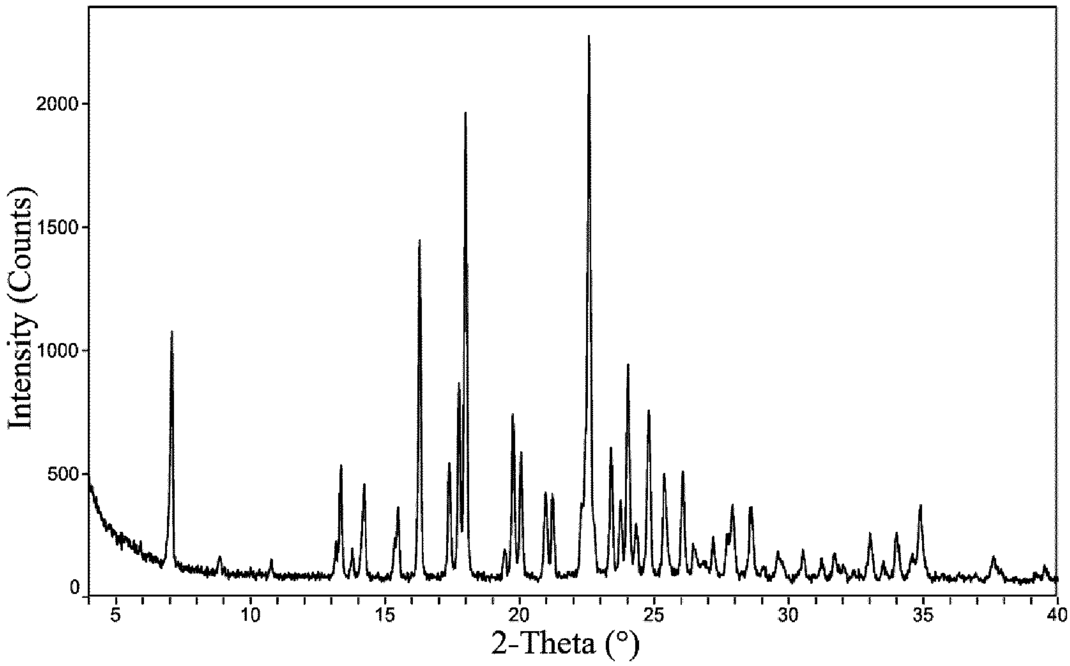


Figure 5

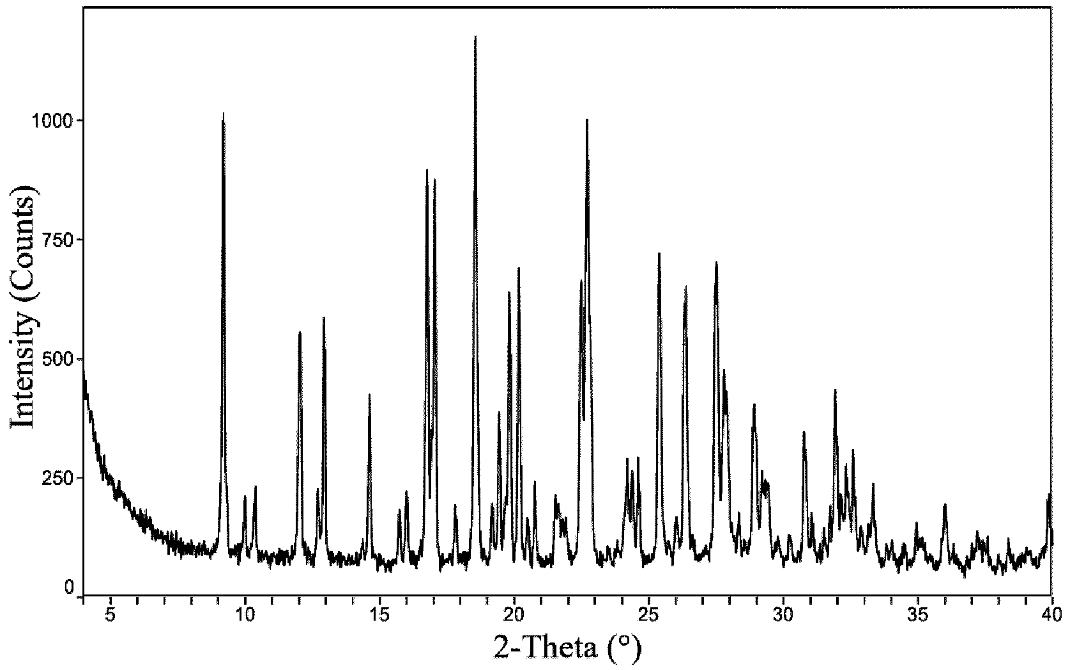


Figure 6

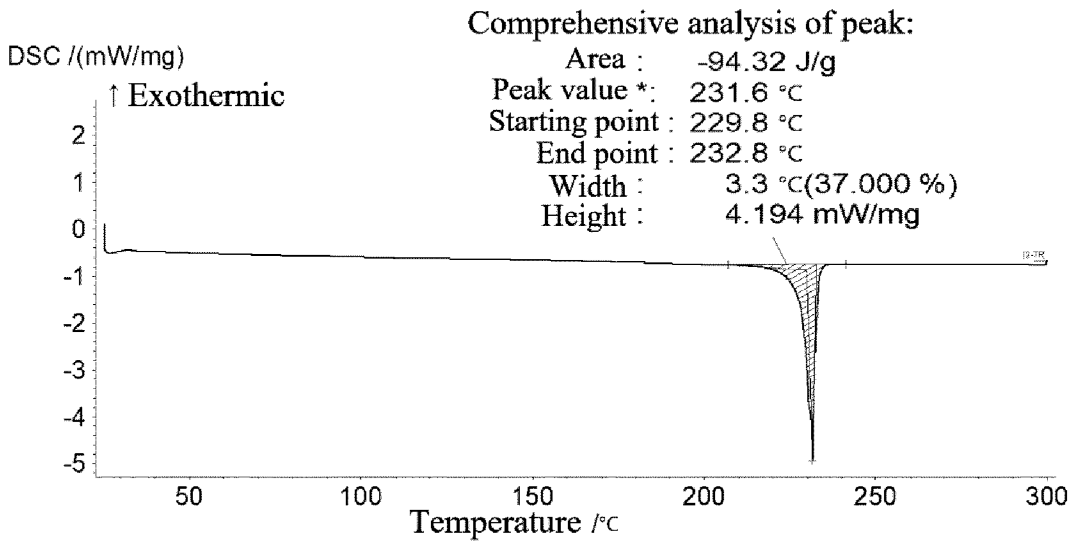
Comprehensive analysis of peak:
Area : -94.32 J/g
Peak value *: 231.6 °C
Starting point : 229.8 °C
End point : 232.8 °C
Width : 3.3 °C(37.000 %)
Height : 4.194 mW/mg
DSC /(mW/mg)
↑ Exothermic
2
1
0
-1
-2
-3
-4
-5
50
100
150
200
250
300
Temperature /°C

Figure 7

## INTERNATIONAL SEARCH REPORT

International application No. **PCT/CN2023/073323**

**A. CLASSIFICATION OF SUBJECT MATTER**

C07D487/04(2006.01)i;A61K31/495(2006.01)i;A61K31/4985(2006.01)i;A61P25/22(2006.01)i

According to International Patent Classification (IPC) or to both national classification and IPC

**B. FIELDS SEARCHED**

Minimum documentation searched (classification system followed by classification symbols)

C07D487 A61K A61P

Documentation searched other than minimum documentation to the extent that such documents are included in the fields searched

Electronic data base consulted during the international search (name of data base and, where practicable, search terms used)

CNABS; CNTXT; CJFD; VEN; WOTXT; USTXT; EPTXT; CNKI; PATENTICS; 万方, WANFANG; 超星读秀, CHAOXING DUXIU; ISI-Web of Science; STN-registry; STN-caplus: 上海翰森生物, 江苏豪森药业, 常州恒邦药业, 陈金瑶, 呙临松, 含氮并环, 神经激肽, NK抑制剂, 抑郁, 焦虑, 精神分裂, 氘, 结晶, 晶体, 晶型, 多晶型, 盐, 酸式盐, NK inhibitor, Neurokinin, Depression, Anxiety, Schizophrenia, Deuterium, Crystal, Crystalline, Acid Salt, 结构式(I)

**C. DOCUMENTS CONSIDERED TO BE RELEVANT**

| Category* | Citation of document, with indication, where appropriate, of the relevant passages | Relevant to claim No. |
|---|---|---|
| PX | WO 2022022680 A1 (SHANGHAI HANSOH BIOMEDICAL CO., LTD. et al.) 03 February 2022 (2022-02-03)<br>claims 1-11 and 14-17 | 1-17 |
| X | WO 2020211798 A1 (SHANGHAI HANSOH BIOMEDICAL CO., LTD. et al.) 22 October 2020 (2020-10-22)<br>claims 1, 26, and 29-32 | 1-17 |
| X | CN 111212838 A (EUROSCREEN SA) 29 May 2020 (2020-05-29)<br>claims 1-6 | 1-17 |
| X | CN 105229008 A (EUROSCREEN SA) 06 January 2016 (2016-01-06)<br>claims 1 and 12-15 | 1-17 |
| X | CN 111825677 A (MINGHUI PHARMACEUTICAL (HANGZHOU) CO., LTD. et al.) 27 October 2020 (2020-10-27)<br>claims 1 and 7-10 | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 April 2023** | **27 April 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2023/073323**

| C. DOCUMENTS CONSIDERED TO BE RELEVANT | | |
|---|---|---|
| Category* | Citation of document, with indication, where appropriate, of the relevant passages | Relevant to claim No. |
| A | CN 111212841 A (ASTELLAS PHARMA INC.) 29 May 2020 (2020-05-29)<br>entire document | 1-17 |

Form PCT/ISA/210 (second sheet) (July 2022)

**INTERNATIONAL SEARCH REPORT**
**Information on patent family members**

International application No.
**PCT/CN2023/073323**

| Patent document cited in search report | Publication date (day/month/year) | Patent family member(s) | Publication date (day/month/year) |
|---|---|---|---|
| WO 2022022680 A1 | 03 February 2022 | CN 115427412 A | 02 December 2022 |
| | | TW 202214646 A | 16 April 2022 |
| WO 2020211798 A1 | 22 October 2020 | CN 112272670 A | 26 January 2021 |
| | | HK 40036075 A0 | 21 May 2021 |
| CN 111212838 A | 29 May 2020 | WO 2019012033 A1 | 17 January 2019 |
| | | EP 3428168 A1 | 16 January 2019 |
| | | US 2020270254 A1 | 27 August 2020 |
| | | US 11078203 B2 | 03 August 2021 |
| | | CN 111212838 B | 02 December 2022 |
| CN 105229008 A | 06 January 2016 | HK 1244273 A1 | 03 August 2018 |
| | | JP 2016515565 A | 30 May 2016 |
| | | JP 6316932 B2 | 25 April 2018 |
| | | IL 241473 B | 31 March 2019 |
| | | KR 20210021406 A | 25 February 2021 |
| | | KR 102364835 B1 | 17 February 2022 |
| | | EA 201591689 A1 | 29 January 2016 |
| | | EA 027570 B1 | 31 August 2017 |
| | | US 2016318941 A1 | 03 November 2016 |
| | | US 2017240551 A9 | 24 August 2017 |
| | | US 10030025 B2 | 24 July 2018 |
| | | HK 1214816 A1 | 05 August 2016 |
| | | ES 2646488 T3 | 14 December 2017 |
| | | MX 2015013711 A | 26 February 2016 |
| | | MX 370000 B | 28 November 2019 |
| | | EP 2948455 A1 | 02 December 2015 |
| | | EP 2948455 B1 | 27 September 2017 |
| | | JP 2018118988 A | 02 August 2018 |
| | | KR 20160007515 A | 20 January 2016 |
| | | KR 102218621 B1 | 22 February 2021 |
| | | EP 3219715 A1 | 20 September 2017 |
| | | US 2015232471 A1 | 20 August 2015 |
| | | US 9422299 B2 | 23 August 2016 |
| | | SG 11201508005 XA | 29 October 2015 |
| | | WO 2014154895 A1 | 02 October 2014 |
| | | US 2017095472 A1 | 06 April 2017 |
| | | US 9987274 B2 | 05 June 2018 |
| | | CA 2907809 A1 | 02 October 2014 |
| | | CA 2907809 C | 04 May 2021 |
| | | AU 2014242906 A1 | 22 October 2015 |
| | | AU 2014242906 A2 | 05 November 2015 |
| | | AU 2014242906 B2 | 26 July 2018 |
| | | BR 112015024907 A2 | 18 July 2017 |
| | | IN 201502460 P3 | 03 June 2016 |
| | | ZA 201506234 A | 30 January 2019 |
| | | NZ 712802 A2 | 28 August 2020 |
| | | SG 11201508005 A1 | 29 October 2015 |
| | | SG 11201508005 B | 06 September 2018 |
| | | CN 105229008 B | 15 December 2017 |
| | | US 2018111943 A1 | 26 April 2018 |
| | | US 10183948 B2 | 22 January 2019 |

Form PCT/ISA/210 (patent family annex) (July 2022)

**INTERNATIONAL SEARCH REPORT**
**Information on patent family members**

International application No.
**PCT/CN2023/073323**

| Patent document cited in search report | Publication date (day/month/year) | Patent family member(s) | Publication date (day/month/year) |
|---|---|---|---|
| | | ZA 201506234 A | 30 January 2019 |
| | | US 2019092777 A1 | 28 March 2019 |
| | | US 10836768 B2 | 17 November 2020 |
| | | US 2021094959 A1 | 01 April 2021 |
| | | IN 413366 B | 02 December 2022 |
| CN 111825677 A | 27 October 2020 | None | |
| CN 111212841 A | 29 May 2020 | JP 2021014404 A | 12 February 2021 |
| | | US 2021094955 A1 | 01 April 2021 |
| | | WO 2019074081 A1 | 18 April 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2021109577 W

### Non-patent literature cited in the description


- Polymorphism in Pharmaceutical Solids. Theory and Origin of Polymorphism. 1999
- **SMITH, D.K**. A FORTRAN Program for Calculating X-Ray Powder Diffraction Patterns. *Lawrence Radiation Laboratory, Livermore, California, UCRL-7196*, April 1963
- **YIN. S.** ; **SCARINGE, R.P.** ; **DIMARCO, J.** ; **GALELLA, M** ; **GOUGOUTAS, J.Z**. *American Pharmceutical Review.*, 2003, vol. 6 (2), 80
- **S.R. BYRN** ; **R.R. PFEIFFER** ; **J.G. STOWELL**. Solid-State Chemistry of Drugs. SSCI, 1999
- **J. W. MULLIN** ; **J. NYVLT**. Programmed cooling of batch crystallizers. *Chemical Engineering Science*, 1971, vol. 26, 369-377